# EUROPEAN PATENT APPLICATION

(11) **EP 4 772 642 A1**
(43) Date of publication of application: **08.07.2026**
(21) Application number: 24858665.3
(22) Date of filing: 29.08.2024
(51) Int. Cl.: C12N 15/70, C12N 15/64, C12N 1/21, C12N 15/861

(54) **CLONING/EXPRESSION VECTOR FOR NON-B DNA SEQUENCE**

(30) Priority: 29.08.2023 CN 202311099203
(71) Applicant: Nanjing GenScript Biotech Co., Ltd., Nanjing, Jiangsu 211100 (CN)
(72) Inventor: ZUO, Wenlu, Nanjing, Jiangsu 211100 (CN); ZHANG, Kun, Nanjing, Jiangsu 211100 (CN); MENG, Qingwei, Nanjing, Jiangsu 211100 (CN); WANG, Shiqin, Nanjing, Jiangsu 211100 (CN); LI, Meng, Nanjing, Jiangsu 211100 (CN); FAN, Weicheng, Nanjing, Jiangsu 211100 (CN); SHAN, Yeqi, Nanjing, Jiangsu 211100 (CN)
(74) Representative: Ström & Gulliksson AB
(86) International application number: PCT/CN2024/115387
(87) International publication number: WO 2025/045133

(57) **Abstract**

Provided is a vector. The vector comprises i) a replication origin; ii) a first terminator, iii) a) a target DNA sequence, comprising a DNA sequence capable of forming a non-B DNA conformation, or b) a target DNA expression cassette, comprising a promoter and a target DNA sequence, wherein the target DNA sequence comprises a DNA sequence capable of forming a non-B DNA conformation; iv) a second terminator; and v) a selectable marker and a promoter thereof, wherein in a replication direction, the replication origin is at least 150 bp apart from the DNA sequence capable of forming the non-B DNA conformation, the first terminator and the second terminator are respectively located upstream and downstream of the target DNA sequence in a), or the first terminator and the second terminator are respectively located upstream and downstream of the target DNA expression cassette in b). The provided vector can improve the amplification and/or expression (transcription) stability of poly(A) tail and ITR sequences.

## Description

### CROSS-REFERENCE TO RELATED APPLICATIONS

The present application claims priority to Chinese Patent Application No. 202311099203.6 filed on August 29, 2023, which is incorporated herein by reference in its entirety.

### TECHNICAL FIELD

The present application relates to a cloning vector or expression vector that can be used for sequences capable of forming, for example, a non-B-DNA conformation, and construction and use thereof.

### BACKGROUND

mRNA vaccines are nucleic acid preparations produced by processes such as transcribing exogenous target genes or direct synthesis. These vaccines can be introduced into the cells in the body through specific delivery systems to express the target protein, thereby stimulating the body to produce specific immunological responses and enabling the body to acquire immune protection.

The main sequence of a mature mRNA is the coding region, with untranslated regions (UTRs) located on its upstream 5' side and downstream 3' side. Eukaryotic mRNA molecules further have a 5' cap at one end and a 3' poly(A) tail at the other. The poly(A) tail plays a crucial role in maintaining mRNA stability, regulating mRNA translation efficiency, and maintaining mRNA transport. For the large-scale synthesis of mRNA, the currently efficient method is *in-vitro* transcription (IVT). There are mainly two approaches for poly(A) tailing in IVT. The first is enzymatic synthesis. Specifically, after transcribing mRNA from a linear DNA template, poly(A) polymerase is added to catalyze the addition of AMP converted from ATP to the 3' end of the single-stranded RNA, thereby forming a Poly(A) tail. The advantages of this approach are that the template DNA does not need to comprise a Poly(A) sequence and the operation is simple. However, this approach has the limitation of unstable tail length. The second is co-transcription, which involves the direct transcription of the poly(A) tail-coding sequence already present in the template plasmid DNA or PCR product. The advantages of this approach include eliminating the need for Poly(A) polymerase, reducing process steps, saving costs, and achieving relatively stable transcribed tail lengths. However, during the preparation of upstream plasmids, it is necessary to reduce the deletion of the poly(A) tail-coding sequence during cultivation, which is crucial for providing a high-yield, high-quality, and homogeneous plasmid template.

The large-scale production of plasmids is usually performed by *Escherichia coli* fermentation. During amplification, plasmids carrying long poly(A) tail-coding sequences are inherently unstable, and the poly(A) tail-coding sequence is prone to deletion during replication. To enhance the stability of the poly(A) tail-coding sequence on plasmids, some scientists have adopted segmented poly(A) tail sequences. Since each poly(A)-binding protein (PABP) only binds to about 30 adenines (A), a small number of A residues between two binding proteins act as a spacer sequence. Replacing the spacer sequence with other non-A bases reduces the probability of recombination in the poly(A) tail-coding sequence. In addition, the length and bases of the spacer sequence can be optimized to further reduce the risk of poly(A) tail deletion. However, even with these modifications, the risk of A residue deletion still persists, and plasmids with a segmented poly(A) tail prepared in this way exert a certain negative impact on the expression levels of downstream proteins. In addition, it has also been reported that the integrity of the plasmid Poly(A) tail-coding sequence can be improved by low-temperature fermentation (30°C). However, low-temperature cultivation slows down bacterial growth, reduces plasmid copy number, significantly decreases yield, and prolongs the cycle, posing difficulties for plasmid production. Currently, common commercial vectors used for mRNA vaccine production, such as pVAX1, also exhibit low plasmid copy number, low yield, and impure plasmid Poly(A) tail sequences in practical production.

Therefore, developing more stable poly(A) tail cloning vectors that reduce the risk of poly(A) tail sequence deletion or impurity without affecting plasmid yield may become a new research direction. However, there are currently few reports on the impact of vectors and elements thereof on the stability of poly(A) tail sequences.

Similarly, the inverted terminal repeats (ITRs) of adeno-associated virus (AAV) also suffer from unstable amplification in vectors. ITRs are located at both ends of the AAV genome, each forming a unique self-complementary T-shaped palindromic hairpin structure with a high GC content. They are prone to mutation or recombination-mediated loss during bacterial propagation. The integrity of ITRs is crucial for the efficient replication and packaging of recombinant AAV (rAAV), and the ITR deletion rate should preferably be less than 20% (Flotte, T. R., and B. J. Carter. (1995) Adeno-associated virus vectors for gene therapy, Gene therapy 2(6): 357-362). Single-molecule real-time sequencing, which comprehensively analyses the rAAV genome as a single intact molecule, has revealed a correlation between deleted ITRs and the packaging of non-vector sequences in a HEK293 triple-plasmid packaging system. The impact of ITR deletion on rAAV viral packaging and the application of rAAV in gene therapy is complex, and unexpected biological consequences may arise through vector-host interactions. If incomplete ITR sequences are used during the preclinical stage, the reproducibility of experiments both within and across laboratories may be reduced, and the reproducibility of clinical trials using intact ITR sequences may also be compromised, thereby delaying translation into clinical applications.

Currently, there is no effective method to avoid ITR deletion during the construction of rAAV-ITR plasmids and the amplification thereof in *Escherichia coli.* Some commercial recombination-deficient strains, such as JC8111, SURE2, Stabl3, and XL10-Gold, are often used for the construction and amplification of plasmids comprising ITR structures, but they are not always effective.

The citation of any document in the present application shall not be construed as an admission that such document constitutes prior art to the present application.

### SUMMARY

The inventors of the present application have found through experiments that the amplification and/or expression (transcription) stability of sequences prone to mutation and deletion can be significantly enhanced by: i) increasing a spacing distance between an origin of replication in a vector and sequences prone to mutation and deletion during replication, such as poly(A) tail-coding sequences and ITR sequences; ii) adding terminators upstream and downstream of sequences prone to mutation and deletion during replication, such as a poly(A) tail-coding sequences and ITRs, or expression cassettes thereof; and/or iii) disrupting a functional region of a native lac promoter in a vector. In addition, the above modifications to the vector can increase the yield of the vector, particularly the yield (e.g., packaging titer) of recombinant adeno-associated virus (rAAV).

Therefore, in a first aspect, the present application provides a vector, which may comprise:
i) an origin of replication; and
ii) a) a target DNA expression cassette, comprising a promoter and a target DNA sequence, or b) a target DNA sequence.

The target DNA sequence in the target DNA expression cassette of a) may comprise a DNA sequence capable of forming a non-B DNA conformation. The target DNA sequence in the target DNA expression cassette of a) may comprise a DNA sequence capable of forming a non-B DNA conformation, and a cloning site. The cloning site may be a multiple cloning site.

The target DNA sequence of b) may comprise a DNA sequence capable of forming a non-B DNA conformation. The target DNA sequence of b) may comprise a DNA sequence capable of forming a non-B DNA conformation, and a cloning site. The cloning site may be a multiple cloning site.

The DNA sequence capable of forming a non-B DNA conformation may be a direct repeat sequence, an inverted repeat sequence, or a homopurine-homopyrimidine sequence, including but not limited to polyadenine (poly(A)), polyguanine (poly(G)), polycytosine (poly(C)), polythymine (poly(T)), an shRNA-coding sequence, and an ITR sequence. The direct repeat sequence may be a poly(A) tail-coding sequence. The homopurine-homopyrimidine sequence may be a poly(A) tail-coding sequence. The inverted repeat sequence may be an ITR or a derivative sequence thereof. In some embodiments, the DNA sequence capable of forming a non-B DNA conformation may be a poly(A)-coding sequence.

The origin of replication may be spaced at least 150 bp, 200 bp, 300 bp, 500 bp, or 700 bp in a replication direction from the DNA sequence capable of forming a non-B DNA conformation in the target DNA expression cassette of a), or the DNA sequence capable of forming a non-B DNA conformation in the target DNA sequence of b). In some embodiments, the origin of replication may be spaced at least 300 bp in a replication direction from the DNA sequence capable of forming a non-B DNA conformation in the target DNA expression cassette of a), or the DNA sequence capable of forming a non-B DNA conformation in the target DNA sequence of b). In some embodiments, the origin of replication may be spaced at least 500 bp in a replication direction from the DNA sequence capable of forming a non-B DNA conformation in the target DNA expression cassette of a), or the DNA sequence capable of forming a non-B DNA conformation in the target DNA sequence of b). In some embodiments, the origin of replication may be spaced at least 700 bp in a replication direction from the DNA sequence capable of forming a non-B DNA conformation in the target DNA expression cassette of a), or the DNA sequence capable of forming a non-B DNA conformation in the target DNA sequence of b).

The origin of replication enables the vector to replicate in a suitable host cell. The suitable host cell may be a prokaryotic cell such as *Escherichia coli,* or a eukaryotic cell. The origin of replication may be selected from a pMB1 origin of replication, a pBR322 origin of replication, a ColE1 origin of replication, an R6K origin of replication, a p15A origin of replication, a pSC101 origin of replication, a pUC origin of replication, a pET origin of replication, and derivatives of these origins of replication. These origin of replication derivatives may also enable the vector to replicate in a suitable host cell. In some embodiments, the origin of replication may be a pUC origin of replication.

The vector may further comprise a second origin of replication. The second origin of replication may be spaced at least 150 bp, 200 bp, 300 bp, 500 bp, or 700 bp in a replication direction from the DNA sequence capable of forming a non-B DNA conformation in the target DNA expression cassette of a), or the DNA sequence capable of forming a non-B DNA conformation in the target DNA sequence of b). The second origin of replication enables the vector to replicate in a suitable host cell. The suitable host cell may be a prokaryotic cell such as *Escherichia coli,* or a eukaryotic cell.

Depending on the type of origin of replication, the vector may further comprise a Rep protein expression element correspondingly, including a Rep protein-coding sequence and a corresponding promoter.

The vector may further comprise a first terminator and a second terminator. The first terminator and the second terminator are respectively located upstream and downstream of the target DNA expression cassette of a), and configured to terminate transcription outside the target DNA expression cassette of a) upstream and downstream of the target DNA expression cassette of a); or the first terminator and the second terminator are respectively located upstream and downstream of the target DNA sequence of b), and configured to terminate transcription outside the target DNA sequence of b) upstream and downstream of the target DNA sequence of b). The first terminator may be a forward terminator, a bidirectional terminator, or a reverse terminator. The second terminator may be a forward terminator, a bidirectional terminator, or a reverse terminator. In some embodiments, both the first terminator and the second terminator may be bidirectional terminators. The first terminator and the second terminator may be independently selected from an rrnB T1 terminator, an rrnB T2 terminator, a lambda t0 terminator, a TonB terminator, an Amp terminator, a T7 terminator, and a CYC1 terminator. In some embodiments, the first terminator may be an rrnB T1 terminator and an rrnB T2 terminator. In some embodiments, the second terminator may be a lambda t0 terminator.

The vector may further comprise a selectable marker. The selectable marker may be an antibiotic resistance gene, such as an ampicillin resistance gene, a tetracycline resistance gene, a chloramphenicol resistance gene, a kanamycin resistance gene, or a streptomycin resistance gene. In particular, the selectable marker is not present in the genome of the host cell into which the vector is to be transformed/transfected. In some embodiments, the selectable marker is a kanamycin resistance gene. The vector may further comprise a promoter for the expression of the selectable marker upstream of the selectable marker.

The vector may be a circular vector. The vector may be a non-viral vector. The vector may be a plasmid vector.

The vector does not comprise a lac promoter capable of inducing transcription.

The target DNA expression cassette of a) may comprise a type IIS restriction endonuclease (IIS enzyme) recognition site. The target DNA sequence of b) may comprise an IIS enzyme recognition site. Except for the target DNA expression cassette of a) or the target DNA sequence of b), the vector does not comprise the same IIS enzyme recognition site as those in the target DNA expression cassette of a) and the target DNA sequence of b).

The target DNA expression cassette of a) may be an expression cassette suitable for expressing the target DNA sequence in a eukaryotic cell, which may comprise components that enable expression of the target DNA sequence in a eukaryotic cell. The promoter comprised in the target DNA expression cassette of a) may be a promoter that enables expression of the target DNA sequence in a eukaryotic cell. The target DNA expression cassette of a) may further comprise an enhancer, a transcription terminator, etc., particularly an enhancer and a transcription terminator that enable expression of the target DNA sequence in a eukaryotic cell. The transcription terminator suitable for the expression of the target DNA sequence in eukaryotic cells may be selected from SV40 poly(A), hGH poly(A), BGH poly(A), and rbGlob poly(A). In some embodiments, the target DNA expression cassette of a) may be an expression cassette suitable for expressing mRNA in a eukaryotic cell, which may comprise an optional enhancer, a promoter, a 5' UTR, an open reading frame (ORF), a 3' UTR, a Poly(A) tail-coding sequence, and a transcription terminator.

The target DNA expression cassette of a) may further comprise a restriction site for vector linearization.

In some embodiments, the vector may comprise:
i) an origin of replication;
ii) a) a target DNA expression cassette, comprising a promoter and a target DNA sequence, wherein the target DNA sequence comprises a DNA sequence capable of forming a non-B DNA conformation, or b) a target DNA sequence, comprising a DNA sequence capable of forming a non-B DNA conformation; and
iii) a selectable marker and a promoter that controls the expression of the selectable marker,
wherein the origin of replication may be spaced at least 500 bp, or particularly at least 700 bp in a replication direction from the DNA sequence capable of forming a non-B DNA conformation in the target DNA expression cassette of a), or the DNA sequence capable of forming a non-B DNA conformation in the target DNA sequence of b).

In some embodiments, the vector may comprise:
i) an origin of replication;
ii) a first terminator;
iii) a) a target DNA expression cassette, comprising a promoter and a target DNA sequence, wherein the target DNA sequence comprises a DNA sequence capable of forming a non-B DNA conformation, or b) a target DNA sequence, comprising a DNA sequence capable of forming a non-B DNA conformation;
iv) a second terminator; and
v) a selectable marker and a promoter that controls the expression of the selectable marker,

wherein the first terminator and the second terminator may be respectively located upstream and downstream of the target DNA expression cassette of a), and configured to terminate transcription outside the target DNA expression cassette of a) in the vector upstream and downstream of the target DNA expression cassette of a); or the first terminator and the second terminator may be respectively located upstream and downstream of the target DNA sequence of b), and configured to terminate transcription outside the target DNA sequence of b) upstream and downstream of the target DNA sequence of b); and
the origin of replication may be spaced at least 500 bp or 700 bp in a replication direction from the DNA sequence capable of forming a non-B DNA conformation in the target DNA expression cassette of a), or the DNA sequence capable of forming a non-B DNA conformation in the target DNA sequence of b).

In some embodiments, the vector may sequentially comprise a pUC origin of replication, an rrnB T1 terminator and an rrnB T2 terminator, a target DNA sequence, a lambda t0 terminator, and a kanamycin resistance element.

In some embodiments, the vector may sequentially comprise a pUC origin of replication, an rrnB T1 terminator and an rrnB T2 terminator, a CMV enhancer, a CMV promoter, a target DNA sequence, a bGH poly(A) signal, a lambda t0 terminator, and a kanamycin resistance element.

In some embodiments, the vector may sequentially comprise a pUC origin of replication, an rrnB T1 terminator and an rrnB T2 terminator, a CMV enhancer, a CMV promoter, an mRNA functional region sequence comprising a poly(A) tail-coding sequence, a bGH poly(A) signal, a lambda t0 terminator, and a kanamycin resistance element. In some embodiments, the vector may sequentially comprise a pUC origin of replication, an rrnB T1 terminator and an rrnB T2 terminator, a CMV enhancer, a CMV promoter, a 5' UTR, an open reading frame (ORF), a 3' UTR, a Poly(A) tail-coding sequence, a vector linearization site, a bGH poly(A) signal, a lambda t0 terminator, and a kanamycin resistance element.

Correspondingly, the present application provides a vector backbone, which may comprise:
i) an origin of replication; and
ii) a) a cloning site, b) a cloning site and a DNA sequence capable of forming a non-B DNA conformation, c) a DNA expression cassette, comprising a promoter and a cloning site, or d) a DNA expression cassette, comprising a promoter, a cloning site, and a DNA sequence capable of forming a non-B DNA conformation.

The DNA sequence capable of forming a non-B DNA conformation may be a direct repeat sequence, an inverted repeat sequence, or a homopurine-homopyrimidine sequence, including but not limited to polyadenine (poly(A)), polyguanine (poly(G)), polycytosine (poly(C)), polythymine (poly(T)), an shRNA-coding sequence, and an ITR sequence. The direct repeat sequence may be a poly(A) tail-coding sequence. The homopurine-homopyrimidine sequence may be a poly(A) tail-coding sequence. The inverted repeat sequence may be an ITR or a derivative sequence thereof. In some embodiments, the DNA sequence capable of forming a non-B DNA conformation in the DNA expression cassette of d) may be a poly(A) tail-coding sequence.

The origin of replication may be spaced at least 150 bp, 200 bp, 300 bp, 500 bp, or 700 bp in a replication direction from the cloning site of a), the DNA sequence capable of forming a non-B DNA conformation of b), the cloning site in the DNA expression cassette of c), or the DNA sequence capable of forming a non-B DNA conformation in the DNA expression cassette of d). In some embodiments, the origin of replication may be spaced at least 500 bp in a replication direction from the cloning site of a), the DNA sequence capable of forming a non-B DNA conformation of b), the cloning site in the DNA expression cassette of c), or the DNA sequence capable of forming a non-B DNA conformation in the DNA expression cassette of d). In some embodiments, the origin of replication may be spaced at least 700 bp in a replication direction from the cloning site of a), the DNA sequence capable of forming a non-B DNA conformation of b), the cloning site in the DNA expression cassette of c), or the DNA sequence capable of forming a non-B DNA conformation in the DNA expression cassette of d).

The origin of replication enables the vector to replicate in a suitable host cell. The suitable host cell may be a prokaryotic cell such as *Escherichia coli,* or a eukaryotic cell. The origin of replication may be selected from a pMB1 origin of replication, a pBR322 origin of replication, a ColE1 origin of replication, an R6K origin of replication, a p15A origin of replication, a pSC101 origin of replication, a pUC origin of replication, a pET origin of replication, and derivatives of these origins of replication. These origin of replication derivatives may also enable the vector to replicate in a suitable host cell. In some embodiments, the origin of replication may be a pUC origin of replication.

The vector backbone may further comprise a second origin of replication. The second origin of replication may be spaced at least 150 bp, 200 bp, 300 bp, 500 bp, or 700 bp in a replication direction from the cloning site of a), the DNA sequence capable of forming a non-B DNA conformation of b), the cloning site in the DNA expression cassette of c), or the DNA sequence capable of forming a non-B DNA conformation in the DNA expression cassette of d). The second origin of replication enables the vector to replicate in a suitable host cell. The suitable host cell may be a prokaryotic cell such as *Escherichia coli,* or a eukaryotic cell.

Depending on the type of origin of replication, the vector may further comprise a Rep protein expression element correspondingly, including a Rep protein-coding sequence and a corresponding promoter.

The vector backbone may further comprise a first terminator and a second terminator. The first terminator and the second terminator are respectively located upstream and downstream of the cloning site of a), and configured to terminate transcription outside the cloning site of a) in the vector upstream and downstream of the cloning site of a); the first terminator and the second terminator are respectively located upstream and downstream of the cloning site and the DNA sequence capable of forming a non-B DNA conformation of b), and configured to terminate transcription outside the cloning site and the DNA sequence capable of forming a non-B DNA conformation in b) in the vector upstream and downstream of the cloning site and the DNA sequence capable of forming a non-B DNA conformation of b); the first terminator and the second terminator are respectively located upstream and downstream of the DNA expression cassette of c), and configured to terminate transcription of the DNA expression cassette of c) in the vector upstream and downstream of the DNA expression cassette of c); or the first terminator and the second terminator are respectively located upstream and downstream of the DNA expression cassette of d), and configured to terminate transcription outside the DNA expression cassette of d) in the vector upstream and downstream of the DNA expression cassette of d). The first terminator may be a forward terminator, a bidirectional terminator, or a reverse terminator. The second terminator may be a forward terminator, a bidirectional terminator, or a reverse terminator. In some embodiments, both the first terminator and the second terminator may be bidirectional terminators. The first terminator and the second terminator may be independently selected from an rrnB T1 terminator, an rrnB T2 terminator, a lambda t0 terminator, a TonB terminator, an Amp terminator, a T7 terminator, and a CYC1 terminator. In some embodiments, the first terminator may be an rrnB T1 terminator and an rrnB T2 terminator. In some embodiments, the second terminator may be a lambda t0 terminator.

The cloning site of a) in the vector backbone may be a multiple cloning site. The cloning site of b) in the vector backbone may be a multiple cloning site. The cloning site in the DNA expression cassette of c) in the vector backbone may be a multiple cloning site. The cloning site in the DNA expression cassette of d) in the vector may be a multiple cloning site.

Any sequence may be inserted at the cloning site of a) in the vector backbone, particularly a target DNA sequence, or a promoter and a target DNA sequence, wherein the target DNA sequence may comprise a DNA sequence capable of forming a non-B DNA conformation. Any sequence may be inserted at the cloning site of b) in the vector backbone. Any sequence may be inserted at the cloning site in the DNA expression cassette of c) in the vector backbone, particularly a target DNA sequence, wherein the target DNA sequence may comprise a DNA sequence capable of forming a non-B DNA conformation. Any sequence may be inserted at the cloning site in the DNA expression cassette of d) in the vector backbone. In some embodiments, the DNA sequence capable of forming a non-B DNA conformation in the DNA expression cassette of d) may be a poly(A)-coding sequence, and an mRNA-coding sequence may be inserted at the cloning site in the DNA expression cassette of d).

The vector backbone may further comprise a selectable marker. The selectable marker may be an antibiotic resistance gene, such as an ampicillin resistance gene, a tetracycline resistance gene, a chloramphenicol resistance gene, a kanamycin resistance gene, or a streptomycin resistance gene. In particular, the selectable marker is not present in the genome of the host cell into which the vector is to be transformed/transfected. In some embodiments, the selectable marker is a kanamycin resistance gene. The vector may further comprise a promoter for the expression of the selectable marker upstream of the selectable marker.

The vector backbone may be a circular vector backbone. The vector may be a non-viral vector backbone. The vector may be a plasmid vector backbone.

The vector backbone does not comprise a lac promoter capable of inducing transcription.

The sequence to be incorporated into the cloning site of a), the DNA sequence capable of forming a non-B DNA conformation of b), the sequence to be incorporated into the cloning site of the DNA expression cassette of c), or the DNA sequence capable of forming a non-B DNA conformation in the DNA expression cassette of d) may comprise an IIS enzyme recognition site. Except for the above sequences, the vector backbone does not comprise the same IIS enzyme recognition site as those in a) to d).

The DNA expression cassettes of c) and d) may be expression cassettes suitable for expressing DNA sequences in eukaryotic cells, which may comprise components that enable expression of the DNA sequence incorporated into the cloning site, or the DNA sequence incorporated into the cloning site and the DNA sequence capable of forming a non-B DNA conformation, in a eukaryotic cell. The promoter comprised in the DNA expression cassettes of c) and d) may be a promoter that enables expression of the DNA sequence incorporated into the cloning site, or the DNA sequence incorporated into the cloning site and the DNA sequence capable of forming a non-B DNA conformation, in a eukaryotic cell. The DNA expression cassettes of c) and d) may further comprise an enhancer, a transcription terminator, etc., particularly an enhancer and a transcription terminator that enable expression of the DNA sequence incorporated into the cloning site, or the DNA sequence incorporated into the cloning site and the DNA sequence capable of forming a non-B DNA conformation, in a eukaryotic cell. The transcription terminator suitable for expression of the DNA sequence in a eukaryotic cell may be selected from SV40 poly(A), hGH poly(A), BGH poly(A), and rbGlob poly(A). In some embodiments, the DNA expression cassette of d) may be an expression cassette suitable for expressing mRNA in a eukaryotic cell, wherein the DNA sequence capable of forming a non-B DNA conformation may be a poly(A) tail-coding sequence, and the DNA expression cassette may comprise an optional enhancer, a promoter, a 5' UTR, a cloning site, a 3' UTR, a poly(A) tail-coding sequence, and a transcription terminator. The DNA expression cassette of c) or d) may further comprise a restriction site for vector linearization.

In some embodiments, the vector backbone may comprise:
i) an origin of replication;
ii) a) a cloning site, b) a cloning site and a DNA sequence capable of forming a non-B DNA conformation, c) a DNA expression cassette, comprising a promoter and a cloning site, or d) a DNA expression cassette, comprising a promoter, a cloning site, and a DNA sequence capable of forming a non-B DNA conformation; and
iii) a selectable marker and a promoter that controls the expression of the selectable marker,
wherein the origin of replication may be spaced at least 500 bp or 700 bp in a replication direction from the cloning site of a), the DNA sequence capable of forming a non-B DNA conformation of b), the cloning site in the DNA expression cassette of c), or the DNA sequence capable of forming a non-B DNA conformation in the DNA expression cassette of d).

In some embodiments, the vector backbone may comprise:
i) an origin of replication;
ii) a first terminator;
iii) a) a cloning site, b) a cloning site and a DNA sequence capable of forming a non-B DNA conformation, c) a DNA expression cassette, comprising a promoter and a cloning site, or d) a DNA expression cassette, comprising a promoter, a cloning site, and a DNA sequence capable of forming a non-B DNA conformation;
iv) a second terminator; and
v) a selectable marker and a promoter that controls the expression of the selectable marker,

wherein the first terminator and the second terminator may be respectively located upstream and downstream of the cloning site of a), and configured to terminate transcription outside the cloning site of a) in the vector upstream and downstream of the cloning site of a);
the first terminator and the second terminator may be respectively located upstream and downstream of the cloning site and the DNA sequence capable of forming a non-B DNA conformation of b), and configured to terminate transcription outside the cloning site and the DNA sequence capable of forming a non-B DNA conformation in b) in the vector upstream and downstream of the cloning site and the DNA sequence capable of forming a non-B DNA conformation of b);
the first terminator and the second terminator may be respectively located upstream and downstream of the DNA expression cassette of c), and configured to terminate transcription outside the DNA expression cassette of c) in the vector upstream and downstream of the DNA expression cassette of c); or
the first terminator and the second terminator may be respectively located upstream and downstream of the DNA expression cassette of d), and configured to terminate transcription outside the DNA expression cassette of d) in the vector upstream and downstream of the DNA expression cassette of d); and
the origin of replication may be spaced at least 500 bp or 700 bp in a replication direction from the cloning site of a), the DNA sequence capable of forming a non-B DNA conformation of b), the cloning site in the DNA expression cassette of c), or the DNA sequence capable of forming a non-B DNA conformation in the DNA expression cassette of d).

In some embodiments, the vector backbone may sequentially comprise a pUC origin of replication, an rrnB T1 terminator and an rrnB T2 terminator, a cloning site, a lambda t0 terminator, and a kanamycin resistance element.

In some embodiments, the vector backbone may sequentially comprise a pUC origin of replication, an rrnB T1 terminator and an rrnB T2 terminator, a cloning site, a target DNA sequence, a lambda t0 terminator, and a kanamycin resistance element.

In some embodiments, the vector backbone may sequentially comprise a pUC origin of replication, an rrnB T1 terminator and an rrnB T2 terminator, a CMV enhancer, a CMV promoter, a cloning site, a bGH poly(A) signal, a lambda t0 terminator, and a kanamycin resistance element.

In some embodiments, the vector backbone may sequentially comprise a pUC origin of replication, an rrnB T1 terminator and an rrnB T2 terminator, a CMV enhancer, a CMV promoter, a cloning site, a poly(A) tail-coding sequence, a vector linearization site, a bGH poly(A) signal, a lambda t0 terminator, and a kanamycin resistance element. In some embodiments, the vector backbone may sequentially comprise a pUC origin of replication, an rrnB T1 terminator and an rrnB T2 terminator, a CMV enhancer, a CMV promoter, a 5' UTR, a cloning site, a 3' UTR, a poly(A) tail-coding sequence, a vector linearization site, a bGH poly(A) signal, a lambda t0 terminator, and a kanamycin resistance element.

In a second aspect, the present application provides a vector, which may comprise:
i) an origin of replication; and
ii) a) a target DNA expression cassette, sequentially comprising a first ITR or a derivative sequence thereof, a promoter, a target DNA sequence, and a second ITR or a derivative sequence thereof; or b) a target DNA sequence set, sequentially comprising a first ITR or a derivative sequence thereof, a target DNA sequence, and a second ITR or a derivative sequence thereof.

The origin of replication may be spaced at least 150 bp, 200 bp, 300 bp, 500 bp, 700 bp, 800 bp, 900 bp, 1000 bp, 1100 bp, 1200 bp, 1300 bp, 1400 bp, 1500 bp, 1600 bp, or 1700 bp in a replication direction from the target DNA expression cassette of a) or the target DNA sequence set of b). In some embodiments, the origin of replication may be spaced at least 300 bp in a replication direction from the target DNA expression cassette of a) or the target DNA sequence set of b). In some embodiments, the origin of replication may be spaced at least 500 bp in a replication direction from the target DNA expression cassette of a) or the target DNA sequence set of b). In some embodiments, the origin of replication may be spaced at least 700 bp in a replication direction from the target DNA expression cassette of a) or the target DNA sequence set of b).

The origin of replication enables the vector to replicate in a suitable host cell. The suitable host cell may be a prokaryotic cell such as *Escherichia coli,* or a eukaryotic cell. The origin of replication may be selected from a pMB1 origin of replication, a pBR322 origin of replication, a ColE1 origin of replication, an R6K origin of replication, a p15A origin of replication, a pSC101 origin of replication, a pUC origin of replication, a pET origin of replication, and derivatives of these origins of replication. These origin of replication derivatives may also enable the vector to replicate in a suitable host cell. In some embodiments, the origin of replication may be a pUC origin of replication.

The vector may further comprise a second origin of replication. The second origin of replication may be spaced at least 150 bp, 200 bp, 300 bp, 500 bp, 700 bp, 800 bp, 900 bp, 1000 bp, 1100 bp, 1200 bp, 1300 bp, 1400 bp, 1500 bp, 1600 bp, or 1700 bp in a replication direction from the target DNA expression cassette of a) or the target DNA sequence set of b). The second origin of replication enables the vector to replicate in a suitable host cell. The suitable host cell may be a prokaryotic cell such as *Escherichia coli,* or a eukaryotic cell.

Depending on the type of origin of replication, the vector may further comprise a Rep protein expression element correspondingly, including a Rep protein-coding sequence and a corresponding promoter.

The vector may further comprise a first terminator and a second terminator. The first terminator and the second terminator are respectively located upstream and downstream of the target DNA expression cassette of a), and configured to terminate transcription outside the target DNA expression cassette of a) in the vector upstream and downstream of the target DNA expression cassette of a); or the first terminator and the second terminator are respectively located upstream and downstream of the target DNA sequence set of b), and configured to terminate transcription outside the target DNA sequence set of b) upstream and downstream of the target DNA sequence set of b) in the vector. The first terminator may be a forward terminator, a bidirectional terminator, or a reverse terminator. The second terminator may be a forward terminator, a bidirectional terminator, or a reverse terminator. In some embodiments, both the first terminator and the second terminator may be bidirectional terminators. The first terminator and the second terminator may be independently selected from an rrnB T1 terminator, an rrnB T2 terminator, a lambda t0 terminator, a TonB terminator, an Amp terminator, a T7 terminator, and a CYC1 terminator. In some embodiments, the first terminator may be an rrnB T1 terminator and an rrnB T2 terminator. In some embodiments, the second terminator may be a lambda t0 terminator.

The vector may further comprise a selectable marker. The selectable marker may be an antibiotic resistance gene, such as an ampicillin resistance gene, a tetracycline resistance gene, a chloramphenicol resistance gene, a kanamycin resistance gene, or a streptomycin resistance gene. In particular, the selectable marker is not present in the genome of the host cell into which the vector is to be transformed/transfected. In some embodiments, the selectable marker is a kanamycin resistance gene. The vector may further comprise a promoter for the expression of the selectable marker upstream of the selectable marker.

The first ITR and the second ITR may be wild-type AAV-ITRs, comprising, for example, a nucleotide sequence set forth in SEQ ID NO: 9.

The derivative sequence of the first ITR and the derivative sequence of the second ITR may form a palindromic structure similar to that of a native AAV-ITR, comprising, for example, one palindromic arm and one palindromic stem, or only one palindromic stem, while retaining Rep protein-binding ability. In some embodiments, the derivative sequence of the first ITR and the derivative sequence of the second ITR may comprise a nucleotide sequence set forth in SEQ ID NO: 10, 11, or 12.

The first ITR or the derivative sequence thereof, and the second ITR or the derivative sequence thereof, may comprise the same or different nucleotide sequences.

The vector may be a circular vector. The vector may be a non-viral vector. The vector may be a plasmid vector.

The vector does not comprise a lac promoter capable of inducing transcription.

The target DNA expression cassette of a) may comprise a type IIS restriction endonuclease (IIS enzyme) recognition site. The target DNA sequence set of b) may comprise an IIS enzyme recognition site. Except for the target DNA expression cassette of a) or the target DNA sequence set of b), the vector does not comprise the same IIS enzyme recognition site as those in the target DNA expression cassette of a) and the target DNA sequence set of b). The target DNA sequence may be any DNA sequence to be expressed, such as an mRNA-coding sequence and a poly(A) tail-coding sequence. The target DNA sequence may comprise a DNA sequence capable of forming a non-B DNA conformation. The target DNA sequence may comprise a cloning site, for example, a multiple cloning site.

The target DNA expression cassette of a) may be an expression cassette suitable for expressing the target DNA sequence in a eukaryotic cell, which may comprise components that enable expression of the target DNA sequence in a eukaryotic cell. The promoter comprised in the target DNA expression cassette of a) may be a promoter that enables expression of the target DNA sequence in a eukaryotic cell. The target DNA expression cassette of a) may further comprise an enhancer, a transcription terminator, etc., particularly an enhancer and a transcription terminator that enable expression of the target DNA sequence in a eukaryotic cell. The transcription terminator suitable for the expression of the target DNA sequence in eukaryotic cells may be selected from SV40 poly(A), hGH poly(A), BGH poly(A), and rbGlob poly(A). In some embodiments, the target DNA expression cassette of a) may be an expression cassette suitable for expressing mRNA in a eukaryotic cell, which may comprise an optional enhancer, a promoter, a 5' UTR, an open reading frame (ORF), a 3' UTR, a Poly(A) tail-coding sequence, and a transcription terminator. The target DNA expression cassette of a) may further comprise a restriction site for vector linearization.

In some embodiments, the vector may comprise:
i) an origin of replication;
ii) a) a target DNA expression cassette, sequentially comprising a first ITR or a derivative sequence thereof, a promoter, a target DNA sequence, and a second ITR or a derivative sequence thereof; or b) a target DNA sequence set, sequentially comprising a first ITR or a derivative sequence thereof, a target DNA sequence, and a second ITR or a derivative sequence thereof; and
iii) a selectable marker and a promoter that controls the expression of the selectable marker,
wherein the origin of replication may be spaced at least 500 bp or 700 bp in a replication direction from the target DNA expression cassette of a) or the target DNA sequence set of b).

In some embodiments, the vector backbone may comprise:
i) an origin of replication;
ii) a first terminator;
iii) a) a target DNA expression cassette, sequentially comprising a first ITR or a derivative sequence thereof, a promoter, a target DNA sequence, and a second ITR or a derivative sequence thereof; or b) a target DNA sequence set, sequentially comprising a first ITR or a derivative sequence thereof, a target DNA sequence, and a second ITR or a derivative sequence thereof;
iv) a second terminator; and
v) a selectable marker and a promoter that controls the expression of the selectable marker,

wherein the first terminator and the second terminator are respectively located upstream and downstream of the target DNA expression cassette of a), and configured to terminate transcription outside the target DNA expression cassette of a) in the vector upstream and downstream of the target DNA expression cassette of a); or the first terminator and the second terminator are respectively located upstream and downstream of the target DNA sequence set of b), and configured to terminate transcription outside the target DNA sequence set of b) in the vector upstream and downstream of the target DNA sequence set of b); and
the origin of replication may be spaced at least 500 bp or 700 bp in a replication direction from the DNA expression cassette of a) or the target DNA sequence set of b).

In some embodiments, the vector may sequentially comprise a pUC origin of replication, an rrnB T1 terminator and an rrnB T2 terminator, a first ITR or a derivative sequence thereof, a CMV enhancer, a chicken β-actin promoter, a target DNA sequence, a bGH poly(A) signal, a second ITR or a derivative sequence thereof, a lambda t0 terminator, and a kanamycin resistance element.

Correspondingly, the present application provides a vector backbone, which may comprise:
i) an origin of replication; and
ii) a) a DNA sequence set, sequentially comprising a first ITR or a derivative sequence thereof, a cloning site, and a second ITR or a derivative sequence thereof; or b) a DNA expression cassette, sequentially comprising a first ITR or a derivative sequence thereof, a promoter, a cloning site, and a second ITR or a derivative sequence thereof.

The origin of replication may be spaced at least 150 bp, 200 bp, 300 bp, 500 bp, 700 bp, 800 bp, 900 bp, 1000 bp, 1100 bp, 1200 bp, 1300 bp, 1400 bp, 1500 bp, 1600 bp, or 1700 bp in a replication direction from the DNA sequence set of a) or the DNA expression cassette of b). In some embodiments, the origin of replication may be spaced at least 500 bp in a replication direction from the DNA sequence set of a) or the DNA expression cassette of b). In some embodiments, the origin of replication may be spaced at least 700 bp in a replication direction from the DNA sequence set of a) or the DNA expression cassette of b).

The origin of replication enables the vector to replicate in a suitable host cell. The suitable host cell may be a prokaryotic cell such as *Escherichia coli,* or a eukaryotic cell. The origin of replication may be selected from a pMB1 origin of replication, a pBR322 origin of replication, a ColE1 origin of replication, an R6K origin of replication, a p15A origin of replication, a pSC101 origin of replication, a pUC origin of replication, a pET origin of replication, and derivatives of these origins of replication. These origin of replication derivatives may also enable the vector to replicate in a suitable host cell. In some embodiments, the origin of replication may be a pUC origin of replication.

The vector backbone may further comprise a second origin of replication. The second origin of replication may be spaced at least 150 bp, 200 bp, 300 bp, 500 bp, 700 bp, 800 bp, 900 bp, 1000 bp, 1100 bp, 1200 bp, 1300 bp, 1400 bp, 1500 bp, 1600 bp, or 1700 bp in a replication direction from the DNA sequence set of a) or the DNA expression cassette of b). The second origin of replication enables the vector to replicate in a suitable host cell. The suitable host cell may be a prokaryotic cell such as *Escherichia coli,* or a eukaryotic cell.

Depending on the type of origin of replication, the vector may further comprise a Rep protein expression element correspondingly, including a Rep protein-coding sequence and a corresponding promoter.

The vector backbone may further comprise a first terminator and a second terminator. The first terminator and the second terminator are respectively located upstream and downstream of the DNA sequence set of a), and configured to terminate transcription outside the DNA sequence set of a) in the vector upstream and downstream of the DNA sequence set of a); or the first terminator and the second terminator are respectively located upstream and downstream of the DNA expression cassette of b), and configured to terminate transcription outside the DNA expression cassette of b) in the vector upstream and downstream of the DNA expression cassette of b). The first terminator may be a forward terminator, a bidirectional terminator, or a reverse terminator. The second terminator may be a forward terminator, a bidirectional terminator, or a reverse terminator. In some embodiments, both the first terminator and the second terminator may be bidirectional terminators. The first terminator and the second terminator may be independently selected from an rrnB T1 terminator, an rrnB T2 terminator, a lambda t0 terminator, a TonB terminator, an Amp terminator, a T7 terminator, and a CYC1 terminator. In some embodiments, the first terminator may be an rrnB T1 terminator and an rrnB T2 terminator. In some embodiments, the second terminator may be a lambda t0 terminator.

The cloning site in the DNA sequence set of a) in the vector backbone may be a multiple cloning site. The cloning site in the DNA expression cassette of b) in the vector backbone may be a multiple cloning site.

Any sequence may be inserted at the cloning site in the DNA sequence set of a), particularly a target DNA sequence, or a promoter and a target DNA sequence. The target DNA sequence may be any DNA sequence to be expressed, such as an mRNA-coding sequence.

Any sequence may be inserted at the cloning site in the DNA expression cassette of b), particularly a target DNA sequence. The target DNA sequence may be any DNA sequence to be expressed, such as an mRNA-coding sequence.

The vector backbone may further comprise a selectable marker. The selectable marker may be an antibiotic resistance gene, such as an ampicillin resistance gene, a tetracycline resistance gene, a chloramphenicol resistance gene, a kanamycin resistance gene, or a streptomycin resistance gene. In particular, the selectable marker is not present in the genome of the host cell into which the vector is to be transformed/transfected. In some embodiments, the selectable marker is a kanamycin resistance gene. The vector may further comprise a promoter for the expression of the selectable marker upstream of the selectable marker.

The first ITR and the second ITR may be wild-type AAV-ITRs, comprising, for example, a nucleotide sequence set forth in SEQ ID NO: 9.

The derivative sequence of the first ITR and the derivative sequence of the second ITR may form a palindromic structure similar to that of a native AAV-ITR, comprising, for example, one palindromic arm and one palindromic stem, or only one palindromic stem, while retaining Rep protein-binding ability. In some embodiments, the derivative sequence of the first ITR and the derivative sequence of the second ITR may comprise a nucleotide sequence set forth in SEQ ID NO: 10, 11, or 12.

The first ITR or the derivative sequence thereof, and the second ITR or the derivative sequence thereof, may comprise the same or different nucleotide sequences.

The vector backbone may be a circular vector backbone. The vector backbone may be a non-viral vector backbone. The vector backbone may be a plasmid vector backbone.

The vector backbone does not comprise a lac promoter capable of inducing transcription.

The DNA expression cassette of b) may be an expression cassette suitable for expressing the DNA sequence in a eukaryotic cell, which may comprise components that enable expression of the DNA sequence in a eukaryotic cell. The promoter comprised in the DNA expression cassette of b) may be a promoter that enables expression of the DNA sequence in a eukaryotic cell. The DNA expression cassette of b) may further comprise an enhancer, a transcription terminator, etc., particularly an enhancer and a transcription terminator that enable expression of the DNA sequence in a eukaryotic cell. The transcription terminator suitable for expression of the DNA sequence in a eukaryotic cell may be selected from SV40 poly(A), hGH poly(A), BGH poly(A), and rbGlob poly(A). In some embodiments, the DNA expression cassette of b) may be an expression cassette suitable for expressing mRNA in a eukaryotic cell, which may comprise an optional enhancer, a promoter, a 5' UTR, an open reading frame (ORF), a 3' UTR, and a transcription terminator. The DNA expression cassette of b) may further comprise a restriction site for vector linearization.

In some embodiments, the vector backbone may comprise:
i) an origin of replication;
ii) a) a DNA sequence set, sequentially comprising a first ITR or a derivative sequence thereof, a cloning site, and a second ITR or a derivative sequence thereof; or b) a DNA expression cassette, sequentially comprising a first ITR or a derivative sequence thereof, a promoter, a cloning site, and a second ITR or a derivative sequence thereof; and
iii) a selectable marker and a promoter that controls the expression of the selectable marker,
wherein the origin of replication may be spaced at least 500 bp or 700 bp in a replication direction from the DNA sequence set of a) or the DNA expression cassette of b).

In some embodiments, the vector backbone may comprise:
i) an origin of replication;
ii) a first terminator;
iii) a) a DNA sequence set, sequentially comprising a first ITR or a derivative sequence thereof, a cloning site, and a second ITR or a derivative sequence thereof; or b) a DNA expression cassette, sequentially comprising a first ITR or a derivative sequence thereof, a promoter, a cloning site, and a second ITR or a derivative sequence thereof;
iv) a second terminator; and
v) a selectable marker and a promoter that controls the expression of the selectable marker,

wherein the first terminator and the second terminator are respectively located upstream and downstream of the DNA sequence set of a), and configured to terminate transcription outside the DNA sequence set of a) in the vector upstream and downstream of the DNA sequence set of a); or
the first terminator and the second terminator are respectively located upstream and downstream of the DNA expression cassette of b), and configured to terminate transcription outside the DNA expression cassette of b) in the vector upstream and downstream of the DNA expression cassette of b); and
the origin of replication may be spaced at least 500 bp or 700 bp in a replication direction from the DNA sequence set of a) or the DNA expression cassette of b).

In some embodiments, the vector backbone may sequentially comprise a pUC origin of replication, an rrnB T1 terminator and an rrnB T2 terminator, a first ITR or a derivative sequence thereof, a CMV enhancer, a chicken β-actin promoter, a cloning site, a bGH poly(A) signal, a second ITR or a derivative sequence thereof, a lambda t0 terminator, and a kanamycin resistance element.

In a third aspect, the present application provides a method for preparing the vector of the present application, which method comprises synthesizing the vector *de novo* according to the design of the vector backbone of the present application described above.

The present application provides a method for preparing the vector backbone of the present application, which method comprises synthesizing the vector *de novo* according to the design of the vector backbone of the present application described above.

The present application further provides a method for modifying a vector or vector backbone, which method comprises i) providing a vector or vector backbone, and ii) modifying the vector or vector backbone, thereby ensuring that the modified vector or vector backbone comprises:
a) an origin of replication;
b) a first terminator;
c) c-i) a cloning site,
   c-ii) a DNA expression cassette, comprising a promoter and a cloning site,
   c-iii) a DNA expression cassette, comprising a promoter, a cloning site, and a DNA sequence capable of forming a non-B DNA conformation,
   c-iv) a target DNA sequence, comprising a DNA sequence capable of forming a non-B DNA conformation,
   c-v) a target DNA expression cassette, comprising a promoter and a target DNA sequence, wherein the target DNA sequence comprises a DNA sequence capable of forming a non-B DNA conformation,
   c-vi) a DNA sequence set, sequentially comprising a first ITR or a derivative sequence thereof, a cloning site, and a second ITR or a derivative sequence thereof,
   c-vii) a DNA expression cassette, sequentially comprising a first ITR or a derivative sequence thereof, a promoter, a cloning site, and a second ITR or a derivative sequence thereof,
   c-viii) a target DNA expression cassette, sequentially comprising a first ITR or a derivative sequence thereof, a promoter, a target DNA sequence, and a second ITR or a derivative sequence thereof, or
   c-ix) a target DNA sequence set, sequentially comprising a first ITR or a derivative sequence thereof, a target DNA sequence, and a second ITR or a derivative sequence thereof;
d) a second terminator; and
e) a selectable marker and a promoter that controls the expression of the selectable marker,

wherein the first terminator and the second terminator are respectively located upstream and downstream of the sequence of c), and configured to terminate transcription outside the sequence of c) upstream and downstream of the sequence of c);
optionally, the origin of replication is spaced at least 150 bp, 200 bp, 300 bp, 500 bp, or 700 bp in a replication direction from the cloning site of c-i), the cloning site in the DNA expression cassette of c-ii), the DNA sequence capable of forming a non-B DNA conformation in the DNA expression cassette of c-iii), the DNA sequence capable of forming a non-B DNA conformation in the target DNA sequence of c-iv), the DNA sequence capable of forming a non-B DNA conformation in the target DNA expression cassette of c-v), the DNA sequence set of c-vi), the DNA expression cassette of c-vii), the target DNA expression cassette of c-viii), or the target DNA sequence set of c-ix); and
optionally, the vector does not comprise an active lac promoter.

The cloning site in the sequence of c) may be a multiple cloning site.

Specifically, the present application further provides a method for modifying a vector or vector backbone, which method comprises:
i) providing a vector or vector backbone, comprising a) an origin of replication, and b) b-i) a cloning site; b-ii) a DNA expression cassette, comprising a promoter and a cloning site; b-iii) a DNA expression cassette, comprising a promoter, a cloning site, and a DNA sequence capable of forming a non-B DNA conformation; b-iv) a target DNA sequence, comprising a DNA sequence capable of forming a non-B DNA conformation; b-v) a target DNA expression cassette, comprising a promoter and a target DNA sequence, wherein the target DNA sequence comprises a DNA sequence capable of forming a non-B DNA conformation; b-vi) a DNA sequence set, sequentially comprising a first ITR or a derivative sequence thereof, a cloning site, and a second ITR or a derivative sequence thereof; b-vii) a DNA expression cassette, sequentially comprising a first ITR or a derivative sequence thereof, a promoter, a cloning site, and a second ITR or a derivative sequence thereof; b-viii) a target DNA expression cassette, sequentially comprising a first ITR or a derivative sequence thereof, a promoter, a target DNA sequence, and a second ITR or a derivative sequence thereof; or b-ix) a target DNA sequence set, sequentially comprising a first ITR or a derivative sequence thereof, a target DNA sequence, and a second ITR or a derivative sequence thereof; and
ii) incorporating a first terminator and a second terminator into the vector or vector backbone, wherein the first terminator and the second terminator are respectively located upstream and downstream of the sequence of b), and configured to terminate transcription outside the sequence of b) upstream and downstream of the sequence of b).

The method may further comprise spacing the origin of replication at least 150 bp, 200 bp, 300 bp, 500 bp, or 700 bp in a replication direction from the cloning site of b-i), the cloning site in the DNA expression cassette of b-ii), the DNA sequence capable of forming a non-B DNA conformation in the DNA expression cassette of b-iii), the DNA sequence capable of forming a non-B DNA conformation in the target DNA sequence of b-iv), the DNA sequence capable of forming a non-B DNA conformation in the target DNA expression cassette of b-v), the DNA sequence set of b-vi), the DNA expression cassette of b-vii), the target DNA expression cassette of b-viii), or the target DNA sequence set of b-ix).

In the case that the vector or vector backbone further comprises a lac promoter, the method further comprises inactivating the lac promoter.

The DNA sequence capable of forming a non-B DNA conformation may be a direct repeat sequence, an inverted repeat sequence, or a homopurine-homopyrimidine sequence, including but not limited to polyadenine (poly(A)), polyguanine (poly(G)), polycytosine (poly(C)), polythymine (poly(T)), an shRNA-coding sequence, and an ITR sequence. The direct repeat sequence may be a poly(A) tail-coding sequence. The homopurine-homopyrimidine sequence may be a poly(A) tail-coding sequence. The inverted repeat sequence may be an ITR or a derivative sequence thereof. In some embodiments, the DNA sequence capable of forming a non-B DNA conformation may be a poly(A)-coding sequence, an ITR, or a derivative sequence thereof.

The origin of replication enables the vector or vector backbone to replicate in a suitable host cell. The suitable host cell may be a prokaryotic cell such as *Escherichia coli,* or a eukaryotic cell. The origin of replication may be selected from a pMB1 origin of replication, a pBR322 origin of replication, a ColE1 origin of replication, an R6K origin of replication, a p15A origin of replication, a pSC101 origin of replication, a pUC origin of replication, a pET origin of replication, and derivatives of these origins of replication. These origin of replication derivatives may also enable the vector to replicate in a suitable host cell. In some embodiments, the origin of replication may be a pUC origin of replication.

Depending on the type of origin of replication, the vector may further comprise a Rep protein expression element correspondingly, including a Rep protein-coding sequence and a corresponding promoter.

The first terminator may be a forward terminator, a bidirectional terminator, or a reverse terminator. The second terminator may be a forward terminator, a bidirectional terminator, or a reverse terminator. In some embodiments, both the first terminator and the second terminator may be bidirectional terminators. The first terminator and the second terminator may be independently selected from an rrnB T1 terminator, an rrnB T2 terminator, a lambda t0 terminator, a TonB terminator, an Amp terminator, a T7 terminator, and a CYC1 terminator. In some embodiments, the first terminator may be an rrnB T1 terminator and an rrnB T2 terminator. In some embodiments, the second terminator may be a lambda t0 terminator.

The vector or vector backbone may comprise a selectable marker. The selectable marker may be an antibiotic resistance gene, such as an ampicillin resistance gene, a tetracycline resistance gene, a chloramphenicol resistance gene, a kanamycin resistance gene, or a streptomycin resistance gene. In particular, the selectable marker is not present in the genome of the host cell into which the vector is to be transformed/transfected. In some embodiments, the selectable marker is a kanamycin resistance gene. The vector may further comprise a promoter for the expression of the selectable marker upstream of the selectable marker.

The vector or vector backbone may be circular. The vector or vector backbone may be of non-viral origin. The vector or vector backbone may be a plasmid vector or a plasmid vector backbone.

In a fourth aspect, the present application provides a composition, comprising the vector backbone or vector of the present application, including the vector backbone or vector prepared by the method of the present application and the vector backbone or vector modified by the method of the present application.

In a fifth aspect, the present application provides the use of the vector backbone or vector of the present application, including those prepared by the method of the present application and those modified by the method of the present application, in replicating and/or expressing a target DNA sequence, particularly a DNA sequence comprising a DNA sequence capable of forming a non-B DNA conformation (e.g., an ITR or a poly(A) tail-coding sequence).

Specifically, the present application provides a method for replicating a target DNA sequence, which method comprises:
i) providing the vector according to the first aspect of the present application;
   providing the vector backbone described in the first aspect of the present application, inserting the target DNA sequence, or a promoter and the target DNA sequence at the cloning site of a), inserting the target DNA sequence at the cloning site of b), inserting the target DNA sequence at the cloning site of the DNA expression cassette of c), or inserting the target DNA sequence at the cloning site of the DNA expression cassette of d) to obtain a new vector;
   providing the vector according to the second aspect of the present application; or
   providing the vector backbone according to the second aspect of the present application, inserting the target DNA sequence, or a promoter and the target DNA sequence at the cloning site of the DNA sequence set of a), or inserting the target DNA sequence at the cloning site of the DNA expression cassette of b) to obtain a new vector;
ii) transforming/transfecting the vector of step i) into a host cell; and
iii) culturing the host cell under conditions conducive to vector replication.

The present application further provides a method for expressing a target DNA sequence, which method comprises:
i) providing the vector comprising a) the target DNA expression cassette according to the first aspect of the present application; or
   providing the vector backbone according to the first aspect of the present application, inserting a promoter and the target DNA sequence at the cloning site of a), inserting the target DNA sequence at the cloning site of b), inserting the target DNA sequence at the cloning site of the DNA expression cassette of c), or inserting the target DNA sequence at the cloning site of the DNA expression cassette of d) to obtain a new vector; and
ii) transforming/transfecting the vector of step i) into a host cell, and culturing the host cell under conditions conducive to expression of the target DNA sequence; or linearizing the vector of step i) and performing *in-vitro* transcription; or

i) providing the vector comprising a) the target DNA expression cassette according to the second aspect of the present application;
   or providing the vector backbone according to the second aspect of the present application, inserting a promoter and the target DNA sequence at the cloning site of the DNA sequence set of a), or inserting the target DNA sequence at the cloning site of the DNA expression cassette of b) to obtain a new vector; and
ii) transforming/transfecting the vector of step i) into a host cell, and culturing the host cell under conditions conducive to expression of the target DNA sequence;
   linearizing the vector of step i) and performing *in-vitro* transcription; or
   co-transforming/co-transfecting the vector of step i) with a packaging plasmid and a helper plasmid into a host cell, culturing the host cell under conditions conducive to vector packaging, recovering the packaged vector, transforming/transfecting the packaged vector into a host cell, and culturing the host cell under conditions conducive to expression of the target DNA sequence.

The method may further comprise, between step i) and step ii), transforming/transfecting the vector of step i) into a host cell, and culturing the host cell under conditions conducive to vector replication.

In a sixth aspect, the present application provides the use of the vector backbone or vector of the present application, including those prepared by the method of the present application and those modified by the method of the present application, in the preparation of a recombinant adeno-associated virus (rAAV) vector.

Specifically, the present application provides a method for preparing a recombinant adeno-associated virus vector, which method comprises:
i) providing the vector according to the second aspect of the present application; or
   providing the vector backbone according to the second aspect of the present application, inserting a target DNA sequence, or a promoter and a target DNA sequence at the cloning site of the DNA expression cassette of a), or inserting a target DNA sequence at the cloning site of the DNA expression cassette of b) to obtain a new vector;
ii) optionally, transforming/transfecting the vector of step i) into a host cell, and culturing the host cell under conditions conducive to vector replication; and
iii) harvesting the vector of step i) or ii), co-transforming/co-transfecting the harvested vector with a packaging plasmid and a helper plasmid into a host cell, and culturing the host cell under conditions conducive to vector packaging.

Other features and advantages of the present disclosure will become more apparent based on the following detailed description and examples, which should not be construed as limiting. The content of all of the documents, Genbank records, patents, and published patent applications cited in the present application is expressly incorporated herein by reference.

### BRIEF DESCRIPTION OF THE DRAWINGS

The following description is provided by way of example only, which is not intended to limit the present disclosure to the stated specific embodiments and can be better understood with reference to the accompanying drawings.
FIG. 1 shows a schematic diagram of a pUC57-kana test plasmid.
FIG. 2 shows sequencing schematic diagrams of a failed clone with deletion of the poly(A) tail-coding sequence (A), a failed clone with an impure poly(A) tail-coding sequence and a sequencing background peak higher than 20% of the main peak (B), and a positive clone with a relatively pure poly(A) tail-coding sequence and a sequencing background peak lower than 20% of the main peak (C).
FIG. 3 shows the monoclonal success rate of pUC57-kana plasmids with spacer sequences of different lengths between Ori and the poly(A) tail-coding sequence.
FIG. 4 shows a schematic diagram of a pUC57-kana plasmid comprising a terminator in a 247 bp spacer sequence between Ori and the poly(A) tail-coding sequence.
FIG. 5 shows sequencing schematic diagrams of a pUC57-kana plasmid without a terminator (A) or with a terminator (B) in a 247 bp spacer sequence, and the monoclonal success rate of the above two plasmids (C).
FIG. 6 shows a schematic diagram of a pUC57-kana plasmid with the lacP functional region disrupted by a 465 bp spacer sequence.
FIG. 7 shows the impact of the lacP functional region on the construction success rate of a monoclonal plasmid comprising the poly(A) tail-coding sequence.
FIG. 8 shows the map of a GS-MK plasmid.
FIG. 9 shows the map of a GS-PV plasmid.
FIG. 10 shows sequencing chromatograms of the GS-MK vector inserted with different poly(A) tail-coding sequences.
FIG. 11 shows sequencing chromatograms of the GS-PV vector inserted with different poly(A) tail-coding sequences.
FIG. 12 shows sequencing chromatograms of the GS-CMV vector inserted with different poly(A) tail-coding sequences.
FIG. 13 shows the monoclonal success rates of pUC57-kana, GS-MK, PVAX1, GS-PV and GS-CMV comprising the poly(A) tail-coding sequence.
FIG. 14 shows the passage stability of pUC57-kana, GS-MK, PVAX1, GS-PV and GS-CMV comprising the poly(A) tail-coding sequence.
FIG. 15 shows the yields of pUC57-kana, GS-MK, PVAX1, GS-PV and GS-CMV comprising the poly(A) tail-coding sequence.
FIG. 16 shows the *in-vitro* transcription product purity of pUC57-kana, GS-MK, PVAX1, GS-PV and GS-CMV comprising the poly(A) tail-coding sequence.
FIG. 17 shows the protein expression levels of mRNA transcribed from pUC57-kana, GS-MK, PVAX1, GS-PV and GS-CMV comprising the poly(A) tail-coding sequence.
FIG. 18 shows the maps of four plasmids comprising lacP.
FIG. 19 shows a schematic diagram of the pUC57-kana vector with ITR sequences added (A), and a schematic diagram of the vector with ITR sequences added, the Ori position adjusted, the lacP functional region disrupted, and terminator sequences inserted downstream of the 5' ITR and upstream of the 3' ITR (B).
FIG. 20 shows a schematic diagram of the pAAV-tTA plasmid (A), and a schematic diagram of the vector with the promoter replaced and terminator sequences added downstream of the 5' ITR and upstream of the 3' ITR (B).
FIG. 21 shows the restriction enzyme digestion electrophoresis diagram of pUC57 kana, GenS-pUC57-AAV-NoTer, and GenS-pUC57-AAV-Ter after gene cloning, in which lanes 1-3: pUC57 kana vector, SmaI digestion product, and standard DNA size marker; lanes 4-6: GenS-pUC57-AAV-NoTer vector, SmaI digestion product, and standard DNA size marker; lanes 7-9: GenS-pUC57-AAV-Ter vector, SmaI digestion product, and standard DNA size marker.

### DETAILED DESCRIPTION

Unless otherwise specified, the terms used herein have the ordinary meanings as defined in dictionaries, textbooks, and technical handbooks, or the meanings commonly understood by those skilled in the art. The following descriptions of some terms are provided only for the purpose of facilitating the understanding of the present application and are not intended to specifically limit such terms, unless otherwise specified.

As used herein and in the appended claims, the singular forms "a", "an", and "the" include the plural referents, unless otherwise clearly indicated in the context. For example, "a vector" may include a plurality of such vectors.

The term "cloning vector" refers to a type of DNA molecule that has self-replication capability, comprises genetic elements such as an origin of replication, a selectable marker, and a cloning site, and can carry DNA fragments into host cells for large-scale amplification of the DNA fragments. A cloning vector may include a nucleic acid sequence that enables the vector to replicate autonomously in one or more host cells, for example, an origin of replication.

The term "expression vector" refers to a vector obtained by incorporating elements related to expression regulation, such as a promoter, a ribosome-binding site, and a terminator, into a cloning vector.

"Vector backbone" refers to a vector that comprises basic replication or expression elements, and other required elements. A DNA sequence of interest can be inserted into the vector backbone via a cloning site or replaced into the vector backbone through homologous recombination, thereby enabling the replication, expression, etc., of the DNA sequence of interest.

An "origin of replication" or "Ori" refers to the site where DNA replication initiates, which controls the replication of the plasmid and determines the host specificity and copy number of the plasmid. An Ori is usually rich in adenine (A) and thymine (T), which facilitates unwinding and provides binding space for replication regulatory elements such as Rep proteins. Common origins of replication include pUC (with a nucleotide sequence as set forth in SEQ ID NO: 23), ColE1, pMB1, pSC101, and R6K. Vectors can be classified into relaxed-type and stringent-type based on the differences in the control mechanism of the origin of replication. In stringent-type plasmid vectors, the replication of the plasmid is controlled by the same factors as the replication of the host cell chromosome. The plasmid replicates once for each replication of the host cell, with only 1-2 plasmids present per host cell. In contrast, relaxed-type plasmids can continue to replicate even after the replication of the host cell chromosome stops, indicating that they are controlled by different factors from those controlling chromosomal replication, with each host cell typically containing dozens to hundreds of plasmids. In addition, based on the design of the regulatory elements associated with the origin of replication, vectors can be classified into narrow-host-range type and broad-host-range type. In narrow-host-range vectors, the Ori relies on the replication elements of the host cell or Rep proteins provided by the host cell. However, in broad-host-range vectors, the vector autonomously comprises Rep protein expression elements. The "Rep protein" can bind to the origin of replication and move in a specific direction to unwind the double-stranded DNA. The vector comprises one or more origins of replication for autonomous replication in one or more host cells.

"Selectable marker" refers to a gene in the vector that functions as a specific marker, which can be used to verify whether the vector has been successfully transformed/transfected into the host cell. The selectable marker may be inherent to the vector or recombinantly introduced. The selectable marker may be a resistance gene, preferably a resistance gene that the host cell does not naturally possess. Thus, when the vector is successfully introduced into the host cell, the selectable marker can confer the corresponding resistance to the host cell, thereby confirming that the vector has entered the host cell. Common selectable markers include ampicillin (Ampr) resistance gene, chloramphenicol (Camr) resistance gene, kanamycin (Kanr) resistance gene, tetracycline (Tetr) resistance gene, puromycin (Puro) resistance gene, G418 resistance gene, hygromycin β (Hygr) resistance gene, bleomycin resistance gene, blasticidin (which specifically inhibits protein synthesis in prokaryotes and eukaryotes by interfering with peptide bond formation in ribosomes) resistance gene, etc.

"Cloning site" refers to a DNA fragment comprised in the vector that includes one or more restriction endonuclease recognition sites. The cloning site, after being treated with a corresponding restriction endonuclease, allows for the insertion of foreign DNA that has been treated with the same restriction endonuclease. Based on the number of restriction endonuclease recognition sites in the sequence, cloning sites can be classified into single cloning sites and multiple cloning sites (MCS). The cloning site may comprise recognition sites for NheI, BmtI, AflII, HindIII, Acc65I, KpnI, BamHI, ApoI, RcoRI, PstI, EcoRV, BstXI, and/or NotI. A person skilled in the art can routinely determine the sequence of the cloning site. The cloning site may comprise recognition sites for type IIS restriction endonucleases. Type IIS restriction endonucleases are a special group of endonucleases that cleave DNA at a specific position downstream of the recognition site thereof, with no requirement for the sequence at the cleavage site. In some embodiments, the ends of a target DNA sequence to be inserted into or inserted into a vector backbone, such as an mRNA-coding sequence and a poly(A) tail-coding sequence, comprise IIS enzyme recognition sites, while the vector backbone does not comprise the corresponding IIS enzyme recognition sites. For example, IIS sites such as BsaI, BspQI, BspMI, BpiI, and BsmbI can be removed by synonymous mutation. Thus, after the vector comprising the target DNA sequence is linearized, the type IIS restriction endonuclease only recognises outside, for example, the poly(A) tail-coding sequence and cleaves within the poly(A)-coding sequence. First, the resulting restriction enzyme-digested fragments will not be disorganized due to the presence of multiple IIS enzyme restriction sites. Second, the polyT sequence in the digested fragments will not incorporate non-T sequences, and the corresponding poly(A) tail obtained will thus be highly pure. A pure poly(A) tail can maintain a high level of protein expression from the mRNA (Holtkamp S et al., (2006) Modification of antigen-coding RNA increases stability, translational efficacy, and T-cell stimulatory capacity of dendritic cells. Blood. 108(13): 4009-4017). This design also facilitates Golden Gate cloning of the vector.

"Promoter" refers to a DNA sequence that RNA polymerase recognises, binds to, and initiates transcription from, comprising conserved sequences required for specific binding and transcription initiation by RNA polymerase. Most promoters are located upstream of the transcription start site of a gene and are not transcribed themselves. Promoters can be classified into constitutive promoters, tissue/cell-specific promoters, and inducible promoters. Constitutive promoters, also known as non-specific expression promoters, show no significant differences in gene expression across different cells, tissues, organs or developmental stages under regulation thereof. Common examples of such promoters include Ubc, CMV, SV40, EF1a, U6, and CAG (composed of the CMV enhancer and the chicken β-actin promoter). Under the regulation of tissue/cell-specific promoters, the foreign gene is specifically expressed only in specific target cells. An inducible promoter refers to a promoter that significantly increases the transcriptional level of a gene upon stimulation by specific physical or chemical signals. A commonly used example is the tetracycline-inducible promoter.

An "enhancer" refers to a region on DNA that can bind to transcription factors. Upon binding to transcription factors, the transcription of the target gene is enhanced. The enhancer may be located upstream or downstream of a gene and does not necessarily need to be close to the gene the enhancer acts on. Common examples of enhancers include the CMV enhancer, etc.

"Terminator" refers to a DNA sequence capable of terminating the transcription by RNA polymerase and initiating the dissociation of the newly synthesized RNA from the transcription machinery. Terminators are typically located after the 3' regulatory sequence of a gene. Prokaryotic terminators can be classified into two categories: One category requires a protein cofactor to achieve termination, and this protein cofactor is usually called the p factor (Rho factor); The other category can achieve termination without relying on a protein cofactor. Such terminators share some common sequence characteristics. That is, the terminators comprise a GC-rich inverted repeat sequence. The transcribed RNA tends to form a hairpin structure, which disrupts the normal structure at the 5' end of the RNA-DNA hybrid strand, causing RNA polymerase to pause transcription. This GC-rich inverted repeat sequence is immediately followed by a sequence composed of 4-8 A bases. The transcription product of this sequence is oligo(U), which results in an unstable U·A region at the 3' end of the RNA-DNA hybrid strand. Upon release, a weakly bonded polyuridine loop is formed. Almost all commonly used bacterial expression vectors use ρ-independent terminators, including some naturally occurring terminators such as T7 and rrnB, and some engineered high-efficiency terminators such as T0. No terminator can initiate the termination step with 100% efficiency, but the termination efficiency of some engineered terminators can approach 95%.

In the present application, the terminator suitable for DNA sequence expression in a eukaryotic cell may be referred to as a "transcription terminator" under certain circumstances. Eukaryotic terminators refer to DNA fragments located at the end of a gene or operon, which can interrupt transcription in eukaryotic systems and bind polyadenylation enzymes to promote polyadenylation. Commonly used eukaryotic terminators include SV40 poly(A), hGH poly(A), bGH poly(A), and rbGlob poly(A) signals. The "bGH poly(A) signal" refers to the bovine growth hormone (BGH) polyadenylation signal sequence, the "hGH poly(A) signal" refers to the human growth hormone (hGH) polyadenylation signal sequence, the "SV40 poly(A) signal" refers to the simian vacuolating virus polyadenylation signal sequence, and the "rbGlob poly(A) signal" refers to the rabbit β-globin polyadenylation signal sequence, all of which can effectively terminate transcription and promote mRNA polyadenylation.

In the present application, the "DNA expression cassette" or "target DNA expression cassette" refers to an expression component comprising a promoter, a target DNA sequence (or a cloning site for the insertion of the target DNA sequence), and optionally the bGH poly(A) signal, etc. When the DNA expression cassette is inserted into a vector and comprises the target DNA sequence, transcription of the target DNA sequence, and potentially subsequent translation, can be performed under specific conditions. In the present application, the "DNA expression cassette" or "target DNA expression cassette" refers, in some embodiments, to an expression cassette suitable for DNA sequence expression in a eukaryotic cell.

In the present application, the "target DNA sequence" may refer to any DNA sequence fragment, particularly a DNA sequence fragment of interest. When the "target DNA sequence" is an mRNA-coding sequence, it may include a 5' UTR, an open reading frame (ORF), and a 3' UTR. The "open reading frame" or "ORF" refers to a portion of a gene sequence that comprises a nucleotide sequence capable of encoding a protein, and is a theoretical amino acid-coding region. The "target DNA sequence" may also be a coding sequence for a target protein, which can be any protein of interest, such as an antibody or an enzyme. The "target DNA sequence" of the present application may include a DNA sequence capable of forming a non-B DNA conformation.

*"In-vitro* transcription" or "IVT" refers to the process of transcribing RNA using linear DNA as a template in a cell-free environment. An *in-vitro* transcription reaction generally requires a purified linear DNA template comprising a promoter, ribonucleoside triphosphates, a buffer system comprising DTT and magnesium ions, and an appropriate amount of RNA polymerase.

"Host cell" encompasses cells that contain a vector and preferably support the replication, or replication and expression, of the vector. The host cell may be a prokaryotic cell (e.g., *Escherichia coli*) or a eukaryotic cell (e.g., yeast, insect, amphibian, or mammalian cells). In some embodiments, the host cell is an *Escherichia coli* cell, such as *Escherichia coli* TOP10 or the recombinant strain GenITRv1. In some embodiments, the host cell is a eukaryotic cell, such as a HEK293 cell.

The term "transformation" refers to a technique in which a vector comprising an exogenous gene (such as a recombinant plasmid) introduces the exogenous gene into a prokaryotic host cell, while "transfection" refers to a technique for introducing an exogenous gene or vector (such as a recombinant plasmid) into a eukaryotic cell (generally a mammalian cell). Transformation and transfection are generally performed using competent cells, and may also be achieved by methods such as lipofection, particle bombardment, microinjection, and electroporation. "Competent cell" refers to a cell that, after induction by physical or chemical methods, is in a state suitable for uptake and accommodation of exogenous DNA. For example, treating *Escherichia coli* with divalent cations (e.g., CaCl₂) under cold conditions can render the cell wall permeable to plasmid DNA.

"Adeno-associated virus" or "AAV" is a small, non-pathogenic virus belonging to the *Parvoviridae* family that relies on a helper virus for replication. The genome of AAV is approximately 5 kb in size and is composed of a single-stranded DNA of positive or negative polarity. The ends of the genome are short palindromic/inverted terminal repeats (ITRs), which are the only cis-acting elements in the genome. ITRs can fold into hairpin structures, serve as origins of viral DNA replication, and participate in processes such as genomic integration, rescue, replication, and packaging. Recombinant adeno-associated virus (rAAV), as a DNA delivery vector, has become the most important vector in genetic research and gene therapy due to its advantages such as high safety, low immunogenicity, broad applicability, and long-acting effects. To date, numerous AAV serotypes have been isolated from humans or primates. For example, the genome of AAV2 is 4680 nucleotides in length and comprises two open reading frames.

"Inverted terminal repeat" or "ITR" refers to the inverted repeat sequences at the ends of the AAV genome, which are capable of forming palindromic structures. ITRs are the only genetic component shared by recombinant AAV and wild-type AAV, and play an important role in processes such as recombinant AAV replication, packaging, and sustained exogenous gene expression. ITRs can be sequentially divided into 7 segments: A, C, C', B, B', A', and D. Among them, A and A' are reverse complementary to form a large palindromic stem, while B and B', as well as C and C', are each reverse complementary to form small palindromic arms. One Rep protein-binding site is present in the A-A' region, another Rep protein-binding site is present at the top of the B-B' or C-C' region, and a TRS sequence is present at the junction between A-A' and D. By truncating the B and B' palindromic arms and/or the C and C' palindromic arms while retaining one or both key Rep protein-binding sites and the TRS sequence, an ITR with a simplified structure that maintains the above functions of rAAV replication, packaging, and exogenous gene expression functions can be obtained. In the present application, "ITR-derived sequence" refers to an inverted palindromic sequence that can form a complete wild-type ITR structure or a partial wild-type ITR structure, while retaining the functions of rAAV replication, packaging, and exogenous gene expression.

The lac operon is a group of genes involved in lactose catabolism, composed of a regulatory gene (lacI), a promoter (lacP), an operator (lacO), and structural genes (lacZ, lacY, and lacA). In the present application, "lac promoter" or "lacP" refers to a promoter within the lac operon, which is the binding site for RNA polymerase during transcription initiation. lacP exhibits "leakiness", that is, transcription from the promoter is not completely "shut down", and there is always some basal transcription (F. Jacob, J. Monod (1961) Genetic regulatory mechanisms in the synthesis of proteins, J. Mol. Biol. 3: 318-356). lacP is often constructed in plasmid cloning vectors for expressing recombinant proteins in bacteria, and provides the ability for high-level expression of the introduced gene. For expression cloning, structures are generally constructed in medium-to-high copy number plasmids (e.g., pUC, pBluescript, and pET vectors and derivatives thereof) to increase the copy number of the recombinant gene, which enhances the "leaky" expression of lacP to a certain extent (Nielsen, Brent L., Van C. Willis, and Chin-Yo Lin. (2007) Western blot analysis to illustrate relative control levels of the lac and ara promoters in Escherichia coli, Biochemistry and Molecular Biology Education 35(2): 133-137).

"Non-B DNA conformations" refer to DNA conformations that differ from the typical right-handed double helix (B-form), including A-form DNA, Z-form DNA, H-form DNA, G-quadruplexes, triplexes, hairpins or cruciform structures, and slipped-strand DNA. Studies have shown that these DNA conformations are present at least transiently in DNA replication, transcription, repair, or recombination processes, where they can impede the progression of these processes and increase the DNA mutation rate. The poly(A) tail-coding sequence is composed of a large number of adenines (A) or thymines (T), and is prone to forming H-DNA or triplex structures. Inverted repeat sequences, such as coding sequences for ITRs or shRNA, are prone to forming hairpin or cruciform structures. Direct repeat sequences are likely to form slipped-strand structures (Guiblet WM et al., (2021) Non-B DNA: a major contributor to small- and large-scale variation in nucleotide substitution frequencies across the genome. Nucleic Acids Res. 49(3): 1497-1516).

"Direct repeat sequences" refer to DNA sequences that are repeated in the same orientation, and a spacer sequence may be included between the two repeats. Direct repeat sequences, particularly when rich in guanine (G), are prone to forming non-B DNA conformations such as G-quadruplexes during replication and transcription.

"Inverted repeat sequences" refer to sequences that are complementary in the reverse orientation, with or without a spacer sequence in between. An example of inverted repeat sequences is the coding sequences for ITRs or shRNA, which are prone to forming hairpin or cruciform structures in processes such as replication and transcription.

"Homopurine-homopyrimidine sequence" refers to a DNA duplex in which one strand is essentially or completely composed of purines, while the complementary strand is essentially or completely composed of pyrimidines. Special examples include polyA sequences, polyT sequences, polyG sequences, and polyC sequences. Such sequences can form H-DNA conformations or triplex structures. The poly(A) tail-coding sequence can also be classified into this category of sequences, including but not limited to polyT:polyA.

"Poly(A) tail" or "polyA tail" sequence may be composed of consecutive adenines, such as a polynucleotide sequence composed of 120 consecutive adenines. The sequence may also be a segmented polynucleotide sequence composed of one or more non-adenine spacer sequences interspersed among consecutive adenines, for example, a polynucleotide sequence composed of 30 consecutive adenines, 3 to 20 non-adenines, 30 consecutive adenines, 3 to 20 non-adenines, and 30 consecutive adenines.

In the present application, "upstream" and "downstream" refer to positions in a vector that are respectively located before and after (particularly immediately adjacent to) a target nucleic acid sequence along a specific transcriptional orientation. When multiple promoters are present in the vector, which results in multiple transcriptional orientations, the target nucleic acid sequence may have multiple paired "upstream" and "downstream" positions. In particular, the arrangement of the first terminator and the second terminator in the present application ensures that the target nucleic acid sequence located between the first terminator and the second terminator is not transcribed along with the transcriptional activities occurring outside the target nucleic acid sequence in the vector.

Mature mRNA has a long poly(A) tail structure, which plays an important role in maintaining mRNA stability, regulating mRNA translation efficiency, and maintaining mRNA transport. For the large-scale synthesis of mRNA, the currently efficient method is *in-vitro* transcription. There are mainly two approaches for poly(A) tailing in IVT. The first is enzymatic synthesis. Specifically, after transcribing mRNA from a linear DNA template, poly(A) polymerase is added to catalyze the addition of AMP converted from ATP to the 3' end of the single-stranded RNA, thereby forming a Poly(A) tail. The advantages of this approach are that the transcription template does not need to comprise a Poly(A)-coding sequence and the operation is simple. However, this approach has the limitation of unstable tail length. The second is co-transcription, which involves the direct transcription of the poly(A) tail-coding sequence already present in the template plasmid DNA or PCR product. The advantages of this approach include eliminating the need for Poly(A) polymerase, reducing process steps, saving costs, and achieving relatively stable transcribed tail lengths. However, during the preparation of upstream plasmids, it is necessary to reduce the deletion of the poly(A) tail-coding sequence during cultivation, which is crucial for providing a high-yield, high-quality, and homogeneous plasmid template.

The inventors of the present application have found through experiments that the amplification and/or expression (transcription) stability of the poly(A) tail-coding sequence can be significantly enhanced by: i) increasing a spacing distance between the origin of replication and the poly(A) tail-coding sequence in the vector, ii) adding terminators upstream and/or downstream of the poly(A) tail-coding sequence or an mRNA expression cassette comprising the poly(A) tail-coding sequence, and/or iii) disrupting a functional region of a native lac promoter in the vector, thereby improving the purity of the plasmid for *in-vitro* transcription and/or the protein expression level of the produced mRNA. In addition, the yield of the vector can be further improved by modifying the origin of replication sequence of certain low-to-medium copy vectors.

Therefore, the present application provides a vector or vector backbone, which may comprise:
i) an origin of replication; and
ii) a) a target DNA expression cassette, comprising a promoter and a target DNA sequence, b) a target DNA sequence, c) a cloning site, d) a cloning site and a DNA sequence capable of forming a non-B DNA conformation, e) a DNA expression cassette, comprising a promoter and a cloning site, or f) a DNA expression cassette, comprising a promoter, a cloning site, and a DNA sequence capable of forming a non-B DNA conformation.

The vector or vector backbone comprising a), e), or f) in ii) may be used as a cloning vector or an expression vector. The target DNA sequence in a) may be any sequence, and may comprise a sequence forming a B DNA conformation and/or a sequence forming a non-B DNA conformation. A target DNA sequence may be inserted at the cloning site in e), wherein the target DNA sequence may comprise a DNA sequence capable of forming a non-B DNA conformation. Any sequence may be inserted at the cloning site in f), including a sequence forming a B DNA conformation and/or a sequence forming a non-B DNA conformation.

The vector or vector backbone comprising b) or d) in ii) may be used only as a cloning vector. The target DNA sequence in b) may be any sequence, and may comprise a sequence forming a B DNA conformation and/or a sequence forming a non-B DNA conformation. Any sequence may be inserted at the cloning site in d), including a sequence forming a B DNA conformation and/or a sequence forming a non-B DNA conformation.

The vector backbone comprising c) in ii) may be used as a cloning vector or a cloning/expression vector, depending on the sequence to be inserted into the cloning site. In the case that a promoter and a target DNA sequence are inserted into the cloning site, the vector may be used as a cloning/expression vector.

The origin of replication may be spaced at least 150 bp, 200 bp, 300 bp, 500 bp, or 700 bp in a replication direction from a) a target DNA sequence in a target DNA expression cassette, b) a target DNA sequence, c) a cloning site, d) a DNA sequence capable of forming a non-B DNA conformation, e) a cloning site in a DNA expression cassette, or f) a DNA sequence capable of forming a non-B DNA conformation in a DNA expression cassette. The purpose of spacing at least 150 bp, 200 bp, 300 bp, 500 bp, or 700 bp is to minimize the impact of the origin of replication, when unwound during replication or transcription, on the DNA sequence capable of forming a non-B DNA conformation in the vector, such as a poly(A) tail-coding sequence. The DNA sequences capable of forming a non-B DNA conformation may adopt such a non-B DNA conformation during DNA unwinding, thereby impeding the progression of replication or transcription, and increasing the risk of mutations therein, such as deletions. As the unwinding time increases, the probability of mutations in non-B DNA also increases. Notably, the unwinding time at the origin of replication may be longer than that at other locations due to factors such as protein binding. Therefore, increasing the spacing distance between the origin of replication and the DNA sequences capable of forming a non-B DNA conformation can reduce mutations in the DNA sequences of a non-B DNA conformation. The inventors of the present application have found through experiments that when the spacer sequence between the origin of replication and the DNA sequence capable of forming a non-B DNA conformation is increased from 100 bp to 247 bp, 500 bp, or even 700 bp, the proportion of vectors that retain an intact poly(A) tail-coding sequence during replication can be increased from 0.00% to 3.13%, 46.67%, or even 83.3%. The origin of replication enables the vector to replicate in a suitable host cell. The suitable host cell may be a prokaryotic cell such as *Escherichia coli,* or a eukaryotic cell. In some embodiments, the origin of replication may be a pUC origin of replication, which may comprise a sequence such as SEQ ID NO: 23.

In the case of, for example, a shuttle plasmid vector, the vector or vector backbone may further comprise a second origin of replication. By the same reason, the second origin of replication may be spaced at least 150 bp, 200 bp, 300 bp, 500 bp, or 700 bp in a replication direction from a) a target DNA sequence in a target DNA expression cassette, b) a target DNA sequence, c) a cloning site, d) a DNA sequence capable of forming a non-B DNA conformation, e) a cloning site in a DNA expression cassette, or f) a DNA sequence capable of forming a non-B DNA conformation in a DNA expression cassette. The second origin of replication enables the vector to replicate in a suitable host cell. The suitable host cell may be a prokaryotic cell such as *Escherichia coli,* or a eukaryotic cell.

Depending on the type of origin of replication in the vector or vector backbone, the vector or vector backbone may further comprise a Rep protein expression element correspondingly, including a Rep protein-coding sequence and a corresponding promoter, thereby enabling the vector or vector backbone to be a broad-host-range type.

The vector or vector backbone may further comprise a first terminator and a second terminator. The first terminator and the second terminator are respectively located upstream and downstream of the sequence in ii) (i.e., a) a target DNA expression cassette, b) a target DNA sequence, c) a cloning site, d) a cloning site and a DNA sequence capable of forming a non-B DNA conformation, or e) a DNA expression cassette), and configured to terminate transcription outside the above sequence in the vector or vector backbone upstream and downstream of the sequence. Such a design can significantly reduce unwinding within the regions of ii), thereby maintaining the stability of the sequences in these regions, particularly the stability of the DNA sequence capable of forming a non-B DNA conformation, such as the poly(A) tail-coding sequence. The inventors of the present application have found through experiments that when the spacing distance between the origin of replication and the specific sequence in ii) is 247 bp, and terminators are added to both ends of the sequence in ii), the proportion of vectors that retain an intact poly(A) tail-coding sequence during replication can be increased from 3.13% to 37.14%. The first terminator may be a forward terminator, a bidirectional terminator, or a reverse terminator. The second terminator may be a forward terminator, a bidirectional terminator, or a reverse terminator. In some embodiments, both the first terminator and the second terminator may be bidirectional terminators. "Forward terminator" refers to a terminator capable of terminating transcription on the strand where the terminator is located. "Reverse terminator" refers to a terminator capable of terminating transcription on the complementary strand of the strand where the terminator is located. "Bidirectional terminator" refers to a terminator capable of terminating transcription on both the strand where the terminator is located and the complementary strand thereof. The terminators may be configured according to the specific structure of the vector or vector backbone, such as the transcription direction of the selectable marker. Any terminator may be used, as long as the terminator ensures that transcription initiated by promoters outside the sequence region of ii) in the vector or vector backbone is terminated upstream and downstream of the sequence of ii), thereby ensuring that transcription occurring outside the sequence region of ii) does not induce unwinding within these regions. A bidirectional terminator may be configured in the universal vector backbone. In some embodiments, the first terminator may be an rrnB T1 terminator and an rrnB T2 terminator. In some embodiments, the second terminator may be a lambda t0 terminator. The rrnB T1 terminator, rrnB T2 terminator, and lambda t0 terminator are known in the art, and may comprise the nucleotide sequences set forth in SEQ ID NO: 24, 25, or 26, respectively.

The cloning site in the vector or vector backbone may be a single cloning site or a multiple cloning site. The cloning site may be configured according to actual needs.

The vector or vector backbone may further comprise a selectable marker. The selectable marker may be an antibiotic resistance gene, such as an ampicillin resistance gene, a tetracycline resistance gene, a chloramphenicol resistance gene, or a kanamycin resistance gene. In particular, the selectable marker is not present in the genome of the host cell into which the vector or vector backbone is to be transformed/transfected. In some embodiments, the selectable marker is a kanamycin resistance gene. The vector or vector backbone may further comprise a promoter for the expression of the selectable marker upstream of the selectable marker. In addition to confirming whether the vector has been introduced into the host cell, the selectable marker may also promote the replication and/or expression of the vector under certain circumstances. For example, when the vector or vector backbone comprises a kanamycin resistance gene and the culture medium of the host cell contains kanamycin, the replication of the vector may be accelerated.

The vector or vector backbone may be circular. The vector or vector backbone may be a non-viral vector or a non-viral vector backbone. The vector or vector backbone may be a plasmid vector or a plasmid vector backbone. In some embodiments, the vector or vector backbone may be a circular plasmid vector or a circular vector backbone.

In view of the "leakiness" of the lac promoter, in particular, the vector or vector backbone does not comprise a fully functional lac promoter. The inventors have demonstrated through experiments that after eliminating the activity of the lacP functional region, the proportion of vectors that retain an intact poly(A) tail-coding sequence during vector replication or passage can be increased from 31.25% to 50%. Even if the vector or vector backbone already comprises two terminators, inactivating the lac promoter region is still advantageous because terminators cannot guarantee 100% termination of transcription.

In some embodiments, the above vector or vector backbone of the present application may be configured to replicate and/or express mRNA and a poly(A) tail thereof.

In addition to inserting the desired sequence at the cloning site, a DNA sequence of interest (such as a DNA sequence capable of forming a non-B DNA conformation, a specific mRNA-coding sequence, or a promoter) may be introduced into the vector or vector backbone of the present application by methods such as homologous recombination. Vectors or vector backbones obtained by methods such as homologous recombination are also within the scope of protection of the present application.

Recombinant adeno-associated virus (rAAV), as a DNA delivery vector, has become the most important vector in genetic research and gene therapy due to its advantages such as high safety, low immunogenicity, broad applicability, and long-acting effects. Both ends of rAAV comprise ITRs of wild-type AAV or their derivative sequences thereof, which are the only cis-acting elements in rAAV, and are involved in the replication, packaging, and exogenous gene expression of rAAV.

However, the palindromic sequence of ITRs may form a highly stable T-shaped hairpin structure. Additionally, ITRs, due to their high GC content, are prone to mutations or recombination-mediated deletion during bacterial propagation. The integrity of ITRs is critical for the efficient replication and packaging of rAAV. Single-molecule real-time sequencing, which comprehensively analyses the rAAV genome as a single intact molecule, has revealed a correlation between deleted ITRs and the packaging of non-vector sequences in a HEK293 triple-plasmid packaging system. The impact of ITR deletion on rAAV viral packaging and the application of rAAV in gene therapy is complex, and unexpected biological consequences may arise through vector-host interactions. If incomplete ITR sequences are used during the preclinical stage, the reproducibility of experiments both within and across laboratories may be reduced, and the reproducibility of clinical trials using intact ITR sequences may also be compromised, thereby delaying translation into clinical applications. The issue is further exacerbated by the fact that partial loss of ITR may go unnoticed due to its self-repairing capability during vector packaging under certain circumstances (Wilmott, P., Lisowski, L., Alexander, I. E., & Logan, G. J. (2019). A user's guide to the inverted terminal repeats of adeno-associated virus. Human Gene Therapy Methods, 30(6): 206-213).

Currently, there is no effective method to avoid ITR deletion during the construction of rAAV-ITR plasmids and the amplification thereof in *Escherichia coli.* Some commercial recombination-deficient strains, such as JC8111, SURE2, Stabl3, and XL10-Gold, are often used for the construction and amplification of plasmids comprising ITR structures, but they are not always effective.

The inventors of the present application have unexpectedly found that the amplification and/or expression (transcription) stability of ITR sequences can be significantly enhanced by: i) increasing a spacing distance between the origin of replication and the ITR sequences in the vector; ii) adding terminators upstream and downstream of the ITRs; and/or iii) disrupting a functional region of a native lac promoter in the vector. In addition, the above modifications to the vector can further increase the yield of the vector and/or the yield of recombinant adeno-associated virus (rAAV), such as the packaging titer.

Therefore, the present application provides a vector or vector backbone, which may comprise:
i) an origin of replication; and
ii) a) a target DNA expression cassette, sequentially comprising a first ITR or a derivative sequence thereof, a promoter, a target DNA sequence, and a second ITR or a derivative sequence thereof; b) a target DNA sequence set, sequentially comprising a first ITR or a derivative sequence thereof, a target DNA sequence, and a second ITR or a derivative sequence thereof; c) a DNA sequence set, sequentially comprising a first ITR or a derivative sequence thereof, a cloning site, and a second ITR or a derivative sequence thereof; or d) a DNA expression cassette, sequentially comprising a first ITR or a derivative sequence thereof, a promoter, a cloning site, and a second ITR or a derivative sequence thereof.

The vector or vector backbone comprising a) and d) in ii) may be used as a replication vector, or may be used for expressing a target DNA sequence after AAV packaging. A target DNA sequence, such as an mRNA-coding sequence or an mRNA-coding sequence plus a poly(A) tail-coding sequence, may be inserted at the cloning site in d). The target DNA sequence may be any sequence, including a DNA sequence capable of forming a B DNA conformation and/or a DNA sequence capable of forming a non-B DNA conformation.

The vector comprising b) in ii) may be used as a replication vector. The target DNA sequence may be any sequence, including a DNA sequence capable of forming a B DNA conformation and/or a DNA sequence capable of forming a non-B DNA conformation.

The vector backbone comprising c) in ii) may have a target DNA sequence inserted at the cloning site to become a cloning vector. The target DNA sequence may be any sequence, such as a DNA sequence capable of forming a B DNA conformation and/or a DNA sequence capable of forming a non-B DNA conformation. The vector backbone may also have a promoter and a target DNA sequence inserted at the cloning site to become a cloning/expression vector. The target DNA sequence may be any sequence, such as a DNA sequence capable of forming a B DNA conformation and/or a DNA sequence capable of forming a non-B DNA conformation.

The origin of replication may be spaced at least 150 bp, 200 bp, 300 bp, 500 bp, or 700 bp in a replication direction from a) a target DNA expression cassette, b) a target DNA sequence set, c) a DNA sequence set, or d) a DNA expression cassette. The purpose of spacing at least 150 bp, 200 bp, 300 bp, 500 bp, or 700 bp is to minimize the impact of the origin of replication, when unwound during replication or transcription, on the DNA sequence capable of forming a non-B DNA conformation in the vector, such as ITRs. ITRs may form hairpin or cruciform structures during DNA unwinding, thereby impeding the progress of replication or transcription, and increasing the risk of mutations therein, such as deletions. As the unwinding time increases, the probability of mutations in ITRs also increases. Notably, the unwinding time at the origin of replication may be longer than that at other locations due to factors such as protein binding. Therefore, increasing the spacing distance between the origin of replication and ITRs can reduce mutations in ITRs. If a) the target DNA expression cassette, b) the target DNA sequence set, c) the DNA sequence set, or d) the DNA expression cassette further comprises other DNA sequences capable of forming a non-B DNA conformation, increasing the distance from the origin of replication can also reduce the mutation rate of these sequences. The inventors have demonstrated through experiments that when the positions of the origin of replication and the selectable marker in the vector are swapped to increase the spacing distance between the origin of replication and ITRs in the replication direction, the proportion of nonspecific digestion bands of the vector caused by replication can be reduced from an average of 60.63% to 9.96%. In experiments involving deletion of the lacP functional region in the vector, it can also be seen that the closer the distance between lacP and ITRs, the higher the instability of the ITR sequence induced by, for example, transcription, which indirectly confirms the impact of the spacing distance between the origin of replication and ITRs on the stability of the ITR sequence. The origin of replication enables the vector to replicate in a suitable host cell. The suitable host cell may be a prokaryotic cell such as *Escherichia coli,* or a eukaryotic cell. In some embodiments, the origin of replication may be a pUC origin of replication, which may comprise a sequence such as SEQ ID NO: 23.

In the case of, for example, a shuttle plasmid vector, the vector or vector backbone may further comprise a second origin of replication. By the same reason, the second origin of replication may be spaced at least 150 bp, 200 bp, 300 bp, 500 bp, or 700 bp in a replication direction from a) a target DNA expression cassette, b) a target DNA sequence set, c) a DNA sequence set, or d) a DNA expression cassette. The second origin of replication enables the vector to replicate in a suitable host cell. The suitable host cell may be a prokaryotic cell such as *Escherichia coli,* or a eukaryotic cell.

Depending on the type of origin of replication in the vector or vector backbone, the vector or vector backbone may further comprise a Rep protein expression element correspondingly, including a Rep protein-coding sequence and a corresponding promoter, thereby enabling the vector to be a broad-host-range type.

The vector or vector backbone may further comprise a first terminator and a second terminator. The first terminator and the second terminator are respectively located upstream and downstream of a) a target DNA expression cassette, b) a target DNA sequence set, c) a DNA sequence set, or d) a DNA expression cassette, and configured to terminate transcription outside a) the target DNA expression cassette, b) the target DNA sequence set, c) the DNA sequence set, or d) the DNA expression cassette in the vector or vector backbone upstream and downstream of a) the target DNA expression cassette, b) the target DNA sequence set, c) the DNA sequence set, or d) the DNA expression cassette. Such a design ensures that transcription occurring outside a) the target DNA expression cassette, b) the target DNA sequence set, c) the DNA sequence set, or d) the DNA expression cassette does not induce unwinding within a) the target DNA expression cassette, b) the target DNA sequence set, c) the DNA sequence set, or d) the DNA expression cassette, thereby maintaining the stability of the sequences in the region, particularly the stability of ITRs. The inventors have found through experiments that after adding terminators to both ends of the ITRs, the proportion of nonspecific digestion bands of the vector caused by replication can be further reduced from an average of 9.96% to 7.31%, or from 21.90% to 17.42%. The first terminator may be a forward terminator, a bidirectional terminator, or a reverse terminator. The second terminator may be a forward terminator, a bidirectional terminator, or a reverse terminator. In some embodiments, both the first terminator and the second terminator may be bidirectional terminators. The terminators may be configured according to the specific structure of the vector, such as the transcription direction of the selectable marker. Any terminator may be used, as long as the terminator ensures that transcription outside a) the target DNA expression cassette, b) the target DNA sequence set, c) the DNA sequence set, or d) the DNA expression cassette in the vector is terminated upstream and downstream of the region, thereby ensuring that transcription occurring outside the region does not induce unwinding within the region. A bidirectional terminator may be configured in the universal vector backbone. In some embodiments, the first terminator may be an rrnB T1 terminator and an rrnB T2 terminator. In some embodiments, the second terminator may be a lambda t0 terminator. The rrnB T1 terminator, rrnB T2 terminator, and lambda t0 terminator are known in the art, and may comprise the nucleotide sequences set forth in SEQ ID NO: 24, 25, or 26, respectively.

The cloning site in the vector or vector backbone may be a single cloning site or a multiple cloning site. The cloning site may be configured according to actual needs.

The vector or vector backbone may further comprise a selectable marker. The selectable marker may be an antibiotic resistance gene, such as an ampicillin resistance gene, a tetracycline resistance gene, a chloramphenicol resistance gene, or a kanamycin resistance gene. Preferably, the selectable marker is not present in the genome of the host cell into which the vector or vector backbone is to be transformed/transfected. In some embodiments, the selectable marker is a kanamycin resistance gene. The vector or vector backbone may further comprise a promoter for the expression of the selectable marker upstream of the selectable marker. In addition to confirming whether the vector has been introduced into the host cell, the selectable marker may also promote the replication of the vector under certain circumstances. For example, when the vector or vector backbone comprises a kanamycin resistance gene and the culture medium of the host cell contains kanamycin, the replication of the vector may be accelerated.

The vector or vector backbone may be circular. The vector or vector backbone may be a non-viral vector or a non-viral vector backbone. The vector or vector backbone may be a plasmid vector or a plasmid vector backbone. In some embodiments, the vector or vector backbone may be a circular plasmid vector or a circular plasmid vector backbone.

In view of the "leakiness" of the lac promoter, preferably, the vector or vector backbone does not comprise a fully functional lac promoter. Experiments have demonstrated that after eliminating the activity of the lacP functional region, the proportion of vectors that retain intact ITRs after replication, passage, or packaging can be increased from an average of 50.00% to 75.00%. Even if the vector or vector backbone already comprises two terminators, inactivating the lac promoter region is still advantageous because terminators cannot guarantee 100% termination of transcription.

The first ITR or the derivative sequence thereof, and the second ITR or the derivative sequence thereof, may comprise the same or different nucleotide sequences. The first ITR and the second ITR may be wild-type AAV-ITRs, comprising, for example, a nucleotide sequence set forth in SEQ ID NO: 9. The derivative sequence of the first ITR and the derivative sequence of the second ITR may form a palindromic structure similar to that of a native AAV-ITR, comprising, for example, one palindromic arm and one palindromic stem, or only one palindromic stem, while retaining Rep protein-binding ability. In some embodiments, the derivative sequence of the first ITR and the derivative sequence of the second ITR may comprise a nucleotide sequence set forth in SEQ ID NO: 10, 11, or 12.

In some embodiments, the above vector or vector backbone of the present application may be configured to replicate and/or express a vector carrying ITRs, or to replicate and package an rAAV vector.

A DNA sequence of interest (such as a DNA sequence capable of forming a non-B DNA conformation, such as ITRs) may be introduced into the vector or vector backbone of the present application by methods such as homologous recombination. Vectors or vector backbones obtained by methods such as homologous recombination are also within the scope of protection of the present application.

In the present application, the inventors have found and verified that i) increasing the spacing distance between the origin of replication and the DNA sequence capable of forming a non-B DNA conformation, ii) adding terminators to both ends of the DNA sequence capable of forming a non-B DNA conformation or the expression cassette thereof, and iii) removing the lacP functional region from the vector can significantly enhance the replication and/or expression stability of the poly(A) tail-coding sequence and the ITR sequence, and correspondingly improve the yield of the vector and/or the purity in *in-vitro* transcription, etc. The inventors have reasonable grounds to believe that the above modifications to the vector are also conducive to the replication and/or expression stability of other direct repeat sequences, inverted repeat sequences, and homopurine-homopyrimidine sequences.

According to the vector design of the present application, vectors can be prepared by means such as *de novo* synthesis.

In addition, existing vectors can be modified according to the vector design of the present application. For example, in an existing vector or vector backbone, i) depending on specific cases, the origin of replication in the vector or vector backbone may be spaced at least 150 bp, 200 bp, 300 bp, 500 bp, or 700 bp from the cloning site or the DNA sequence capable of forming a non-B DNA conformation; ii) in the case of comprising a lac promoter, the lac promoter may be inactivated; and/or iii) depending on specific cases, terminators may be respectively added upstream and downstream of the cloning site, the DNA sequence capable of forming a non-B DNA conformation, or the expression cassette of the DNA sequence capable of forming a non-B DNA conformation in the vector or vector backbone, thereby ensuring that transcription outside the above regions in the vector or vector backbone is terminated upstream and downstream of these regions.

The vectors of the present application, including those prepared or modified, can be used for replicating and/or expressing a target DNA sequence, particularly a target DNA fragment comprising a direct repeat sequence, a homopurine-homopyrimidine sequence, or an inverted repeat sequence. The direct repeat sequence or homopurine-homopyrimidine sequence may be, for example, a poly(A) tail-coding sequence, a polyA, a polyG, a polyC, or a polyT. The inverted repeat sequence may be, for example, an ITR or an shRNA-coding sequence.

When using the vectors of the present application to replicate and/or express a target DNA sequence, the process involves the transformation or transfection of a host cell with the vector, the culture of a host cell, *in-vitro* transcription, etc. The corresponding conditions are known to those skilled in the art and can be adjusted according to actual circumstances.

The vectors of the present application, including those prepared or modified, can be used for preparing a recombinant adeno-associated virus (rAAV) vector. Specifically, the method may comprise: i) providing a vector of the present application that comprises a target DNA sequence set or a target DNA expression cassette flanked by ITRs, or inserting a DNA sequence to be replicated and/or expressed into a cloning site of the target DNA sequence set or target DNA expression cassette flanked by ITRs to obtain a new vector; ii) optionally, transforming/transfecting the vector into a host cell, and culturing the host cell under conditions conducive to vector replication; and iii) harvesting the vector from i) or ii), co-transforming/co-transfecting the vector with a packaging plasmid and a helper plasmid into the host cell, and culturing the host cell under conditions conducive to vector packaging. In the case that the amplification is not required, step ii) may be omitted, and three-plasmid packaging may be performed directly. In the above steps, the culture conditions of the host cell and the selection of the packaging plasmid and helper plasmid are known to those skilled in the art and can be adjusted according to actual circumstances. The packaging of the adeno-associated virus vector may also adopt methods other than three-plasmid packaging, as long as the purpose of vector packaging can be achieved.

Unless otherwise defined, all technical and scientific terms used herein have the meanings commonly understood by those of ordinary skill in the art to which the present disclosure pertains.

Unless otherwise stated, the following sequences are all sequences on the sense strand of the vector.

### Example 1: Adjustment of position of origin of replication (Ori) in vector to enhance stability of Poly(A) tail-coding sequence

The Ori sequence is generally rich in adenine (A) and thymine (T). DNA sequences rich in A-T are more prone to unwinding, and the unwound region of the Ori provides a space for protein binding at the beginning of replication, thereby initiating plasmid replication. Moreover, literature indicates that Ori elements may have promoter activity. When the 3' end of the Ori is adjacent to the poly(A) tail-coding sequence, this coding sequence is highly prone to deletion or mutation during plasmid replication.

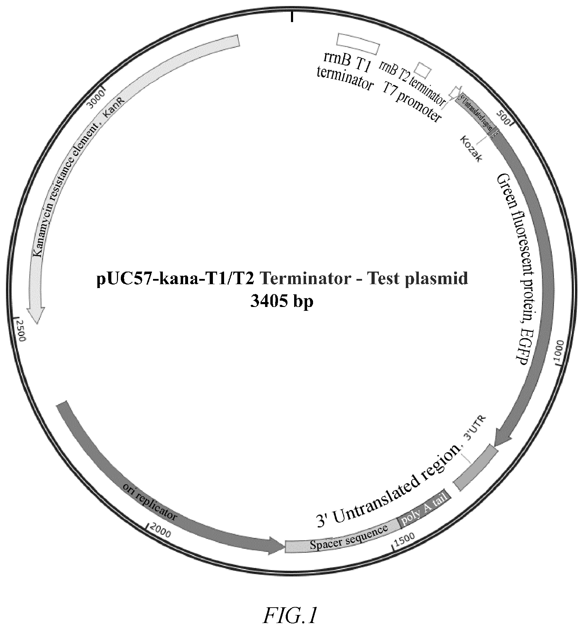

The pUC57-kana-T1/T2 terminator vector (Nanjing GenScript, Cat. No.: C240PHC090-2) was taken to construct plasmid vectors with the eGFP-120 A-mRNA-coding sequence (containing 120 consecutive adenines) inserted and having varying spacing distances between the 3' end of Ori and the poly(A) tail structural sequence. These vectors were used to test the effect of the distance between the 3' end of the Ori sequence and the poly(A) tail structural sequence on the stability of the poly(A) tail structural sequence. The map of the constructed vectors is shown in FIG. 1, where the spacer sequence between the 3' end of the Ori sequence and the poly(A) tail structural sequence is 100 bp, 247 bp, 500 bp, or 700 bp (set forth in SEQ ID NOs: 1-4, respectively, where the italicized and underlined parts at both ends denote sequences for homologous recombination with the pUC57-kana-T1/T2 terminator vector, and the middle part denotes a random sequence.

Specifically, 1.5 ng of the pUC57-kana-T1/T2 terminator vector was taken, followed by the addition of 5 µL of 10× cutsmart buffer, 2.5 µL of XhoI, and 2.5 µL of ApaLI. Water was added to a final volume of 50 µL. Then, the mixture was incubated at 37°C for 1 h to digest the pUC57-kana-T1/T2 terminator vector using XhoI and ApaLI. The obtained linearized vector product was recovered using the AxyPrep-96 DNA gel extraction kit (Axygen Biosciences, Inc.). Subsequently, 0.8 ng of the recovered linearized vector was taken, followed by the addition of 0.8 ng of each of the above artificially synthesized spacer sequences (containing, at both ends, 30-50 bp homologous arms homologous to both ends of the vector) and 10 µL of Genbuilder Plus enzyme + buffer mix (Jiangsu Genscript Biotech Co., Ltd.). Water was added to a final volume of 20 µL. Then, the mixture was incubated at 50°C for 60 min to ligate the spacer sequence fragment into the linearized vector fragment via homologous recombination. The obtained ligation product was transformed into *Escherichia coli* TOP10 competent cells, and the cells were cultured on an LB plate at 37°C for 12 h.

Colonies were screened by PCR. Specifically, the colony PCR reaction system (all reagents were purchased from Jiangsu Genscript Biotech Co., Ltd.) included 1 µL of forward primer, 1 µL of reverse primer, 1 µL of Taq DNA polymerase, 1 µL of 10× buffer, and 1 µL of dNTPs. Water was added to a final volume of 10 µL. The following program was run on a PCR instrument (2720 Thermo Cycler): 95°C for 1 min; 30 cycles of 95°C for 5 s, 55°C for 15 s, and 72°C for 1 min; 72°C for 1 min; and hold at 4°C. Forty-eight clones with the spacer sequences inserted were selected for each recombinant plasmid vector, separately inoculated into 4 mL of fresh LB liquid medium, and cultured with shaking at 37°C for 12 h.

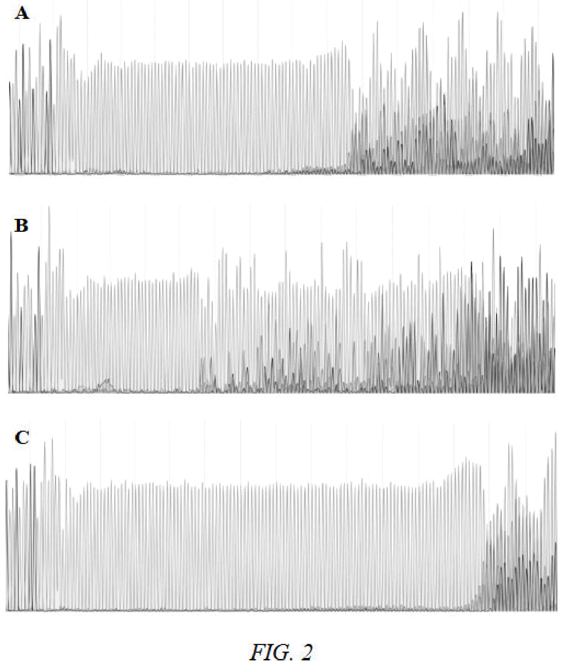

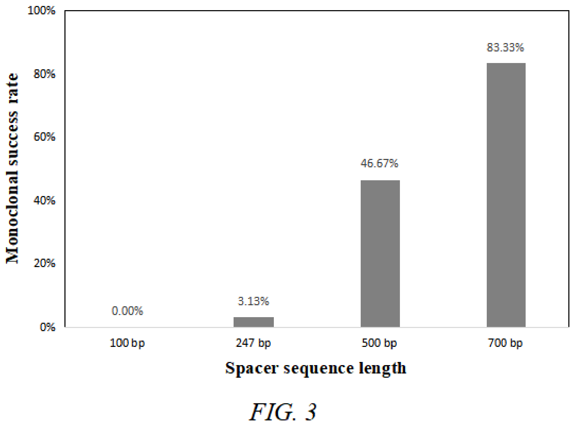

Plasmid extraction was performed on the cultured bacterial broth using the AxyPrep Plasmid Miniprep kit (Axygen Biosciences, Inc.). Subsequently, the obtained plasmids were subjected to Sanger sequencing using an Applied Biosystems 3730XL DNA analyser. The sequencing results were structurally aligned and analysed using SnapGene software. A clone was identified as positive when the sequenced sequence was identical to the reference sequence and the sequencing background peak of the Poly(A) tail did not exceed 20% of the main peak. Based on this, the monoclonal success rate for the construction of each of the recombinant plasmid vectors was calculated as the ratio of the number of positive clones to the total number of picked clones. FIG. 2 shows the sequencing chromatograms of positive clones (C: the poly(A) tail-coding sequence is relatively pure with a sequencing background peak lower than 20% of the main peak) and failed clones (A: the poly(A) tail-coding sequence is deleted; B: the poly(A) tail-coding sequence is impure with a sequencing background peak higher than 20% of the main peak). The results are as shown in FIG. 3. When the spacing length between the 3' end of Ori and the poly(A) tail-coding sequence was 100 bp, the monoclonal success rate of the plasmid was 0%, indicating poor sequencing purity of the poly(A) tail-coding sequence in the plasmid, with a high sequencing background peak. As the spacing length increased, the cloning success rate of the plasmid rose gradually. When the spacing length reached 700 bp, the monoclonal success rate of the plasmid reached 83.33%, indicating high sequencing purity of the Poly(A) tail-coding sequence, with a sequencing background peak lower than 10% of the main peak of the poly(A) tail. This indicates that the greater the distance between Ori and the poly(A) tail-coding sequence, the higher the stability of the poly(A) tail-coding sequence in the plasmid.

### Example 2: Addition of terminator element to enhance stability of Poly(A) tail-coding sequence in vectors

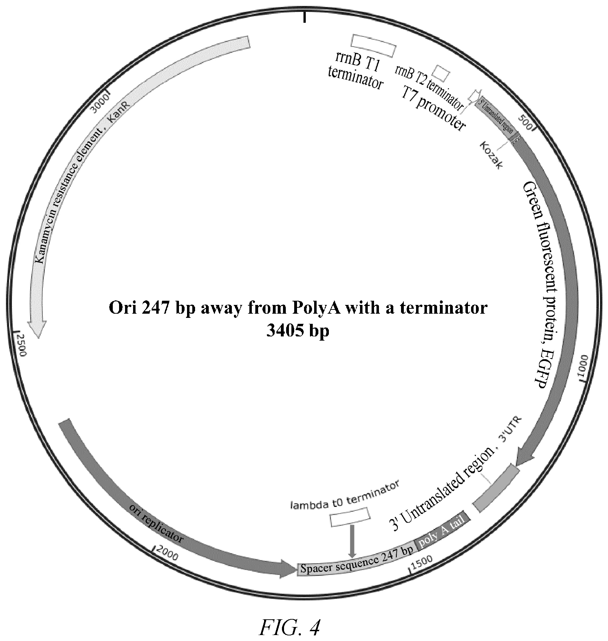

The recombinant plasmid vector with a 247 bp spacer sequence constructed in Example 1 was taken, and the 247 bp spacer sequence in the plasmid was replaced with a 247 bp sequence comprising the lambda t0 terminator (with the sequence as set forth in SEQ ID NO: 5, in which the bold and underlined sequence in the middle part denotes the lambda t0 terminator, and the underlined and italicized parts at both ends are used for homologous recombination). The plasmid map is as shown in FIG. 4.

As described in Example 1, the obtained plasmid ligation product was transformed into *Escherichia coli* TOP10 competent cells, and the cells were cultured on an LB plate at 37°C for 12 h. Colonies were screened by PCR. The selected clones with the terminators inserted were inoculated into 4 mL of fresh LB liquid medium and cultured with shaking at 37°C for 12 h. Plasmid extraction was performed on the cultured bacterial broth, followed by Sanger sequencing and analysis.

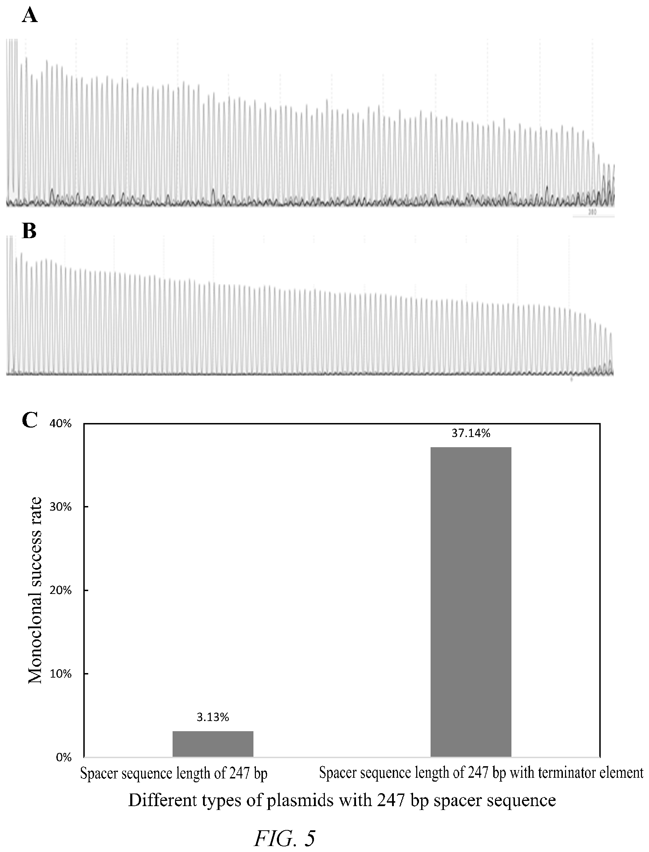

The results are as shown in FIG. 5 (A-C). The addition of a terminator element between Ori and the poly(A) tail-coding sequence increased the monoclonal success rate from 3.13% to 37.14%. Compared to the 247 bp spacer sequence without a terminator, the plasmid exhibited a lower sequencing background peak and higher purity of the Poly(A) tail-coding sequence. This indicates that the addition of a terminator element can enhance the stability of the poly(A) tail-coding sequence, for example, by blocking the impact of leaky promoter activity in the vector backbone on the downstream functional region.

### Example 3: Deletion of lac promoter functional region to enhance stability of poly(A) tail-coding sequence in vectors

As described in Example 1, a vector with the eGFP-120A-mRNA-coding sequence inserted was constructed. In the pUC57-kana-T1/T2 terminator vector without further modifications, the region between Ori and the poly(A) tail-coding sequence is a 465 bp lac promoter functional region, which comprises, in the 5' to 3' direction, a CAP-binding site, the lac promoter, the lactose operator, and an M13 rev element. The results showed that the monoclonal construction success rate of the plasmid was 31.25%.

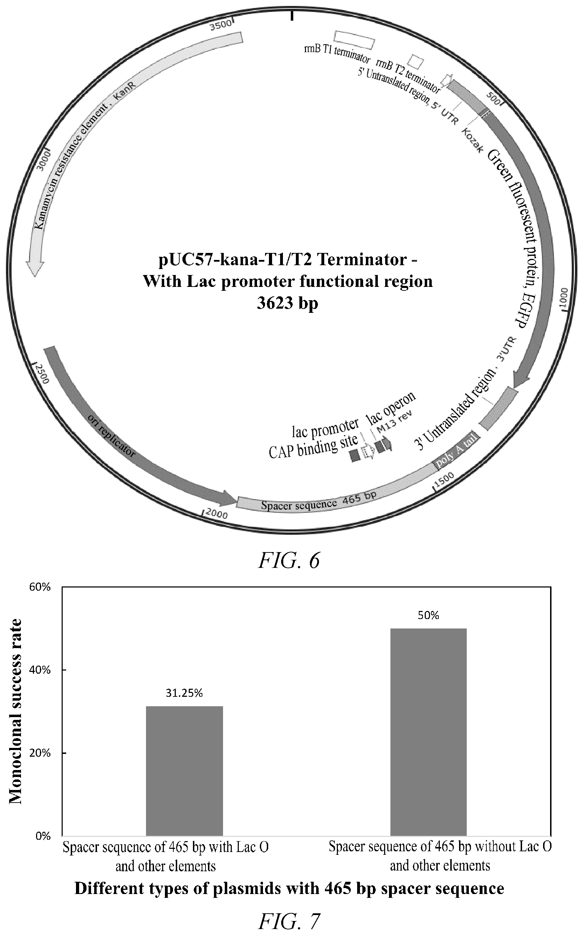

By means of base replacement, the sequence of the lac promoter functional region was modified to SEQ ID NO: 6 to inactivate the functional region, resulting in the construction of a new vector (the map is as shown in FIG. 6). Subsequently, clones with altered sequences of the lac promoter functional region were selected, cultured with shaking in LB medium, followed by plasmid extraction and sequencing analysis.

The results are as shown in FIG. 7. The inactivation of the lac promoter functional region increased the monoclonal success rate from 31.25% to 50%, thereby enhancing the stability of the poly(A) tail-coding sequence in the plasmid vector.

### Example 4: Construction of GS-MK, GS-PV, and GS-CMV series vectors

Based on the results of Examples 1, 2, and 3, optimized vectors were constructed.

Specifically, non-essential elements such as the M13 rev element, lac promoter, lactose operator, and CAP-binding site were deleted from the pUC57-kana plasmid vector. T1/T2 terminator and lambda t0 terminator elements were added upstream and downstream of the multiple cloning site, respectively, and the spacing between the 3' end of Ori and the multiple cloning site was increased to ensure that after inserting a sequence (e.g., an mRNA-coding sequence) into the multiple cloning site, the distance between the 3' end of Ori and the poly(A)-coding sequence would exceed 700 bp.

In addition, synonymous mutations were introduced into IIS sites, such as BsaI, BspQI, BspMI, BpiI and BsmbI, on the vector backbone to eliminate these restriction sites. Meanwhile, IIS enzyme recognition sites were added to the ends of the target DNA sequences to be inserted, such as the mRNA-coding sequence and the poly(A) tail-coding sequence. In this way, after linearization of the vector with the target DNA sequence inserted, the IIS-type restriction endonuclease recognises only outside the poly(A) tail-coding sequence and cleaves within the poly(A)-coding sequence, which results in a relatively pure restriction fragment where the polyT sequence does not incorporate non-T sequences, thereby obtaining a pure poly(A) tail. The pure poly(A) tail can maintain a high level of mRNA protein expression (Holtkamp S et al., (2006) Modification of antigen-coding RNA increases stability, translational efficacy, and T-cell stimulatory capacity of dendritic cells. Blood. 108(13): 4009-4017). This design also facilitates Golden Gate cloning of the vector.

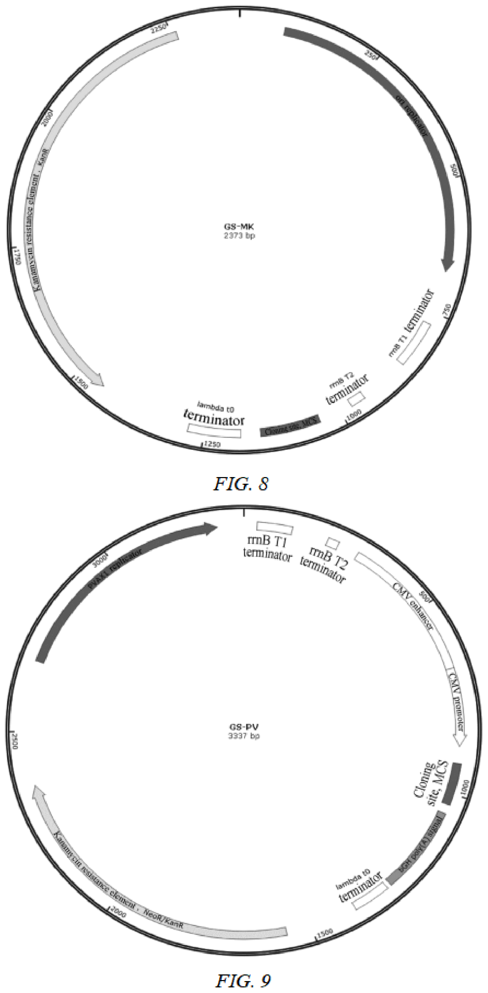

The finally constructed vector, designated as the GS-MK vector, comprised, in the 5' to 3' direction: an origin of replication, rrnB T1 terminator and rrnB T2 terminator elements, a multiple cloning site (MCS), a lambda t0 terminator element, and a kanamycin resistance element. The plasmid map is as shown in FIG. 8.

Similarly, starting with the PVAX1 vector, synonymous mutations were introduced to the BsaI, BspQI, BspMI, and SpeI restriction sites on the vector. Subsequently, the T1/T2 terminator and lambda t0 terminator were inserted upstream of the CMV promoter and downstream of the BGH poly(A) tail signal, respectively. The finally constructed GS-PV vector comprised, in the 5' to 3' direction: an origin of replication, an rrnB T1 terminator and an rrnB T2 terminator, a CMV enhancer and promoter, a multiple cloning site, a bGH poly(A) signal, a lambda t0 terminator, and a kanamycin resistance element. The map is as shown in FIG. 9.

In addition, based on the GS-PV vector, a T-C mutation in the origin of replication of the original PVAX1 vector (compared to that of the pUC origin of replication) was repaired. The newly obtained vector was designated as GS-CMV. This vector can increase the plasmid copy number and reduce the risk of genomic contamination and insufficient yield during plasmid extraction.

### Example 5: Functional test of GS-MK, GS-PV, and GS-CMV series vectors

Various poly(A) tail-coding sequences were inserted into the multiple cloning sites of the GS-MK, GS-PV, and GS-CMV vectors, including 100 adenines (100A), 120 adenines (120A), a segmented sequence of 30 adenines + 30 adenines + 43 adenines (30A + 3 non-A nucleotides + 30A + 3 non-A nucleotides + 43A), and a segmented sequence of 31 adenines + 71 adenines (31A + 8 non-A nucleotides + 71A).

Specifically, the GS-MK vector was digested with NheI and ApaL1 restriction enzymes to obtain a linearized vector. A gene fragment to be inserted was artificially synthesized, which comprised, in the 5' to 3' direction: a multiple cloning site, an eGFP sequence (the function of this sequence was to increase the number of spacer bases between the 3' end of Ori and the poly(A) tail-coding sequence, ensuring that the distance between the two would be > 700 bp), the above various poly(A) tail-coding sequences, and an IIS enzyme recognition site. Recombinant plasmids were constructed using the Gibson assembly method. Taking GS-MK-100A-BspQI as an example, the inserted gene sequence is as set forth in SEQ ID NO: 27.

Similarly, the GS-PV vector or GS-CMV vector was digested with NheI and ApaL1 restriction enzymes to obtain linearized vectors. A gene fragment to be inserted was artificially synthesized, which comprised, in the 5' to 3' direction: a multiple cloning site, the above various poly(A) tail-coding sequences, and an IIS enzyme recognition site. Recombinant plasmids were constructed using the Gibson assembly method. Taking GS-CMV-100A-BspQI as an example, the inserted gene sequence is as set forth in SEQ ID NO: 28.

As described above, the obtained plasmids with the poly(A) tail-coding sequences were transformed into *Escherichia coli* TOP10 competent cells, which were then cultured on LB plates at 37°C for 12 h. Colonies were screened by PCR. The selected clones with the poly(A) tail-coding sequences inserted were inoculated into 4 mL of fresh LB liquid medium and cultured with shaking at 37°C for 12 h. Plasmid extraction was performed on the cultured bacterial broth, followed by Sanger sequencing and analysis.

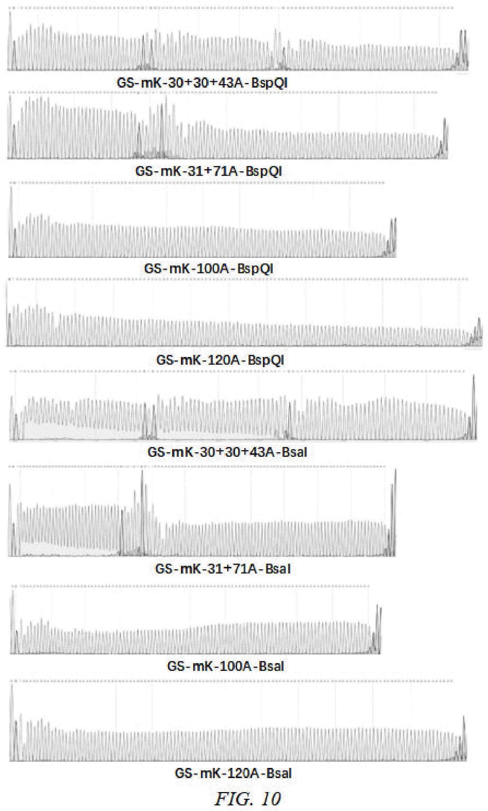

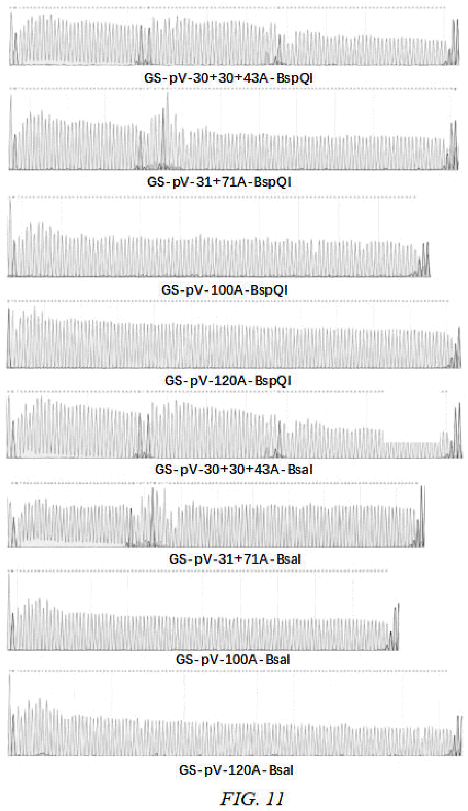

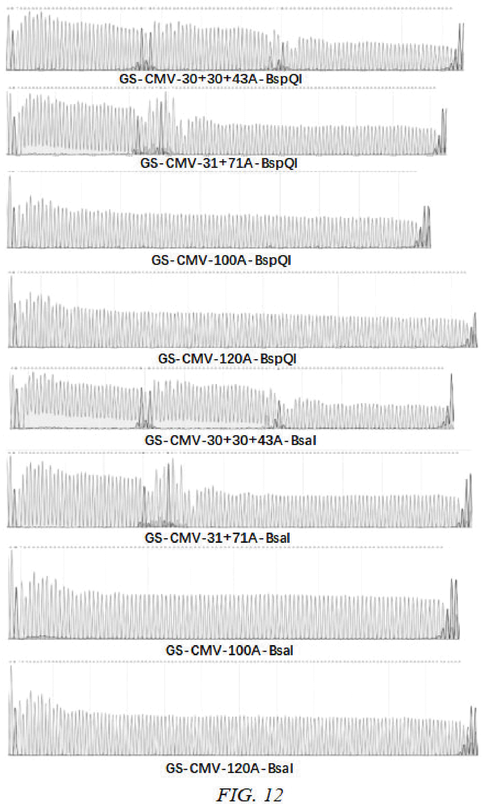

The sequencing chromatograms of the three vectors are as shown in FIG. 10 to FIG. 12, respectively. Overall, the sequencing background peaks of the plasmids are relatively low, indicating high purity of the Poly(A) tail-coding sequences.

### Example 6: Comparison of GS-MK, GS-PV, and GS-CMV series vectors with commercial vectors

The eGFP-100A mRNA-coding sequence was inserted into the GS-MK, GS-PV, and GS-CMV vectors constructed in Example 4, as well as the commercial Puc57-kana and PVAX1 vectors. Subsequently, comparisons and analyses were performed on the following parameters of different vectors: the monoclonal success rate, the passage stability of the poly(A) tail-coding sequence, the plasmid yield, *in-vitro* transcription (IVT) purity, and the eukaryotic protein expression level of the mRNA. The eGFP-100A mRNA-coding sequence inserted into the above vectors is as set forth in SEQ ID NO: 29, which comprises a T7 promoter, a 5' UTR, an eGFP-coding sequence, a 3' UTR, a poly(A) tail-coding sequence, and a linearization restriction site.

According to the map, Puc57-kana comprises LacZ but no terminator. The eGFP-100A mRNA-coding sequence is inserted into the multiple cloning site, and after insertion, the distance between the poly(A) tail-coding sequence and the 3' end of the origin of replication is about 400 bp. According to the map, the pVAX1 vector does not comprise a terminator. The eGFP-100A mRNA-coding sequence is inserted into the multiple cloning site, and after insertion, the distance between poly(A) and the 3' end of the origin of replication is about 700 bp.

The method for detecting the passage stability of the poly(A) tail-coding sequence was as follows: The plasmid was transformed into competent cells and then spread on an LB plate. Single colonies were picked and inoculated into LB medium, and cultured at 37°C with shaking at 150 rpm for 12 h (first-generation bacterial broth). Subsequently, 100 µL of the bacterial broth was transferred into 50 mL of LB medium for passage culture, and cultured at 37°C with shaking at 150 rpm for 12 h (second-generation bacterial broth). Then, 100 µL of the second-generation bacterial broth was transferred into another 50 mL of LB medium for further passage, and cultured at 37°C with shaking at 150 rpm for 12 h (third-generation bacterial broth). Finally, 2 mL of the bacterial broth from each generation was collected for plasmid extraction, followed by Sanger sequencing verification. The passage stability was calculated as the ratio of the number of positive clones to the total number of picked colonies.

The method for measuring plasmid yield was as follows: The plasmid was transformed into competent cells and then spread on a plate. Single colonies were picked and inoculated into 400 mL of 3× LB medium, and cultured in an incubator at 37°C with shaking at 150 rpm for 13 h to enrich the bacterial broth. Then, plasmid extraction was performed to obtain the DNA yield.

The descriptions of IVT and purity detection were as follows. Specifically, in a 300 µL enzymatic digestion system containing 100 µg of plasmid, 10 µL of BspQI enzyme, 30 µL of cutsmart buffer, and nuclease-free water to bring the volume to 300 µL, the enzymatic digestion was performed at 37°C overnight. The linearized product was recovered using the AxyPrep-96 DNA gel extraction kit. According to the instructions of the mMessager mMachine T7 Ultra *in-vitro* transcription kit, the linearized plasmid product was subjected to *in-vitro* transcription and purification. The purity of the IVT product was detected using an Agilent 5200 fragment analyser.

Finally, the eukaryotic expression level of the mRNA was determined. Specifically, 293T cells were seeded and cultured in an incubator at 37°C with 5% CO₂ until the confluency reached 70%-80%. Cell transfection was performed according to the instructions of the Lipofectamine 2000 kit. The transfection complexes were prepared as follows: Solution A contained 5 µL of Opti- and 0.5 µL of Lip2000, while Solution B contained 5 µL of Opti-MEM and 0.1 µg of mRNA. After mixing Solution A and Solution B, the mixture was incubated at room temperature for 5 min. Subsequently, the mRNA product obtained from the above IVT product was added to the cells, which were then cultured further in the incubator. 24 h and 48 h after transfection, the GFP fluorescence transfection efficiency was observed using an inverted fluorescence microscope, and the GFP fluorescence intensity was read using Image J software.

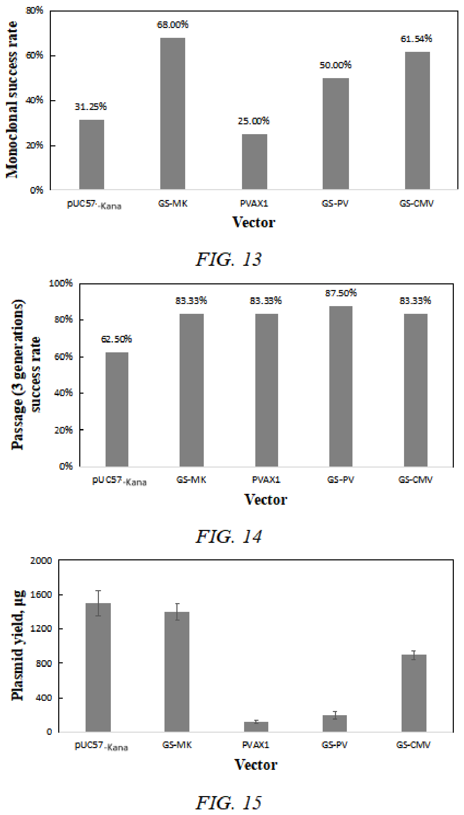

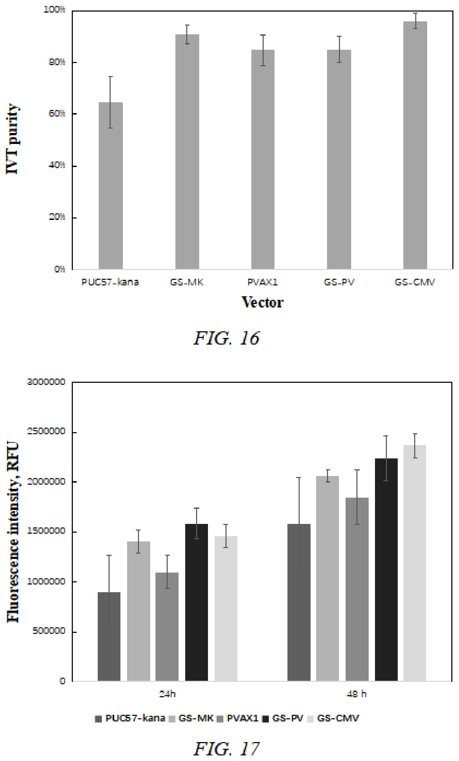

The experimental data indicated that the monoclonal success rate of the GS-MK, GS-PV, and GS-CMV vectors was significantly higher than that of the pUC57-kana and PVAX1 vectors (FIG. 13); the passage stability of the poly(A) tail-coding sequence in the recombinant plasmids of the GS-MK, GS-PV, and GS-CMV vectors was significantly higher than that in the pUC57-kana vector (FIG. 14); under the same shaking fermentation conditions, the yield of the recombinant plasmids of the GS-MK and GS-CMV vectors was significantly higher than that of the PVAX1 and GS-PV vectors (FIG. 15); moreover, due to the higher stability of the Poly(A) tail-coding sequence in the plasmids of the GS-MK, GS-PV, and GS-CMV vectors, the IVT purity and eukaryotic protein expression effect of these vectors were also significantly superior to those of the pUC57-Kna vector (FIG. 16 and FIG. 17).

### Example 7: Deletion of lac promoter functional region to enhance structural stability of ITRs in vectors

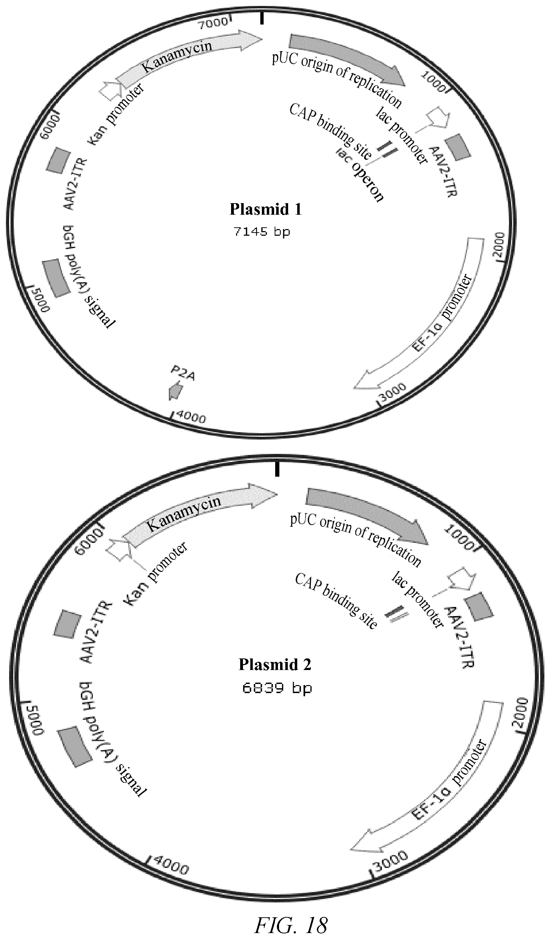

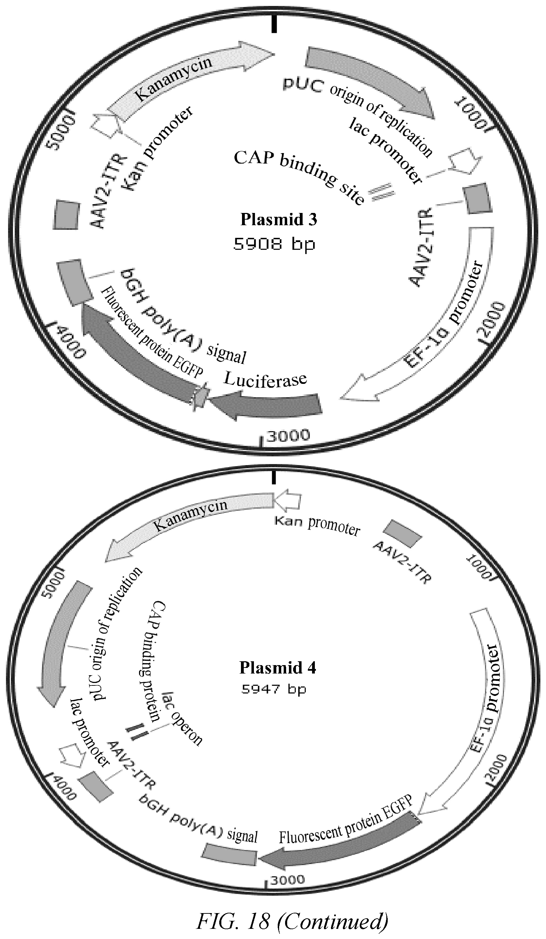

Four plasmids comprising the lac promoter (lacP) functional region element (SEQ ID NO: 7) and AAV ITRs were selected. The plasmid maps are as shown in FIG.18, and the distance from the lacP to the nearest ITR in the direction of transcription initiation is recorded in Table 1.

The lac promoter sequence was replaced with SEQ ID NO: 8, and the lac operator, lac promoter, and CAP-binding site within the lacP functional region were deleted to construct new plasmids.

The plasmids before and after construction were separately transformed into the GenITRV1 strain (Nanjing GenScript), and cultured at 37°C overnight. Eight single colonies were picked, and cultured in LB medium at 37°C for 12 h. Plasmid extraction was performed, and the plasmids were treated with a specialized ITR kit (Nanjing GenScript), and subjected to Sanger sequencing. Clones without deletions or mutations within the ITR region were determined to pass Sanger sequencing, while those with deletions or mutations within the ITR region, as detected by Sanger sequencing, were determined to fail. The Sanger sequencing pass rates of the plasmids before and after lacP deletion were statistically analysed. The results are summarised in Table 1.

**Table 1. Sanger sequencing pass rates of plasmid clones before and after deletion of lacP functional region**

| | Distance from lacP to ITR | Sanger sequencing pass rate | |
|---|---|---|---|
| | | Before lacP deletion | After lacP deletion |
| Plasmid 1 | 66 bp | 62.50% | 87.50% |
| Plasmid 2 | 42 bp | 25% | 50% |
| Plasmid 3 | 66 bp | 62.50% | 87.50% |
| Plasmid 4 | 50 bp | 50% | 75% |
| Mean value | N/A | 50.00% | 75.00% |

As can be seen from the results, the closer the lacP element is to the ITR, the lower the Sanger sequencing pass rate, demonstrating that the lacP element has a certain impact on the stability of ITR. After deletion of the lacP element, the mean Sanger sequencing pass rate of the four plasmids increased from 50% to 75%, which significantly enhanced the stability of the ITR structure.

### Example 8: Design and construction of ITR stabilized vectors

### Modification of pUC57 vector

The pUC57 plasmid used as a control in Example 6 is a commonly used vector in molecular biology experiments, which has wide applications in fields such as clone expression, DNA sequencing, and gene mutation, and has characteristics such as high copy number, ease of manipulation, multiple cloning sites, and selectable marker genes. AAV-ITR shuttle plasmids carrying target genes, designed based on the prokaryotic vector backbone of the pUC57 vector, are extensively used in AAV gene therapy. However, the ITR sequence exhibits replication instability in *Escherichia coli* due to the unique nature thereof, leading to susceptibility to deletions and thus requiring optimization and modification.

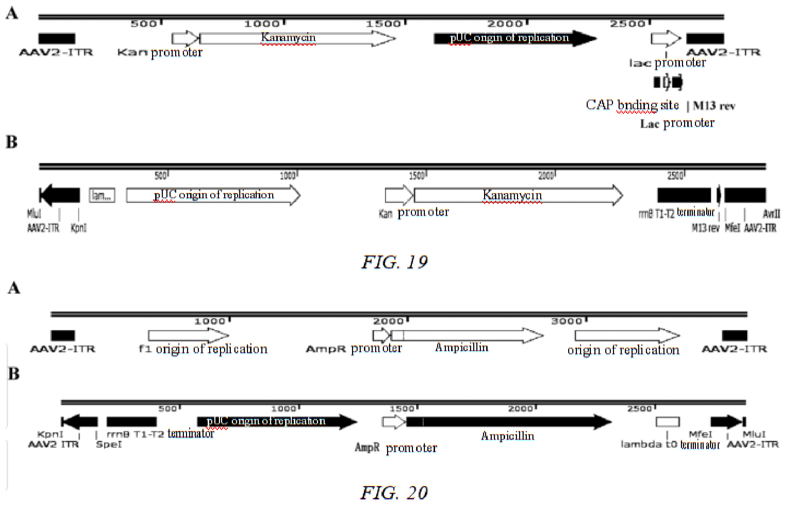

The prokaryotic vector backbone of pUC57 Kana (GenBank: LT671993.1) was selected for modification. Firstly, ITR sequences were added to both ends of the obtained vector, specifically the wild-type AAV2-ITR sequence (SEQ ID NO: 9) or truncated AAV2-ITR sequences (SEQ ID NO: 10, 11, or 12), as shown in FIG. 19 (A). Secondly, the position of the pUC Ori was adjusted to the upstream of the KanR gene, so as to increase the distance between the end of the pUC Ori and the front end of the 3' ITR sequence to 1747 bp. Then, the lac promoter in the plasmid was replaced with a nonsense sequence of the same length (SEQ ID NO: 13). This replacement sequence is derived from a natural *Escherichia coli* plasmid and has no homology with the *Escherichia coli* genome or the pUC57 plasmid vector. The formed new vector backbone was designated as GenS-pUC57-AAV-NoTer.

Based on GenS-pUC57-AAV-NoTer, terminator sequences were inserted downstream of the 5' ITR and upstream of the 3' ITR, respectively, to obtain GenS-pUC57-AAV-Ter, as shown in FIG. 19 (B).

Subsequently, the CMV enhancer, chicken β-actin promoter, EGFP, and bGH poly(A) signal were inserted between the 5' ITR and 3' ITR of the vectors GenS-pUC57-AAV-Ter and GenS-pUC57-AAV-NoTer. Moreover, synonymous mutations were introduced into the IIS enzyme recognition sites, such as BsaI, AarI, BpiI, BsmBI, and BspQI, on the vector backbones, so as to eliminate these restriction sites. Universal vectors GenS-pUC57-AAV-Noter-CMV (SEQ ID NO: 14, comprising the ITR of SEQ ID NO: 9) and GenS-pUC57-AAV-Ter-CMV (SEQ ID NO: 15, comprising the ITR of SEQ ID NO: 9) were thus prepared. The vectors were synthesized *de novo* and assembled by the Gene Synthesis Department of Nanjing GenScript Biotech Co., Ltd.

### Modification of pSub201 vector

Samulski, the founder of AAV gene therapy, first described pSub201 (addgene.org/vector-database/4231/) in the Journal of Virology 61(10): 3096-3101 in October 1987. This vector comprises the coding region of the wild-type genome of AAV-2 adeno-associated virus and cis-acting terminal repeat sequences (ITRs). This vector is an ideal choice for cloning due to its design, which allows for retaining intact AAV-ITRs in the plasmid backbone by restriction digestion with XbaI, while removing the coding region of the AAV genome and replacing the same with a gene of interest (GOI). Therefore, this vector has since been widely selected as a template for cloning in rAAV viral packaging and gene therapy applications, such as the pAAV-tTA plasmid (GenBank: MZ708023.1). However, the prokaryotic backbone of this vector has certain design flaws, which can lead to the instability of the ITR structure. Therefore, specific modifications are required to obtain an ITR structure with high integrity.

The prokaryotic vector backbone of the pAAV-tTA plasmid (GenBank: MZ708023.1) (FIG. 20 (A)) was taken. The phage-derived f1 ori was replaced with an additional sequence (SEQ ID NO: 16, comprising no moieties homologous to the genome of *Escherichia coli).* The pUC Ori on the right side was swapped in position with the ampicillin resistance gene. Elements such as the CMV enhancer, chicken β-actin promoter, EGFP, and bGH poly(A) signal were inserted between the 5' ITR and 3' ITR. Moreover, synonymous mutations were introduced into the commonly used IIS enzyme recognition sites (such as BsaI, AarI, BpiI, BsmBI, and BspQI) and the restriction sites (such as SmaI, AhdI, BglI, and BssHII) frequently used to verify the structural integrity of the ITRs in the vector. The obtained vector was designated as GenS-pSUB201-AAV-NoTer (SEQ ID NO: 17, comprising the ITRs of SEQ ID NO: 10 and 11). Based on this, terminator sequences were added downstream of the 5' ITR and upstream of the 3' ITR to obtain the vector GenS-pSUB201-AAV-Ter (SEQ ID NO: 18, comprising the ITRs of SEQ ID NO: 10 and 11), as shown in FIG. 20 (B). The distance between the pUC Ori and the 3' ITR in this vector is 1814 bp. The newly designed vectors were synthesized *de novo* and assembled by the Gene Synthesis Department of Nanjing GenScript Biotech Co., Ltd.

### Example 9: Structural stability test of ITRs in pUC57 series vectors

pUC57 Kana from Example 8 (with two ITRs inserted, i.e., GenS-pUC57-AAV-NoTer prior to the swapping of the positions of the Ori and KanR genes), GenS-pUC57-AAV-NoTer, and GenS-pUC57-AAV-Ter were taken. Through homologous recombination, gene fragment 1, gene fragment 2, gene fragment 3, or gene fragment 4 (as shown in SEQ ID NO: 19-22, respectively) was inserted between the two ITRs.

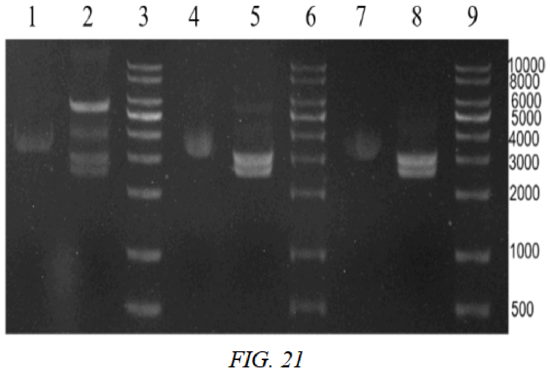

The obtained vectors were cultured in a flask containing 3× LB medium with shaking at 37°C for 16-18 h. Plasmid extraction was performed, and the plasmids were digested with SmaI. The Smal restriction site is located on the ITR, and complete or partial sequence deletion of the ITR would lead to digestion failure. The digested products were subjected to agarose gel electrophoresis. Some results are as shown in FIG. 21. Bands other than the target band were collectively referred to as nonspecific bands. The proportion of nonspecific bands was analysed using Gel Image System (GIS) software. The specific values are listed in Table 2.

**Table 2. Proportion of nonspecific bands from SmaI digestion of pUC57-related vectors**

| | pUC57 Kana | GenS-pUC57-AAV-NoTer | GenS-pUC57-AAV-Ter |
|---|---|---|---|
| Gene 1 | 100% | 12.01% | 9.56% |
| Gene 2 | 78.87% | 21.65% | 19.67% |
| Gene 3 | 36.79% | 6.18% | 0.00% |
| Gene 4 | 26.84% | 0.00% | 0.00% |
| Mean value | 60.63% | 9.96% | 7.31% |

It could be seen from the results that the unmodified pUC57 Kana vector showed a high proportion of nonspecific bands in cloning, and in some cases, the proportion even reached 100%. In contrast, the optimized vectors exhibited a significant reduction in the proportion of nonspecific bands. This indicates that the optimizations of the vector in terms of Ori position, terminator addition, and lacP deletion significantly enhanced the stability of the ITR structure. In particular, the GenS-pUC57-AAV-Ter vector with terminators added at both ends of the ITR showed a significantly lower proportion of nonspecific bands than the GenS-pUC57-AAV-NoTer vector.

Further, the above extracted plasmids were sequenced using an ITR sequencing kit (Nanjing GenScript). The sequencing results showed that in the sequencing of the unmodified pUC57 Kana vector, phenomena such as deletions, background peaks, double peaks, and unreadable regions occurred. The Sanger sequencing pass rate was 0%, and the above issues still tended to occur when additional clones were picked. In contrast, the optimized vectors exhibited sequencing chromatograms with no obvious background peaks, high ITR integrity, and a 100% Sanger sequencing pass rate.

In addition, the shake flask extraction yields of the three vectors were determined. Specifically, clones with correctly synthesized gene fragments from each of the three vectors were transformed into GenITRv1 competent cells, and cultured at 37°C overnight. Single colonies were picked into test tubes containing 4 mL of LB medium and cultured at 30°C with shaking at 180 rpm for 16 h. Then, 400 µL of the cultured seed broth was inoculated into a 400 mL flask containing 3× LB medium, and cultured at 37°C with shaking at 200 rpm for 13 h. Subsequently, the bacterial cells were harvested by centrifugation and lysed using the alkaline lysis method, and the plasmid was precipitated with isopropanol. Finally, the plasmid pellet was dissolved in TE buffer, and the plasmid concentration was measured using a UV spectrophotometer. The results are summarised in Table 3. It could be seen that compared to the unmodified pUC57 Kana vector, the yields of the two optimized vectors were increased by 44% and 26%, respectively.

**Table 3. Shake flask extraction yields of pUC57-related vectors (µg)**

| | pUC57 Kana | GenS-pUC57-AAV-NoTer | GenS-pUC57-AAV-Ter |
|---|---|---|---|
| Gene 1 | 469.1 | 1014.2 | 731.1 |
| Gene 2 | 367.9 | 546.4 | 540.3 |
| Gene 3 | 500.4 | 699.1 | 637.3 |
| Gene 4 | 724.9 | 715.5 | 698.7 |
| Mean yield | 515.6 | 743.8 | 651.9 |

Further, the impact of the three vectors on subsequent AAV packaging was evaluated. Specifically, a three-plasmid packaging system was used, with the three vectors (comprising inserted gene 1) serving as shuttle plasmids. In addition, the packaging plasmids pRC2-mi342 (Takara, Cat. No.: 6230) for AAV2 and pRC-DJ (Cell Biolabs INC, Cat. No.: VPK-420-DJ-8) for AAVDJ, along with the helper plasmid pHelper (Takara, Cat. No.: 6230), were used. The industrial standard for the shuttle plasmid is 1.5 µg/ml. The shuttle plasmid, packaging plasmid, and helper plasmid were mixed at a molar ratio of 2 : 4 : : 1 using the AAV-MAX transfection kit (ThermoFisher, Cat. Nos.: A50515/A50516), and virus packaging was performed according to the instructions of the kit. Viral titer was determined by referring to the second edition instructions of the AAVpro^{®} Titration kit (real-time PCR) (TaKaRa, Cat. No.: 6233), so as to evaluate the viral packaging titer of the AAV. The results are summarised in Table 4.

**Table 4. Viral packaging titers of the three vectors**

| | AAV2 - Run 1 | AAV2 - Run 2 | AAVDJ - Run 1 | AAVDJ - Run 2 |
|---|---|---|---|---|
| pUC57 Kana | 1.03E+10 | 3.54E+09 | 6.65E+09 | 4.73E+09 |
| GenS-pUC57-AAV-NoTer | 1.38E+10 | 4.09E+09 | 8.31E+09 | 4.98E+09 |
| GenS-pUC57-AAV-Ter | 1.74E+10 | 4.31E+09 | 7.42E+09 | 6.30E+09 |

As shown in Table 4, the viral packaging titers obtained with the optimized vectors were higher than that of the unmodified pUC57 Kana vector, and the titer of the GenS-pUC57-AAV-Ter vector was higher than that of the GenS-pUC57-AAV-NoTer vector. This is consistent with the above SmaI digestion results. That is, the GenS-pUC57-AAV-Ter vector exhibited a lower proportion of nonspecific bands and higher ITR integrity after SmaI digestion.

### Example 10: Structural stability test of ITRs in pSub201 series vectors

The pAAV-tTA, GenS-pSUB201-AAV-NoTer, and GenS-pSUB201-AAV-Ter vectors from Example 8 were used to clone four gene fragments (genes 1-4) identical to those in Example 8, respectively. Specifically, gene fragment 1, gene fragment 2, gene fragment 3, or gene fragment 4 was inserted between the two ITRs via homologous recombination.

As described in Example 9, the vectors were transformed into GenITRv1 competent cells, and single colonies were picked. The single colonies containing the target plasmids were used for shake-flask fermentation and plasmid extraction. The yields of the three vectors are summarised in Table 5. It could be seen that after shake-flask fermentation, the mean yields of the modified GenS-pSUB201-AAV-NoTer and GenS-pSUB201-AAV-Ter vectors were increased by 66% and 35%, respectively, compared with the unmodified pAAV-tTA vector.

**Table 5. Yields of the three vectors after shake-flask fermentation (µg)**

| Vector | pAAV-tTA | GenS-pSUB201-AAV-NoTer | GenS-pSUB201-AAV-Ter |
|---|---|---|---|
| Gene 1 | 200.0 | 426.3 | 704.3 |
| Gene 2 | 300.0 | 915.9 | 740.0 |
| Gene 3 | 420.8 | 542.0 | 435.1 |
| Gene 4 | 714.1 | 899.6 | 485.3 |
| Mean yield | 418.6 | 696.0 | 565.2 |

As described in Example 9, the three vectors were digested with SmaI and subjected to agarose gel electrophoresis. Bands other than the target band were collectively referred to as nonspecific bands. The proportion of nonspecific bands after SmaI digestion was analysed using GIS. The specific values are summarised in Table 6. It could be seen that the unmodified pAAV-tTA vector exhibited a high proportion of nonspecific bands, while the modified GenS-pSUB201-AAV-NoTer and GenS-pSUB201-AAV-Ter vectors exhibited a significant reduction in the proportion of nonspecific bands, demonstrating a significant enhancement in the structural stability of ITRs.

**Table 6. Proportions of nonspecific bands of the three vectors after SmaI digestion**

| | pAAV-tTA | GenS-pSUB201-AAV-NoTer | GenS-pSUB201-AAV-Ter |
|---|---|---|---|
| Gene 1 | 23.50% | 31.53% | 14.85% |
| Gene 2 | 29.35% | 21.32% | 15.45% |
| Gene 3 | 30.07% | 20.36% | 26.11% |
| Gene 4 | 21.50% | 14.37% | 13.25% |
| Mean value | 26.11% | 21.90% | 17.42% |

As described in Example 9, a three-plasmid packaging system was used, with the three vectors (comprising inserted gene 1) serving as shuttle plasmids. In addition, the AAV2 packaging plasmid pRC2-mi342 and the helper plasmid pHelper (Takara, Cat. No.: 6230) were used. The industrial standard for the shuttle plasmid is 1.5 µg/ml. The shuttle plasmid, packaging plasmid, and helper plasmid were mixed at a molar ratio of 2 : 4 : : 1 using the AAV-MAX transfection kit (ThermoFisher, Cat. Nos.: A50515/A50516), and virus packaging was performed according to the instructions of the kit. Viral titer was determined by referring to the second edition instructions of the AAVpro^{®} Titration kit (real-time PCR) (TaKaRa, Cat. No.: 6233), so as to evaluate the packaging titer of the AAV. The results are as shown in Table 7. Compared to pAAV-tTA, both GenS-pSUB201-AAV-NoTer and GenS-pSUB201-AAV-Ter increased the rAAV viral titer by 44%.

**Table 7. Viral packaging titers of the three vectors**

| | Titer | Replicate titer | Mean titer | Mean titer/D |
|---|---|---|---|---|
| pAAV-tTA | 4.82E+10 | 3.02E+10 | 3.92E+10 | 1 |
| GenS-pSUB201-AAV-NoTer | 7.04E+10 | 4.24E+10 | 5.64E+ 10 | 1.44 |
| GenS-pSUB201-AAV-Ter | 6.75E+10 | 4.51E+10 | 5.63E+10 | 1.44 |

The sequences involved in the present application are as follows.
SEQ ID NO: 1 
ggaagagcctagctacctactcgagtctagagggcccgtttaaacctcagatcattgtcacatgtgagcggccagga
accgtggccgcgttgctggcgt
SEQ ID NO: 2 
ggaagagcctagctacctactcgagtctagagggcccgtttaaacccgctgatcagcctcgactctagcttggatgg
aagacgccaaaaacataaagaaaggcccggcgccattctatccgctggaagatggaaccgctggagagcaactgca
taaggctatgaagagcaagctcagatcattgtcacatgtgagcaaaaggccagcaaaaggccaggaaccgtaaaaa
ggccgcgttgctggcgtt
SEQ ID NO: 3 
ggaagagcctagctacctactcgagtctagaatggaagacgccaaaaacataaagaaaggcccggcgccattctat
ccgctggaagatggaaccgctggagagcaactgcataaggctatgaagagatacgccctggttcctggaacaattgc
ttttacagatgcacatatcgaggtggacatcacttacgctgagtacttcgaaatgtccgttcggttggcagaagctatgaa
acgatatgggctgaatacaaatcacagaatcgtcgtatgcagtgaaaactctcttcaattctttatgccggtgttgggcgc
gttatttatcggagttgcagttgcgcccgcgaacgacatttataatgaacgtgaattgctcaacagtatgggcatttcgca
gcctaccgtggtgttcgtttccaaaaaggggttgcaaaaaattacatgtgagcaaaaggccagcaaaaggccaggaa
ccgtaaaaaggccgcgttgctggcgtt
SEQ ID NO: 4 
ggaagagcctagctacctactcgagtctagaatggaagacgccaaaaacataaagaaaggcccggcgccattctat
ccgctggaagatggaaccgctggagagcaactgcataaggctatgaagagatacgccctggttcctggaacaattgc
ttttacagatgcacatatcgaggtggacatcacttacgctgagtacttcgaaatgtccgttcggttggcagaagctatgaa
acgatatgggctgaatacaaatcacagaatcgtcgtatgcagtgaaaactctcttcaattctttatgccggtgttgggcgc
gttatttatcggagttgcagttgcgcccgcgaacgacatttataatgaacgtgaattgctcaacagtatgggcatttcgca
gcctaccgtggtgttcgtttccaaaaaggggttgcaaaaaattttgaacgtgcaaaaaaagctcccaatcatccaaaaaa
ttattatcatggattctaaaacggattaccagggatttcagtcgatgtacacgttcgtcacatctcatctacctcccggtttta
atgaatacgattttgtgccagagtccttcgatagggacaagacaattgcactgatcatgaactcctctggatctactggac
atgtgagcaaaaggccagcaaaaggccaggaaccgtaaaaaggccgcgttgctggcgtt
SEQ ID NO: 5 
ggaagagcctagctacctactcgagtctagagggcccgtttaaacccgctgatcagcctcgactctagcttggattct
caccaataaaaaacgcccggcggcaaccgagcgttctgaacaaatccagatggagttctgaggtcattactgg
 
atctatcaacaggagtccaagctcagatcattgtcacatgtgagcaaaaggccagcaaaaggccaggaaccgtaaa
aaggccgcgttgctggcgtt
SEQ ID NO: 6 
ggaagagcctagctacctactcgagtctagaatggaagacgccaaaaacataaagaaaggcccggcgccattctat
ccgctggaagatggaaccgctggagagcaactgcataaggctatgaagagatacgccctggttcctggaacaattgc
ttttacagatgcacatatcgaggtggacatcacttacgctgagtacttcgaaatgtccgttcggttggcagaagctatgaa
acgatatgggctgaatacaaatcacagaatcgtcgtatgcagtgaaaactctcttcaattctttatgccggtgttgggcgc
gttatttatcggagttgcagttgcgcccgcgaacgacatttataatgaacgtgaattgctcaacagtatgggcatttcgcagcctaccgtggtgttcgtttccaaaaaggggttgc
aaaaaattacatgtgagcaaaaggccagcaaaaggccaggaaccgtaaaaaggccgcgttgctggcgtt
SEQ ID NO: 7 
GCGCAACGCAATTAATGTGAGTTAGCTCACTCATTAGGCACCCCAGGCT
TTACACTTTATGCTTCCGGCTCGTATGTTGTGTGGAATTGTGAGCGGAT
AACAATTTCACA
SEQ ID NO: 8 
TTGTTATCCGCTCACAATTCCACACAACATACGAGCCGGAAGCATAAA
GTGTAAAGCCTGGGGTGCCTAATGAGTGAGCTAACTCACATTA
SEQ ID NO: 9 
Ttggccactccctctctgcgcgctcgctcgctcactgaggccgggcgaccaaaggtcgcccgacgcccgggctttg
cccgggcggcctcagtgagcgagcgagcgcgcagagagggagtggccaactccatcactaggggttcct
SEQ ID NO: 10 
AGGAACCCCTAGTGATGGAGTTGGCCACTCCCTCTCTGCGCGCTCGCTC
GCTCACTGAGGCCGGGCGACCAAAGGTCGCCCGACGCCCGGGCTTTGC
CCGGGCGGCCTCAGTGAGCGAGCGAGCGCGCAGCTGCCTGCAGG
SEQ ID NO: 11 
CCTGCAGGCAGCTGCGCGCTCGCTCGCTCACTGAGGCCGCCCGGGCGT
CGGGCGACCTTTGGTCGCCCGGCCTCAGTGAGCGAGCGAGCGCGCAGA
GAGGGAGTGGCCAACTCCATCACTAGGGGTTCCT
SEQ ID NO: 12 
GCGCGCTCGCTCGCTCACTGAGGCCGGGCGACCAAAGGTCGCCCGACG
CCCGGGCTTTGCCCGGGCGGCCTCAGTGAGCGAGCGAGCGCGCAGAGA
GGGAGTGGCCAACTCCATCACTAGGGGTTCCT
SEQ ID NO: 13 
TCTGGCGTACCGTTCCTGTCTAAAATCCCTTTAATCGGCCTCCTGTTTAG
CTCCCGCTCTGATTCTAACGAGGAAAGCACGTTATACGTGCTCGTCAAA
GCAACgATAGT
SEQ ID NO: 14 
GTCGACTAGAGCTCGCTGATCAGCCTCGACTGTGCCTTCTAGTTGCCAG
CCATCTGTTGTTTGCCCCTCCCCCGTGCCTTCCTTGACCCTGGAAGGTGC
CACTCCCACTGTCCTTTCCTAATAAAATGAGGAAATTGCATCGCATTGT
CTGAGTAGGTGTCATTCTATTCTGGGGGGTGGGGTGGGGCAGGACAGC
AAGGGGGAGGATTGGcAAGACAATAGCAGGAACCCCACTCCCacgcgtAG
GAACCCCTAGTGATGGAGTTGGCCACTCCCTCTCTGCGCGCTCGCTCGC
TCACTGAGGCCGCCCGGGCAAAGCCCGGGCGTCGGGCGACCTTTGGTC
GCCCGGCCTCAGTGAGCGAGCGAGCGCGCAGAGAGGGAGTGGCCAAG
GTACCTCTGGCCGTCGTTTTACAACGTCGTGACTGGGAAAACCCTGGCG
TTACCCAACTTAATCGCCTTGCAGCACATCCCGTCAGACCCCGTAGAAA
AGATCAAAGGATCTTCTTGAGATCCTTTTTTTCTGCGCGTAATCTGCTGC
TTGCAAACAAAAAAACCACCGCTACCAGCGGTGGTTTGTTTGCCGGAT
CAAGAGCTACCAACTCTTTTTCCGAAGGTAACTGGCTTCAGCAGAGCGC
AGATACCAAATACTGTTCTTCTAGTGTAGCCGTAGTTAGGCCACCACTT
CAAGAACTCTGTAGCACCGCCTACATACCTCGCTCTGCTAATCCTGTTA
CCAGTGGCTGCTGCCAGTGGCGATAAGTCGTGTCTTACCGGGTTGGACT
CAAGACGATAGTTACCGGATAAGGCGCAGCGGTCGGGCTGAACGGGG
GGTTCGTGCACACAGCCCAGCTTGGAGCGAACGACCTACACCGAACTG
AGATACCTACAGCGTGAGCTATGAGAAAGCGCCACGCTTCCCGAAGGG
AGAAAGGCGGACAGGTATCCGGTAAGCGGCAGGGTCGGAACAGGAGA
GCGCACGAGGGAGCTTCCAGGGGGAAACGCCTGGTATCTTTATAGTCC
TGTCGGGTTTCGCCACCTCTGACTTGAGCGTCGATTTTTGTGATGCTCGT
CAGGGGGGCGGAGCCTATGGAAAAACGCCAGCAACGCGGCCTTTTTAC
GGTTCCTGGCCTTTTGCTGGCCTTTTGCTCACATGTTCTTTCCTTTCGCC
AGCTGGCGTAATAGCGAAGAGGCCCGCACCGATCGCCCTTCCCAACAG
TTGCGCAGCaTGAATGGCGAATGGCGCCTGATGCGGTATTTTCTCCTTA
CGCATCTGTGCGGTATTTCACACCGCATATGGTGCACTCTCAGTACAAT
CTGCTCTGATGCCGCATAGTTAAGCCAGCCCCGACACCCGCCAACACCC
GCTGACGCGCCCTGACGGGCTTGTCTGCTCCCGGCATCCGCTTACAGAC
AAGCTGTGACCGacgCCGGGAGCTGCATGTGTCAGAGGTTTTCACCGTC
 
ATCACCGAAACGCGCGATGCAGCTCTGGCCCGTGTCTCAAAATCTCTGA
TGTTACATTGCACAAGATAAAAATATATCATCATGAACAATAAAACTG
TCTGCTTACATAAACAGTAATACAAGGGGTGTTATGAGCCATATTCAAC
GGGAAACGTCGAGGCCGCGATTAAATTCCAACATGGATGCTGATTTAT
ATGGGTATAAATGGGCTCGCGATAATGTCGGGCAATCAGGTGCGACAA
TCTATCGCTTGTATGGGAAGCCCGATGCGCCAGAGTTGTTTCTGAAACA
TGGCAAAGGTAGCGTTGCCAATGATGTTACAGATGAGATGGTCAGACT
AAACTGGCTGACGGAATTTATGCCTCTTCCGACCATCAAGCATTTTATC
CGTACTCCTGATGATGCATGGTTACTCACCACTGCGATCCCCGGAAAAA
CAGCATTCCAGGTATTAGAAGAATATCCTGATTCAGGTGAAAATATTGT
TGATGCGCTGGCAGTGTTCCTGCGCCGGTTGCATTCGATTCCTGTTTGT
AATTGTCCTTTTAACAGCGATCGCGTATTTCGcCTCGCTCAGGCGCAATC
ACGAATGAATAACGGTTTGGTTGATGCGAGTGATTTTGATGACGAGCG
TAATGGCTGGCCTGTTGAACAAGTCTGGAAAGAAATGCATAAACTTTT
GCCATTCTCACCGGATTCAGTCGTCACTCATGGTGATTTCTCACTTGAT
AACCTTATTTTTGACGAGGGGAAATTAATAGGTTGTATTGATGTTGGAC
GAGTCGGAATCGCAGACCGATACCAGGATCTTGCCATCCTATGGAACT
GCCTCGGTGAGTTTTCTCCTTCATTACAGAAACGGCTTTTTCAAAAATA
TGGTATTGATAATCCTGATATGAATAAATTGCAGTTTCATTTGATGCTC
GATGAGTTTTTCTAActgtcagaccaagtttactcatatatactttagattgatttaaaacttcatttttaatttaa
aaggatctaggtgaagatcctttttgataatctcatgaccaaaatcccttaacgtgagttttcgttccactgagcgtcagac
CCTGCGTTATCCCCTGATTCTGTGGATAACCGTATTACCGCCTTTGAGT
GAGCTGATACCGCTCGCCGCAGCCGAACGACCGAGCGCAGCGAGTCAG
TGAGCGAGGAAGCGGAAGAaCGCCCAATACGCAAACCGCCTCTCCCCG
CGCGTTGGCCGATTCATTAATGCAGCTGGCACGACAGGTTTCCCGACTG
GAAAGCGGGCAGTGATCTGGCGTACCGTTCCTGTCTAAAATCCCTTTAA
TCGGCCTCCTGTTTAGCTCCCGCTCTGATTCTAACGAGGAAAGCACGTT
ATACGTGCTCGTCAAAGCAACgATAGTCAGGAAACAGCTATGACCATG
ATTACACCCAATTGTTGGCCACTCCCTCTCTGCGCGCTCGCTCGCTCACT
GAGGCCGGGCGACCAAAGGTCGCCCGACGCCCGGGCTTTGCCCGGGCG
GCCTCAGTGAGCGAGCGAGCGCGCAGAGAGGGAGTGGCCAACTCCATC
ACTAGGGGTTCCTCCTAGGTTAATGATTAACCCGCCATGCTACTTATCT
ACGTAGCCATGCTCTAGGATCTGAATTCTAGTTATTAATAGTAATCAAT
TACGGGGTCATTAGTTCATAGCCCATATATGGAGTTCCGCGTTACATAA
 
CTTACGGTAAATGGCCCGCCTGGCTGACCGCCCAACGACCCCCGCCCAT
TGACGTCAATAATGACGTATGTTCCCATAGTAACGCCAATAGGGACTTT
CCATTGACGTCAATGGGTGGAGTATTTACGGTAAACTGCCCACTTGGCA
GTACATCAAGTGTATCATATGCCAAGTACGCCCCCTATTGACGTCAATG
ACGGTAAATGGCCCGCCTGGCATTATGCCCAGTACATGACCTTATGGG
ACTTTCCTACTTGGCAGTACATCTACGTATTAGTCATCGCTATTACCATG
GTCGAGGTGAGCCCCACGTTCTGCTTCACTCTCCCCATCTCCCCCCCCTC
CCCACCCCCAATTTTGTATTTATTTATTTTTTAATTATTTTGTGCAGCGA
TGGGGGCGGGGGGGGGGGGGGCGCGCCAGGCGGGGCGGGGCGG
GGCGAGGGGCGGGGCGGGGCGAGGCGGAGAGGTGCGGCGGCAGCCAA
TCAGAGCGGCGCGCTCCGAAAGTTTCCTTTTATGGCGAGGCGGCGG
GCGGCGGCCCTATAAAAAGCGAAGCGCGCGGCGGGCGGGAGTCGCTG
CGaaatgctagcgtttaaacttaagcttACCGGTgagctcggatccACTAGTccagtgtggtgGCATG
CtgcagatatccagcacagtggcggccgcATGGTGAGCAAGGGCGAGGAGCTGTTCAC
CGGGGTGGTGCCCATCCTGGTCGAGCTGGACGGCGACGTAAACGGCCA
CAAGTTCAGCGTGTCCGGCGAGGGCGAGGGCGATGCCACCTACGGCAA
GCTGACCCTGAAGTTCATCTGCACCACCGGCAAGCTGCCCGTGCCCTGG
CCCACCCTCGTGACCACCCTGACCTACGGCGTGCAGTGCTTCAGCCGCT
ACCCCGACCACATGAAGCAGCACGACTTCTTCAAGTCCGCCATGCCCG
AAGGCTACGTCCAGGAGCGCACCATCTTCTTCAAGGACGACGGCAACT
ACAAGACCCGCGCCGAGGTGAAGTTCGAGGGCGACACCCTGGTGAACC
GCATCGAGCTGAAGGGCATCGACTTCAAGGAGGACGGCAACATCCTGG
GGCACAAGCTGGAGTACAACTACAACAGCCACAACGTCTATATCATGG
CCGACAAGCAGAAGAACGGCATCAAGGTGAACTTCAAGATCCGCCACA
ACATCGAGGACGGCAGCGTGCAGCTCGCCGACCACTACCAGCAGAACA
CCCCCATCGGCGACGGCCCCGTGCTGCTGCCCGACAACCACTACCTGA
GCACCCAGTCCGCCCTGAGCAAAGACCCCAACGAGAAGCGCGATCACA
TGGTCCTGCTGGAGTTCGTGACCGCCGCCGGGATCACTCTCGGCATGGA
CGAGCTGTACAAGTAACtcgagtctagagggcccgtttaaac
SEQ ID NO: 15 
GTCGACTAGAGCTCGCTGATCAGCCTCGACTGTGCCTTCTAGTTGCCAG
CCATCTGTTGTTTGCCCCTCCCCCGTGCCTTCCTTGACCCTGGAAGGTGC
CACTCCCACTGTCCTTTCCTAATAAAATGAGGAAATTGCATCGCATTGT
CTGAGTAGGTGTCATTCTATTCTGGGGGGTGGGGTGGGGCAGGACAGC
 
AAGGGGGAGGATTGGcAAGACAATAGCAGGAACCCCACTCCCacgcgtAG
GAACCCCTAGTGATGGAGTTGGCCACTCCCTCTCTGCGCGCTCGCTCGC
TCACTGAGGCCGCCCGGGCAAAGCCCGGGCGTCGGGCGACCTTTGGTC
GCCCGGCCTCAGTGAGCGAGCGAGCGCGCAGAGAGGGAGTGGCCAAG
GTACCTCTGGCCGTCGTTTTACAACGTCGTGACctCTAGCTTGGATTCTC
ACCAATAAAAAACGCCCGGCGGCAACCGAGCGTTCTGAACAAATCCAG
ATGGAGTTCTGAGGTCATTACTGGATCTATCAACAGGAGTCCAAGCTCA
GtctactgggcggttttTGCTTGCAGCACATCCCGTCAGACCCCGTAGAAAAGAT
CAAAGGATCTTCTTGAGATCCTTTTTTTCTGCGCGTAATCTGCTGCTTGC
AAACAAAAAAACCACCGCTACCAGCGGTGGTTTGTTTGCCGGATCAAG
AGCTACCAACTCTTTTTCCGAAGGTAACTGGCTTCAGCAGAGCGCAGAT
ACCAAATACTGTTCTTCTAGTGTAGCCGTAGTTAGGCCACCACTTCAAG
AACTCTGTAGCACCGCCTACATACCTCGCTCTGCTAATCCTGTTACCAG
TGGCTGCTGCCAGTGGCGATAAGTCGTGTCTTACCGGGTTGGACTCAAG
ACGATAGTTACCGGATAAGGCGCAGCGGTCGGGCTGAACGGGGGGTTC
GTGCACACAGCCCAGCTTGGAGCGAACGACCTACACCGAACTGAGATA
CCTACAGCGTGAGCTATGAGAAAGCGCCACGCTTCCCGAAGGGAGAAA
GGCGGACAGGTATCCGGTAAGCGGCAGGGTCGGAACAGGAGAGCGCA
CGAGGGAGCTTCCAGGGGGAAACGCCTGGTATCTTTATAGTCCTGTCG
GGTTTCGCCACCTCTGACTTGAGCGTCGATTTTTGTGATGCTCGTCAGG
GGGGCGGAGCCTATGGAAAAACGCCAGCAACGCGGCCTTTTTACGGTT
CCTGGCCTTTTGCTGGCCTTTTGCTCACATGTTCTTTCCTTTCGCCAGCT
GGCGTAATAGCGAAGAGGCCCGCACCGATCGCCCTTCCCAACAGTTGC
GCAGCaTGAATGGCGAATGGCGCCTGATGCGGTATTTTCTCCTTACGCA
TCTGTGCGGTATTTCACACCGCATATGGTGCACTCTCAGTACAATCTGC
TCTGATGCCGCATAGTTAAGCCAGCCCCGACACCCGCCAACACCCGCT
GACGCGCCCTGACGGGCTTGTCTGCTCCCGGCATCCGCTTACAGACAAG
CTGTGACCGacgCCGGGAGCTGCATGTGTCAGAGGTTTTCACCGTCATCA
CCGAAACGCGCGATGCAGCTCTGGCCCGTGTCTCAAAATCTCTGATGTT
ACATTGCACAAGATAAAAATATATCATCATGAACAATAAAACTGTCTG
CTTACATAAACAGTAATACAAGGGGTGTTATGAGCCATATTCAACGGG
AAACGTCGAGGCCGCGATTAAATTCCAACATGGATGCTGATTTATATG
GGTATAAATGGGCTCGCGATAATGTCGGGCAATCAGGTGCGACAATCT
ATCGCTTGTATGGGAAGCCCGATGCGCCAGAGTTGTTTCTGAAACATGG
 
CAAAGGTAGCGTTGCCAATGATGTTACAGATGAGATGGTCAGACTAAA
CTGGCTGACGGAATTTATGCCTCTTCCGACCATCAAGCATTTTATCCGT
ACTCCTGATGATGCATGGTTACTCACCACTGCGATCCCCGGAAAAACA
GCATTCCAGGTATTAGAAGAATATCCTGATTCAGGTGAAAATATTGTTG
ATGCGCTGGCAGTGTTCCTGCGCCGGTTGCATTCGATTCCTGTTTGTAA
TTGTCCTTTTAACAGCGATCGCGTATTTCGcCTCGCTCAGGCGCAATCAC
GAATGAATAACGGTTTGGTTGATGCGAGTGATTTTGATGACGAGCGTA
ATGGCTGGCCTGTTGAACAAGTCTGGAAAGAAATGCATAAACTTTTGC
CATTCTCACCGGATTCAGTCGTCACTCATGGTGATTTCTCACTTGATAA
CCTTATTTTTGACGAGGGGAAATTAATAGGTTGTATTGATGTTGGACGA
GTCGGAATCGCAGACCGATACCAGGATCTTGCCATCCTATGGAACTGC
CTCGGTGAGTTTTCTCCTTCATTACAGAAACGGCTTTTTCAAAAATATG
GTATTGATAATCCTGATATGAATAAATTGCAGTTTCATTTGATGCTCGA
TGAGTTTTTCTAATCAGAATTGGTTAATTGGTTGTAACATTATTCAGATT
GGGCTTGATTTAAAACTTCATTTTTAATTTAGATAACCGTATTACCGCCT
TTGAGTGAGCTGATACCGCTCGCCGCAGCCGAACGACCgttctttcaaataaaacg
aaaggctcagtcgaaagactgggcctttcgttttatctgttgtttgtcggtgaacgctctcctgagtaggacaaatccgcc
gggagcggatttgaacgttgcgaagcaacggcccggagggtggcgggcaggacgcccgccataaactgccaggc
atcaaattaagcagaaggccatcctgacggatggcctttttgcgtttctacaaactctgactcttCAGGAAACAG
CTATGACCATGATTACACCCAATTGTTGGCCACTCCCTCTCTGCGCGCT
CGCTCGCTCACTGAGGCCGGGCGACCAAAGGTCGCCCGACGCCCGGGC
TTTGCCCGGGCGGCCTCAGTGAGCGAGCGAGCGCGCAGAGAGGGAGTG
GCCAACTCCATCACTAGGGGTTCCTCCTAGGTTAATGATTAACCCGCCA
TGCTACTTATCTACGTAGCCATGCTCTAGGATCTGAATTCTAGTTATTA
ATAGTAATCAATTACGGGGTCATTAGTTCATAGCCCATATATGGAGTTC
CGCGTTACATAACTTACGGTAAATGGCCCGCCTGGCTGACCGCCCAAC
GACCCCCGCCCATTGACGTCAATAATGACGTATGTTCCCATAGTAACGC
CAATAGGGACTTTCCATTGACGTCAATGGGTGGAGTATTTACGGTAAAC
TGCCCACTTGGCAGTACATCAAGTGTATCATATGCCAAGTACGCCCCCT
ATTGACGTCAATGACGGTAAATGGCCCGCCTGGCATTATGCCCAGTAC
ATGACCTTATGGGACTTTCCTACTTGGCAGTACATCTACGTATTAGTCA
TCGCTATTACCATGGTCGAGGTGAGCCCCACGTTCTGCTTCACTCTCCC
CATCTCCCCCCCCTCCCCACCCCCAATTTTGTATTTATTTATTTTTTAATT
ATTTTGTGCAGCGATGGGGGCGGGGGGGGGGGGGGGGCGCGCGCCAG
 
GCGGGGCGGGGCGGGGCGAGGGGCGGGGCGGGGCGAGGCGGAGAGGT
GCGGCGGCAGCCAATCAGAGCGGCGCGCTCCGAAAGTTTCCTTTTATG
GCGAGGCGGCGGCGGCGGCGGCCCTATAAAAAGCGAAGCGCGCGGCG
GGCGGGAGTCGCTGCGaaatgctagcgtttaaacttaagcttACCGGTgagctcggatccACT
AGTccagtgtggtgGCATGCtgcagatatccagcacagtggcggccgcATGGTGAGCAAGGG
CGAGGAGCTGTTCACCGGGGTGGTGCCCATCCTGGTCGAGCTGGACGG
CGACGTAAACGGCCACAAGTTCAGCGTGTCCGGCGAGGGCGAGGGCGA
TGCCACCTACGGCAAGCTGACCCTGAAGTTCATCTGCACCACCGGCAA
GCTGCCCGTGCCCTGGCCCACCCTCGTGACCACCCTGACCTACGGCGTG
CAGTGCTTCAGCCGCTACCCCGACCACATGAAGCAGCACGACTTCTTCA
AGTCCGCCATGCCCGAAGGCTACGTCCAGGAGCGCACCATCTTCTTCAA
GGACGACGGCAACTACAAGACCCGCGCCGAGGTGAAGTTCGAGGGCG
ACACCCTGGTGAACCGCATCGAGCTGAAGGGCATCGACTTCAAGGAGG
ACGGCAACATCCTGGGGCACAAGCTGGAGTACAACTACAACAGCCACA
ACGTCTATATCATGGCCGACAAGCAGAAGAACGGCATCAAGGTGAACT
TCAAGATCCGCCACAACATCGAGGACGGCAGCGTGCAGCTCGCCGACC
ACTACCAGCAGAACACCCCCATCGGCGACGGCCCCGTGCTGCTGCCCG
ACAACCACTACCTGAGCACCCAGTCCGCCCTGAGCAAAGACCCCAACG
AGAAGCGCGATCACATGGTCCTGCTGGAGTTCGTGACCGCCGCCGGGA
TCACTCTCGGCATGGACGAGCTGTACAAGTAACtcgagtctagagggcccgtttaaac
SEQ ID NO: 16 
AAATCCCTTAACGTGAGTTTTCGTTCCACTGAGCCCTGCGTTATCCCCT
GATTCTGTGGATAACCGTATTACCGCCTTTGAGTGAGCTGATACCGCTC
GCCGCAGCCGAACGACCGAGCGCAGCGAGTCAGTGAGCGAGGAAGCG
GAAGAGCGCCCAATACGCAAACCGCCTCTCCCCGCGCGTTGGCCGATT
CATTAATGCAGCTGGCACGACAGGTTTCCCGACTGGAAAGCGGGCAGT
GATCTGGCGTACCGTTCCTGTCTAAAATCCCTTTAATCGGCCTCCTGTTT
AGCTCCCGCTCTGATTCTAACGAGGAAAGCACGTTATACGTGCTCGTCA
AAGCGTTGATATTCAGTCAATTACAAACATTAATAACGAAGAGATGAC
AGAAAAATTTTCATTCTGTGACAGAGAAAAAGTAGCCGAAGATGACGG
TTTGTCACATGGAGTTGGCA
SEQ ID NO: 17 
GTCGACTAGAGCTCGCTGATCAGCCTCGACTGTGCCTTCTAGTTGCCAG
CCATCTGTTGTTTGCCCCTCCCCCGTGCCTTCCTTGACCCTGGAAGGTGC
 
CACTCCCACTGTCCTTTCCTAATAAAATGAGGAAATTGCATCGCATTGT
CTGAGTAGGTGTCATTCTATTCTGGGGGGTGGGGTGGGGCAGGACAGC
AAGGGGGAGGATTGGcAAGACAATAGCAGGAACCCCACTCCCGGTACC
AGGAACCCCTAGTGATGGAGTTGGCCACTCCCTCTCTGCGCGCTCGCTC
GCTCACTGAGGCCGGGCGACCAAAGGTCGCCCGACGCCCGGGCTTTGC
CCGGGCGGCCTCAGTGAGCGAGCGAGCGCGCAGCTGCCTGCAGGACTA
GTTGATGCGGTATTTTCTCCTTACGCATCTGTGCGGTATTTCACACCGCA
TACGTCACATGTGTCAGAGGTTTTCACCGTCATCACCGAACACTGAGCG
TCAGACCCCGTAGAAAAGATCAAAGGATCTTCTTGAGATCCTTTTTTTC
TGCGCGTAATCTGCTGCTTGCAAACAAAAAAACCACCGCTACCAGCGG
TGGTTTGTTTGCCGGATCAAGAGCTACCAACTCTTTTTCCGAAGGTAAC
TGGCTTCAGCAGAGCGCAGATACCAAATACTGTCCTTCTAGTGTAGCCG
TAGTTAGGCCACCACTTCAAGAACTCTGTAGCACCGCCTACATACCTCG
CTCTGCTAATCCTGTTACCAGTGGCTGCTGCCAGTGGCGATAAGTCGTG
TCTTACCGGGTTGGACTCAAGACGATAGTTACCGGATAAGGCGCAGCG
GTCGGGCTGAACGGGGGGTTCGTGCACACAGCCCAGCTTGGAGCGAAC
GACCTACACCGAACTGAGATACCTACAGCGTGAGCTATGAGAAAGCGC
CACGCTTCCCGAAGGGAGAAAGGCGGACAGGTATCCGGTAAGCGGCA
GGGTCGGAACAGGAGAGCGCACGAGGGAGCTTCCAGGGGGAAACGCC
TGGTATCTTTATAGTCCTGTCGGGTTTCGCCACCTCTGACTTGAGCGTCG
ATTTTTGTGATGCTCGTCAGGGGGGCGGAGCCTATGGAAAAACGCCAG
CAACGCGGCCTTTTTACGGTTCCTGGCCTTTTGCTGGCCTTTTGCTCACA
TGTACGCGCGAGtCGAAAGGGCCTCGTGATACGCCTATTTTTATAGGTT
AATGTgATGATAATAATGGTTTCTTAGACGTCAGGTGGCACTTTTCGGG
GAAATGTGCGCGGAACCCCTATTTGTTTATTTTTCTAAATACATTCAAA
TATGTATCCGCTCATGAGACAATAACCCTGATAAATGCTTCAATAATAT
TGAAAAAGGAAGAGTATGAGTATTCAACATTTCCGTGTCGCCCTTATTC
CCTTTTTTGCGGCATTTTGCCTTCCTGTTTTTGCTCACCCAGAAACGCTG
GTGAAAGTAAAAGATGCTGAAGATCAGTTGGGTGCACGAGTGGGTTAC
ATCGAACTGGATCTCAACAGCGGTAAGATCCTTGAGAGTTTTCGCCCCG
AAGAACGTTTTCCAATGATGAGCACTTTTAAAGTTCTGCTATGTGGCGC
GGTATTATCCCGTATTGACGCCGGGCAAGAGCAACTCGGTCGCCGCAT
ACACTATTCTCAGAATGACTTGGTTGAGTACTCACCAGTCACAGAAAA
GCATCTTACGGATGGCATGACAGTAAGAGAATTATGCAGTGCTGCCAT
 
AACCATGAGTGATAACACTGCGGCCAACTTACTTCTGACAACGATCGG
AGGACCGAAGGAGCTAACCGCTTTTTTGCACAACATGGGGGATCATGT
AACTCGCCTTGATCGTTGGGAACCGGAGCTGAATGAAGCCATACCAAA
CGACGAGCGTGACACCACGATGCCTGTAGCAATGGCAACAACGTTGCG
CAAACTATTAACTGGCGAACTACTTACTCTAGCTTCCCGGCAACAATTA
ATAGACTGGATGGAGGCGGATAAAGTTGCAGGACCACTTCTGCGCTCG
GCgCTTCCGGCTGGCTGGTTTATTGCTGATAAATCTGGAGCCGGTGAGC
GTGGaTCTCGCGGTATCATTGCAGCACTGGGGCCAGATGGTAAGCCCTC
CCGTATCGTAGTTATCTACACcACGGGGAGTCAGGCAACTATGGATGAA
CGAAATAGACAGATCGCTGAGATAGGTGCCTCACTGATTAAGCATTGG
TAACTGTCAGACCAAGTTTACTCATATATACTTTAGATTGATTTAAAAC
TTCATTTTTAATTTAAAAGGATCTAGGTGAAGATCCTTTTTGATAATCTC
ATGACCAAAATCCCTTAACGTGAGTTTTCGTTCCACTGAGCGTcAGTTT
ATCCCTTAACCCTGCGTTATCCCCTGATTCTGTGGATAACCGTATTACC
GCCTTTGAGTGAGCTGATACCGCTCGCCGCAGCCGAACGACCGAGCGC
AGCGAGTCAGTGAGCGAGGAAGCGGAAGAaCGCCCAATACGCAAACCG
CCTCTCCCCGCGCGTTGGCCGATTCATTAATGCAGCTGGCACGACAGGT
TTCCCGACTGGAAAGCGGGCAGTGATCTGGCGTACCGTTCCTGTCTAAA
ATCCCTTTAATCGGCCTCCTGTTTAGCTCCCGCTCTGATTCTAACGAGG
AAAGCACGTTATACGTGCTCGTCAAAGCGTTGATATTCAGTCAATTACA
AACATTAATAACGAAGAGATGACAGAAAAATTTTCATTCTGTGACAGA
GAAAAAGTAGCCGAAGATGtCGGTTTGTCACATGGAGTTGGCATATGGT
GCACTCTCAGTACAATCTGCTCTGATGCCGCATAGTTAAGCCAGCCCCG
ACACCCGCCAACACCCGCTGACGCGCCCTGACGGGCTTGTCTGAAGCA
ACCATAGTCTCCCGGCATCCGCTTACAGACAAGCTGTGACCGTCTaCGG
GAGCTGAACAATTGCCTGCAGGCAGCTGCGCGCTCGCTCGCTCACTGA
GGCCGCCCGGGCGTCGGGCGACCTTTGGTCGCCCGGCCTCAGTGAGCG
AGCGAGCGCGCAGAGAGGGAGTGGCCAACTCCATCACTAGGGGTTCCT
acgcgtTTAATGATTAACCCGCCATGCTACTTATCTACGTAGCCATGCTCT
AGGATCTGAATTCTAGTTATTAATAGTAATCAATTACGGGGTCATTAGT
TCATAGCCCATATATGGAGTTCCGCGTTACATAACTTACGGTAAATGGC
CCGCCTGGCTGACCGCCCAACGACCCCCGCCCATTGACGTCAATAATG
ACGTATGTTCCCATAGTAACGCCAATAGGGACTTTCCATTGACGTCAAT
GGGTGGAGTATTTACGGTAAACTGCCCACTTGGCAGTACATCAAGTGT
 
ATCATATGCCAAGTACGCCCCCTATTGACGTCAATGACGGTAAATGGCC
CGCCTGGCATTATGCCCAGTACATGACCTTATGGGACTTTCCTACTTGG
CAGTACATCTACGTATTAGTCATCGCTATTACCATGGTCGAGGTGAGCC
CCACGTTCTGCTTCACTCTCCCCATCTCCCCCCCCTCCCCACCCCCAATT
TTGTATTTATTTATTTTTTAATTATTTTGTGCAGCGATGGGGGCGGGGGGG
GGGGGGGGGCGCGCGCCAGGCGGGGCGGGGCGGGGCGAGGGGCGGG
GCGGGGCGAGGCGGAGAGGTGCGGCGGCAGCCAATCAGAGCGGCGCG
CTCCGAAAGTTTCCTTTTATGGCGAGGCGGCGGCGGCGGCGGCCCTATA
AAAAGCGAAGCGCGCGGCGGGCGGGAGTCGCTGCGaaatgctagcgtttaaacttaa
gcttACCGGTgagctcggatccatgcatccagtgtggtgGCATGCtgcagatatccagcacagtggcggcc
gcATGGTGAGCAAGGGCGAGGAGCTGTTCACCGGGGTGGTGCCCATCCT
GGTCGAGCTGGACGGCGACGTAAACGGCCACAAGTTCAGCGTGTCCGG
CGAGGGCGAGGGCGATGCCACCTACGGCAAGCTGACCCTGAAGTTCAT
CTGCACCACCGGCAAGCTGCCCGTGCCCTGGCCCACCCTCGTGACCACC
CTGACCTACGGCGTGCAGTGCTTCAGCCGCTACCCCGACCACATGAAG
CAGCACGACTTCTTCAAGTCCGCCATGCCCGAAGGCTACGTCCAGGAG
CGCACCATCTTCTTCAAGGACGACGGCAACTACAAGACCCGCGCCGAG
GTGAAGTTCGAGGGCGACACCCTGGTGAACCGCATCGAGCTGAAGGGC
ATCGACTTCAAGGAGGACGGCAACATCCTGGGGCACAAGCTGGAGTAC
AACTACAACAGCCACAACGTCTATATCATGGCCGACAAGCAGAAGAAC
GGCATCAAGGTGAACTTCAAGATCCGCCACAACATCGAGGACGGCAGC
GTGCAGCTCGCCGACCACTACCAGCAGAACACCCCCATCGGCGACGGC
CCCGTGCTGCTGCCCGACAACCACTACCTGAGCACCCAGTCCGCCCTGA
GCAAAGACCCCAACGAGAAGCGCGATCACATGGTCCTGCTGGAGTTCG
TGACCGCCGCCGGGATCACTCTCGGCATGGACGAGCTGTACAAGTAACt
cgagtctagagggcccgtttaaac
SEQ ID NO: 18 
GTCGACTAGAGCTCGCTGATCAGCCTCGACTGTGCCTTCTAGTTGCCAG
CCATCTGTTGTTTGCCCCTCCCCCGTGCCTTCCTTGACCCTGGAAGGTGC
CACTCCCACTGTCCTTTCCTAATAAAATGAGGAAATTGCATCGCATTGT
CTGAGTAGGTGTCATTCTATTCTGGGGGGTGGGGTGGGGCAGGACAGC
AAGGGGGAGGATTGGcAAGACAATAGCAGGAACCCCACTCCCGGTACC
AGGAACCCCTAGTGATGGAGTTGGCCACTCCCTCTCTGCGCGCTCGCTC
GCTCACTGAGGCCGGGCGACCAAAGGTCGCCCGACGCCCGGGCTTTGC
 
CCGGGCGGCCTCAGTGAGCGAGCGAGCGCGCAGCTGCCTGCAGGACTA
GTTGATGCGGTATTTTCTCCTTACGCATCTGTGCGGTgttctttcaaataaaacgaaa
ggctcagtcgaaagactgggcctttcgttttatctgttgtttgtcggtgaacgctctcctgagtaggacaaatccgccggg
agcggatttgaacgttgcgaagcaacggcccggagggtggcgggcaggacgcccgccataaactgccaggcatca
aattaagcagaaggccatcctgacggatggcctttttgcgtttctacaaactctgactcttGCCAGCCCCGAC
ACCCGCCAACACCCGCTGACGCGCCCTGACGGGCTTGTCTGCTCCCGGC
ATCCGCTTACAGACAAGCTGTGACCaTCTCCGGGAGCTGCATGTGTCAG
AGGTTTTCACCGTCATCACCGAACACTGAGCGTCAGACCCCGTAGAAA
AGATCAAAGGATCTTCTTGAGATCCTTTTTTTCTGCGCGTAATCTGCTGC
TTGCAAACAAAAAAACCACCGCTACCAGCGGTGGTTTGTTTGCCGGAT
CAAGAGCTACCAACTCTTTTTCCGAAGGTAACTGGCTTCAGCAGAGCGC
AGATACCAAATACTGTCCTTCTAGTGTAGCCGTAGTTAGGCCACCACTT
CAAGAACTCTGTAGCACCGCCTACATACCTCGCTCTGCTAATCCTGTTA
CCAGTGGCTGCTGCCAGTGGCGATAAGTCGTGTCTTACCGGGTTGGACT
CAAGACGATAGTTACCGGATAAGGCGCAGCGGTCGGGCTGAACGGGG
GGTTCGTGCACACAGCCCAGCTTGGAGCGAACGACCTACACCGAACTG
AGATACCTACAGCGTGAGCTATGAGAAAGCGCCACGCTTCCCGAAGGG
AGAAAGGCGGACAGGTATCCGGTAAGCGGCAGGGTCGGAACAGGAGA
GCGCACGAGGGAGCTTCCAGGGGGAAACGCCTGGTATCTTTATAGTCC
TGTCGGGTTTCGCCACCTCTGACTTGAGCGTCGATTTTTGTGATGCTCGT
CAGGGGGGCGGAGCCTATGGAAAAACGCCAGCAACGCGGCCTTTTTAC
GGTTCCTGGCCTTTTGCTGGCCTTTTGCTCACATGTACGCGCGAGtCGAA
AGGGCCTCGTGATACGCCTATTTTTATAGGTTAATGTgATGATAATAAT
GGTTTCTTAGACGTCAGGTGGCACTTTTCGGGGAAATGTGCGCGGAACC
CCTATTTGTTTATTTTTCTAAATACATTCAAATATGTATCCGCTCATGAG
ACAATAACCCTGATAAATGCTTCAATAATATTGAAAAAGGAAGAGTAT
GAGTATTCAACATTTCCGTGTCGCCCTTATTCCCTTTTTTGCGGCATTTT
GCCTTCCTGTTTTTGCTCACCCAGAAACGCTGGTGAAAGTAAAAGATGC
TGAAGATCAGTTGGGTGCACGAGTGGGTTACATCGAACTGGATCTCAA
CAGCGGTAAGATCCTTGAGAGTTTTCGCCCCGAAGAACGTTTTCCAATG
ATGAGCACTTTTAAAGTTCTGCTATGTGGCGCGGTATTATCCCGTATTG
ACGCCGGGCAAGAGCAACTCGGTCGCCGCATACACTATTCTCAGAATG
ACTTGGTTGAGTACTCACCAGTCACAGAAAAGCATCTTACGGATGGCA
TGACAGTAAGAGAATTATGCAGTGCTGCCATAACCATGAGTGATAACA
 
CTGCGGCCAACTTACTTCTGACAACGATCGGAGGACCGAAGGAGCTAA
CCGCTTTTTTGCACAACATGGGGGATCATGTAACTCGCCTTGATCGTTG
GGAACCGGAGCTGAATGAAGCCATACCAAACGACGAGCGTGACACCA
CGATGCCTGTAGCAATGGCAACAACGTTGCGCAAACTATTAACTGGCG
AACTACTTACTCTAGCTTCCCGGCAACAATTAATAGACTGGATGGAGGC
GGATAAAGTTGCAGGACCACTTCTGCGCTCGGCgCTTCCGGCTGGCTGG
TTTATTGCTGATAAATCTGGAGCCGGTGAGCGTGGaTCTCGCGGTATCA
TTGCAGCACTGGGGCCAGATGGTAAGCCCTCCCGTATCGTAGTTATCTA
CACcACGGGGAGTCAGGCAACTATGGATGAACGAAATAGACAGATCGC
TGAGATAGGTGCCTCACTGATTAAGCATTGGTAACTGTCAGACCAAGTT
TACTCATATATACTTTAGATTGATTTAATGGCACGACAGGTTTCCCGAC
TGGAAAGCGGGCAGTGATCTGGCGTACCGTTCCTGTCTAAAATCCCTTT
AATCGGCCTCCTGTTTAGCTCCCGCTCTGATTCTAACGAGGAAAGCACG
TTATACGTGCTCGTCAActCTAGCTTGGATTCTCACCAATAAAAAACGCC
CGGCGGCAACCGAGCGTTCTGAACAAATCCAGATGGAGTTCTGAGGTC
ATTACTGGATCTATCAACAGGAGTCCAAGCTCAGtctactgggcggttttTACAA
TCTGCTCTGATGCCGCATAGTTAAAACTTCATTTTTAATTTAAAAGGAT
CTAGGTGAAGATCCTTTTTGATAATCTCATGACCAAAATCCCTTAACGT
GAGTTTCAATTGCCTGCAGGCAGCTGCGCGCTCGCTCGCTCACTGAGGC
CGCCCGGGCGTCGGGCGACCTTTGGTCGCCCGGCCTCAGTGAGCGAGC
GAGCGCGCAGAGAGGGAGTGGCCAACTCCATCACTAGGGGTTCCTacgcg
tTTAATGATTAACCCGCCATGCTACTTATCTACGTAGCCATGCTCTAGGA
TCTGAATTCTAGTTATTAATAGTAATCAATTACGGGGTCATTAGTTCAT
AGCCCATATATGGAGTTCCGCGTTACATAACTTACGGTAAATGGCCCGC
CTGGCTGACCGCCCAACGACCCCCGCCCATTGACGTCAATAATGACGT
ATGTTCCCATAGTAACGCCAATAGGGACTTTCCATTGACGTCAATGGGT
GGAGTATTTACGGTAAACTGCCCACTTGGCAGTACATCAAGTGTATCAT
ATGCCAAGTACGCCCCCTATTGACGTCAATGACGGTAAATGGCCCGCCT
GGCATTATGCCCAGTACATGACCTTATGGGACTTTCCTACTTGGCAGTA
CATCTACGTATTAGTCATCGCTATTACCATGGTCGAGGTGAGCCCCACG
TTCTGCTTCACTCTCCCCATCTCCCCCCCCTCCCCACCCCCAATTTTGTA
TTTATTTATTTTTTAATTATTTTGTGCAGCGATGGGGGCGGGGGGGGGG
GGGGGGCGCGCGCCAGGCGGGGCGGGGCGGGGCGAGGGGCGGGGCGG
GGCGAGGCGGAGAGGTGCGGCGGCAGCCAATCAGAGCGGCGCGCTCC
 
GAAAGTTTCCTTTTATGGCGAGGCGGCGGCGGCGGCGGCCCTATAAAA
AGCGAAGCGCGCGGCGGGCGGGAGTCGCTGCGaaatgctagcgtttaaacttaagcttA
CCGGTgagctcggatccatgcatccagtgtggtgGCATGCtgcagatatccagcacagtggcggccgcAT
GGTGAGCAAGGGCGAGGAGCTGTTCACCGGGGTGGTGCCCATCCTGGT
CGAGCTGGACGGCGACGTAAACGGCCACAAGTTCAGCGTGTCCGGCGA
GGGCGAGGGCGATGCCACCTACGGCAAGCTGACCCTGAAGTTCATCTG
CACCACCGGCAAGCTGCCCGTGCCCTGGCCCACCCTCGTGACCACCCTG
ACCTACGGCGTGCAGTGCTTCAGCCGCTACCCCGACCACATGAAGCAG
CACGACTTCTTCAAGTCCGCCATGCCCGAAGGCTACGTCCAGGAGCGC
ACCATCTTCTTCAAGGACGACGGCAACTACAAGACCCGCGCCGAGGTG
AAGTTCGAGGGCGACACCCTGGTGAACCGCATCGAGCTGAAGGGCATC
GACTTCAAGGAGGACGGCAACATCCTGGGGCACAAGCTGGAGTACAAC
TACAACAGCCACAACGTCTATATCATGGCCGACAAGCAGAAGAACGGC
ATCAAGGTGAACTTCAAGATCCGCCACAACATCGAGGACGGCAGCGTG
CAGCTCGCCGACCACTACCAGCAGAACACCCCCATCGGCGACGGCCCC
GTGCTGCTGCCCGACAACCACTACCTGAGCACCCAGTCCGCCCTGAGC
AAAGACCCCAACGAGAAGCGCGATCACATGGTCCTGCTGGAGTTCGTG
ACCGCCGCCGGGATCACTCTCGGCATGGACGAGCTGTACAAGTAACtcga
gtctagagggcccgtttaaac
SEQ ID NO: 19 
GTTCgcaggtgcggccgcacgcgtctagttattaatagtaatcaattacggggtcattagttcatagcccatatatgg
agttccgcgttacataacttacggtaaatggcccgcctggctgaccgcccaacgacccccgcccattgacgtcaataat
gacgtatgttcccatagtaacgtcaatagggactttccattgacgtcaatgggtggagtatttacggtaaactgcccactt
ggcagtacatcaagtgtatcatatgccaagtacgccccctattgacgtcaatgacggtaaatggcccgcctggcattat
gcccagtacatgaccttatgggactttcctacttggcagtacatctacgtattagtcatcgctattaccatggtgatgcggtt
ttggcagtacatcaatgggcgtggatagcggtttgactcacggggatttccaagtctccaccccattgacgtcaatggg
agtttgttttgcaccaaaatcaacgggactttccaaaatgtcgtaacaactccgccccattgacgcaaatgggcggtagg
cgtgtacggtgggaggtctatataagcagagctcgtttagtgaaccgtcagatcgcctggagacgccatccacgctgtt
ttgacctccatagaagacaccgggaccgatccagcctccgcggattcgaatcccggccgggaacggtgcattggaa
cgcggattccccgtgccaagagtgacgtaagtaccgcctatagagtctataggcccacaaaaaatgctttcttcttttaat
atacttttttgtttatcttatttctaatactttccctaatctctttctttcagggcaataatgatacaatgtatcatgcctctttgcac
cattctaaagaataacagtgataatttctgggttaaggcaatagcaatatttctgcatataaatatttctgcatataaattgta
actgatgtaagaggtttcatattgctaatagcagctacaatccagctaccattctgcttttattttatggttgggataaggctg
gattattctgagtccaagctaggcccttttgctaatcatgttcatacctcttatcttcctcccacagctcctgggcaacgtgc
 
tggtctgtgtgctggcccatcactttggcaaagaattgggattcgaacatcgattgaattcgccgccaccgccaccatgg
tgagcaagggcgaggagctgttcaccggggtggtgcccatcctggtcgagctggacggcgacgtaaacggccaca
agttcagcgtgtccggcgagggcgagggcgatgccacctacggcaagctgaccctgaagttcatctgcaccaccgg
caagctgcccgtgccctggcccaccctcgtgaccaccctgacctacggcgtgcagtgcttcagccgctaccccgacc
acatgaagcagcacgacttcttcaagtccgccatgcccgaaggctacgtccaggagcgcaccatcttcttcaaggacg
acggcaactacaagacccgcgccgaggtgaagttcgagggcgacaccctggtgaaccgcatcgagctgaagggca
tcgacttcaaggaggacggcaacatcctggggcacaagctggagtacaactacaacagccacaacgtctatatcatg
gccgacaagcagaagaacggcatcaaggtgaacttcaagatccgccacaacatcgaggacggcagcgtgcagctc
gccgaccactaccagcagaacacccccatcggcgacggccccgtgctgctgcccgacaaccactacctgagcacc
cagtccgccctgagcaaagaccccaacgagaagcgcgatcacatggtcctgctggagttcgtgaccgccgccggga
tcactctcggcatggacgagctgtacaagTGAaagcttgcctcgagcagcgctgctcgagagatctacgggtggc
atccctgtgacccctccccagtgcctctcctggccctggaagttgccactccagtgcccaccagccttgtcctaataaaa
ttaagttgcatcattttgtctgactaggtgtccttctataatattatggggtggaggggggtggtatggagcaaggggcaa
gttgggaagacaacctgtagggcctgcggggtctattgggaaccaagctggagtgcagtggcacaatcttggctcact
gcaatctccgcctcctgggttcaagcgattctcctgcctcagcctcccgagttgttgggattccaggcatgcatgaccag
gctcagctaatttttgtttttttggtagagacggggtttcaccatattggccaggctggtctccaactcctaatctcaggtga
tctacccaccttggcctcccaaattgctgggattacaggcgtgaaccactgctcccttccctgtccttctgattttgtaggt
aaccacgtgcacctgcGTTC
SEQ ID NO: 20 
GTTCgcaggtgCGGCCGGTCGCGTCGAcattgattattgactagttattaatagtaatcaattacggg
gtcattagttcatagcccatatatggagttccgcgttacataacttacggtaaatggcccgcctggctgaccgcccaacg
acccccgcccattgacgtcaataatgacgtatgttcccatagtaacgccaatagggactttccattgacgtcaatgggtg
gagtatttacggtaaactgcccacttggcagtacatcaagtgtatcatatgccaagtacgccccctattgacgtcaatgac
ggtaaatggcccgcctggcattatgcccagtacatgaccttatgggactttcctacttggcagtacatctacgtattagtc
atcgctattaccatggtcgaggtgagccccacgttctgcttcactctccccatctcccccccctccccacccccaattttgt
atttatttattttttaattattttgtgcagcgatgggggcggggggggggggggggcgcgcgccaggcggggcgggg
cggggcgaggggcggggcggggcgaggcggagaggtgcggcggcagccaatcagagcggcgcgctccgaaa
gtttccttttatggcgaggcggcggcggcggcggccctataaaaagcgaagcgcgcggcgggcgggagtcgctgc
gcgctgccttcgccccgtgccccgctccgccgccgcctcgcgccgcccgccccggctctgactgaccgcgttactcc
cacaggtgagcgggcgggacggcccttctcctccgggctgtaattagcgcttggtttaatgacggcttgtttcttttctgt
ggctgcgtgaaagccttgaggggctccgggagggccctttgtgcggggggagcggctcggggggtgcgtgcgtgt
gtgtgtgcgtggggagcgccgcgtgcggctccgcgctgcccggcggctgtgagcgctgcgggcgcggcgcgggg
ctttgtgcgctccgcagtgtgcgcgaggggagcgcggccgggggcggtgccccgcggtgcggggggggctgcga
ggggaacaaaggctgcgtgcggggtgtgtgcgtgggggggtgagcagggggtgtgggcgcgtcggtcgggctgc
 
aaccccccctgcacccccctccccgagttgctgagcacggcccggcttcgggtgcggggctccgtacggggcgtgg
cgcggggctcgccgtgccgggcggggggtggcggcaggtgggggtgccgggcggggcggggccgcctcggg
ccggggagggctcgggggaggggcgcggcggcccccggagcgccggcggctgtcgaggcgcggcgagccgc
agccattgccttttatggtaatcgtgcgagagggcgcagggacttcctttgtcccaaatctgtgcggagccgaaatctgg
gaggcgccgccgcaccccctctagcgggcgcggggcgaagcggtgcggcgccggcaggaaggaaatgggcgg
ggagggccttcgtgcgtcgccgcgccgccgtccccttctccctctccagcctcggggctgtccgcggggggacggc
tgccttcgggggggacggggcagggcggggttcggcttctggcgtgtgaccggcggcTCTAGAGCCTCT
GCTAACCATGTTCATGCCTTCTTCTTTTTCCTACAGCTCCTGGGCAACGT
GCTGGTTATTGTGCTGTCTCATCATTTTGGCAAAGGATCCGGTACCGAG
GAGATCTGCCGCCGCGATCGCCATGgtgagcaagggcgaggagctgttcaccggggtggtg
cccatcctggtcgagctggacggcgacgtaaacggccacaagttcagcgtgtccggcgagggcgagggcgatgcc
acctacggcaagctgaccctgaagttcatctgcaccaccggcaagctgcccgtgccctggcccaccctcgtgaccac
cctgacctacggcgtgcagtgcttcagccgctaccccgaccacatgaagcagcacgacttcttcaagtccgccatgcc
cgaaggctacgtccaggagcgcaccatcttcttcaaggacgacggcaactacaagacccgcgccgaggtgaagttc
gagggcgacaccctggtgaaccgcatcgagctgaagggcatcgacttcaaggaggacggcaacatcctggggcac
aagctggagtacaactacaacagccacaacgtctatatcatggccgacaagcagaagaacggcatcaaggtgaactt
caagatccgccacaacatcgaggacggcagcgtgcagctcgccgaccactaccagcagaacacccccatcggcga
cggccccgtgctgctgcccgacaaccactacctgagcacccagtccgccctgagcaaagaccccaacgagaagcg
cgatcacatggtcctgctggagttcgtgaccgccgccgggatcactctcggcatggacgagctgtacaagTAAA
CGCGTACGCGGCCGCTCGAGGATTATAAGGATGACGACGATAAATTCG
TCGAGCACCACCACCACCACCACTAATAAGGTTTATCCGATCCACCGG
ATCTAGATAAGATATCCGATCCACCGGATCTAGATAACTGATCATAATC
AGCCATACCACATTTGTAGAGGTTTTACTTGCTTTAAAAAACCTCCCAC
ACCTCCCCCTGAACCTGAAACATAAAATGAATGCAATTGTTGTTGTTaact
tgtttattgcagcttataatggttacaaataaagcaatagcatcacaaatttcacaaataaagcatttttttcactgcattctag
ttgtggtttgtccaaactcatcaatgtatcttaACGCGGTAACCACGTGcacctgcGTTC
SEQ ID NO: 21 
GTTCgcaggtgCGGCCGCACGCGTCTAGTTATTAATAGTAATCAATTACGG
GGTCATTAGTTCATAGCCCATATATGGAGTTCCGCGTTACATAACTTAC
GGTAAATGGCCCGCCTGGCTGACCGCCCAACGACCCCCGCCCATTGAC
GTCAATAATGACGTATGTTCCCATAGTAACGCCAATAGGGACTTTCCAT
TGACGTCAATGGGTGGAGTATTTACGGTAAACTGCCCACTTGGCAGTAC
ATCAAGTGTATCATATGCCAAGTACGCCCCCTATTGACGTCAATGACGG
TAAATGGCCCGCCTGGCATTATGCCCAGTACATGACCTTATGGGACTTT
 
CCTACTTGGCAGTACATCTACGTATTAGTCATCGCTATTACCATGGTGA
TGCGGTTTTGGCAGTACATCAATGGGCGTGGATAGCGGTTTGACTCACG
GGGATTTCCAAGTCTCCACCCCATTGACGTCAATGGGAGTTTGTTTTGG
CACCAAAATCAACGGGACTTTCCAAAATGTCGTAACAACTCCGCCCCA
TTGACGCAAATGGGCGGTAGGCGTGTACGGTGGGAGGTCTATATAAGC
AGAGCTCGTTTAGTGAACCGTCAGATCGCCTGGAGACGCCATCCACGC
TGTTTTGACCTCCATAGAAGACACCGGGACCGATCCAGCCTCCGCGGAT
TCGAATCCCGGCCGGGAACGGTGCATTGGAACGCGGATTCCCCGTGCC
AAGAGTGACGTAAGTACCGCCTATAGAGTCTATAGGCCCACAAAAAAT
GCTTTCTTCTTTTAATATACTTTTTTGTTTATCTTATTTCTAATACTTTCC
CTAATCTCTTTCTTTCAGGGCAATAATGATACAATGTATCATGCCTCTTT
GCACCATTCTAAAGAATAACAGTGATAATTTCTGGGTTAAGGCAATAG
CAATATTTCTGCATATAAATATTTCTGCATATAAATTGTAACTGATGTA
AGAGGTTTCATATTGCTAATAGCAGCTACAATCCAGCTACCATTCTGCT
TTTATTTTATGGTTGGGATAAGGCTGGATTATTCTGAGTCCAAGCTAGG
CCCTTTTGCTAATCATGTTCATACCTCTTATCTTCCTCCCACAGCTCCTG
GGCAACGTGCTGGTCTGTGTGCTGGCCCATCACTTTGGCAAAGAATTGG
GATTCGAACATCGATTGAATTCTGAATGGTGGCCGGACATGCCTCTGGA
TCTCCAGCCTTTGGAACAGCCAGCCACAGCAACTGCGAGCACGAGGAA
ATTCACCTGGCCGGCTCTATTCAGCCTCATGGCGCTCTGCTGGTGGTGT
CCGAACACGACCACAGAGTGATTCAGGCCTCTGCCAATGCCGCCGAGT
TCCTGAATCTGGGCTCTGTGCTGGGAGTGCCTCTGGCCGAAATTGACGG
CGACCTGCTGATCAAGATCCTGCCTCACCTGGATCCTACCGCCGAGGGA
ATGCCTGTGGCCGTGCGTTGTAGAATCGGCAACCCTAGCACCGAGTACT
GCGGCCTGATGCACAGACCTCCAGAAGGCGGACTGATCATCGAGCTGG
AAAGAGCTGGCCCCAGCATCGATCTGTCTGGAACACTGGCTCCCGCTCT
GGAAAGAATCAGAACAGCCGGAAGCCTGAGAGCCCTGTGCGACGATA
CAGTGCTGCTGTTCCAGCAGTGCACCGGCTACGATAGAGTGATGGTGT
ACAGATTCGACGAGCAAGGCCACGGCCTGGTGTTCTCTGAATGTCACG
TGCCCGGCCTGGAAAGCTACTTCGGCAACAGATACCCCAGCAGCACCG
TGCCTCAGATGGCCAGACAGCTGTATGTGCGGCAGCGCGTTAGAGTGC
TGGTGGACGTCACATACCAGCCTGTGCCACTGGAACCCAGACTGAGCC
CTCTGACAGGCAGAGATCTGGATATGAGCGGCTGCTTCCTGCGGAGCA
TGAGCCCTTGCCATCTGCAGTTTCTGAAGGACATGGGCGTCAGAGCCAC
 
ACTGGCCGTGTCTCTTGTTGTTGGCGGAAAGCTGTGGGGCCTCGTCGTG
TGTCACCACTACCTGCCTCGGTTCATCAGATTCGAGCTGCGGGCTATCT
GCAAGCGGCTGGCCGAGAGAATCGCCACCAGAATCACAGCCCTGGAAT
CCTGAGGATCCTCTAGAGTCGACCTGCAGAAGCTTGCCTCGAGCAGCG
CTGCTCGAGAGATCTACGGGTGGCATCCCTGTGACCCCTCCCCAGTGCC
TCTCCTGGCCCTGGAAGTTGCCACTCCAGTGCCCACCAGCCTTGTCCTA
ATAAAATTAAGTTGCATCATTTTGTCTGACTAGGTGTCCTTCTATAATAT
TATGGGGTGGAGGGGGGTGGTATGGAGCAAGGGGCAAGTTGGGAAGA
CAACCTGTAGGGCCTGCGGGGTCTATTGGGAACCAAGCTGGAGTGCAG
TGGCACAATCTTGGCTCACTGCAATCTCCGCCTCCTGGGTTCAAGCGAT
TCTCCTGCCTCAGCCTCCCGAGTTGTTGGGATTCCAGGCATGCATGACC
AGGCTCAGCTAATTTTTGTTTTTTTGGTAGAGACGGGGTTTCACCATATT
GGCCAGGCTGGTCTCCAACTCCTAATCTCAGGTGATCTACCCACCTTGG
CCTCCCAAATTGCTGGGATTACAGGCGTGAACCACTGCTCCCTTCCCTG
TCCTTCTGATTTTGTAGGTAACCGTGAGTAGTGGAAGGCGGTTCTCTGG
CCAACGGGCTGCTTGCTGCTGTTAGCAGTTGGAGAATGGATGCCTCTGC
CCGGTAAAGGGCACCTGGGGCCGCGCCCGCAGCATTCACTAACTCGTA
ACTCTCCTTCCCTTCCTCCAAACACAAAATTCCATGACACTAGACAAGA
AAGCTGATGCTTGGAAAAGAGAATTGGCCTTAAATACCTAGATGGACT
GGAGAGACCATCCTTTGTGGTCCATAGCTTGAGGTCTGAGTACCCTGTC
CCCATCCACCTGCAGACCTGCTGAGGCTCCCCTCTGGCTTTCTCCTGCA
GAAACCTCTGCCATGGCCAAGCCTTTGTCTCAAGAAGAATCCACCCTCA
TTGAAAGAGCAACGGCTACAATCAACAGCATCCCCATCTCTGAAGACT
ACAGCGTCGCCAGCGCAGCTCTCTCTAGCGACGGCCGCATCTTCACTGG
TGTCAATGTATATCATTTTACTGGGGGACCTTGTGCAGAACTCGTGGTG
CTGGGCACTGCTGCTGCTGCGGCAGCTGGCAACCTGACTTGTATCGTCG
CGATCGGAAATGAGAACAGGGGCATCTTGAGCCCCTGCGGACGGTGTC
GACAGGTGCTTCTCGATCTGCATCCTGGGATCAAAGCGATAGTGAAGG
ACAGTGATGGACAGCCGACGGCAGTTGGGATTCGTGAATTGCTGCCCT
CTGGTTATGTGTGGGAGGGCTAAAGAGCAGAAGGAAGGGGTTCGTTGT
TGACTTGTGGAGATGTTGAGGAGAATCCAGGACCAGCACCGGGATCCA
TGGTGAGCGGCTGGCGGCTGTTCAAGAAGATTAGCGGCGGAGGTGGTT
CTGGCGGTGGAGGTTCAATGAACCAGTCCATCCCAGTTGCTCCCACCCC
ACCCCGCAGAGTGCGGCTGAAGCCCTGGCTGGTGGCCCAGGTGAACAG
 
CTGCCAGTACCCAGGGCTTCAATGGGTCAACGGGGAAAAGAAATTATT
CTGCATCCCCTGGAGGCATGCCACAAGGCATGGTCCCAGCCAGGACGG
AGATAACACCATCTTCAAGGTAAGCCCCGGGGAGGAGGTTGGCTGGAC
CTCCAGGGCACCCTGTCCCCAGAAGAGGAGCGCACATAACGCACACAG
GCAGCTCCTCGAGGcacctgcGTTC
SEQ ID NO: 22 
GTTCgcaggtgcagtccatcacgagcagctggtttctaagatgctatttcccgtataaagcatgagaccgtgacttgc
cagccccacagagccccgcccttgtccatcactggcatctggactccagcctgggttggggcaaagagggaaatga
gatcatgtcctaaccctgatcctcttgtcccacagatatccagaaccctgaccctgctgcggccgccaccatggacttcc
aggtgcagatcttcagcttcctgctgatcagcgccagcgtgatcatgagccggatggccgacatccagatgacccaga
gcccttcctctctttccgcttccgtgggggacagggtaactattacctgccgagcttcccagggcatccgcaactacctc
gcatggtaccagcagaagcctggcaaagctcccaagctgctgatctacgcagcttcgaccctgcagagcggtgtccc
ctctcgcttcagtggatctggatctggtacggactttactctgaccatctcgtccctgcagccggaagatgtggccactta
ttactgtcagcgttacaaccgcgcgccctataccttcggccagggcaccaaggttgagatcaagggaggctccagcc
gttcttcgtcctctggggggggtggctccggcggtggtggttccgaggtgcagctggtggagagcggcggagggct
ggtgcagccagggcgctcgttgcgcctgtcatgcgccgcttctggcttcacatttgatgactacgccatgcattgggtcc
gccaggcccccggcaagggcctggagtgggtcagcgccatcacctggaactccggccacattgactacgcggaca
gcgtggagggcagattcaccatctcccgtgacaacgcgaaaaacagcctttacttgcagatgaattccctgcgggctg
aggacaccgccgtgtactactgtgccaaggtgagctacctgtctacggccagttcgttggattattggggccaaggca
ctctcgtcaccgtgagcagtctcgagcagaagctgatcagcgaggaggacctgaaccggatccgtggggtcaccgt
ctcttcagcgctgagcaactccatcatgtacttcagccacttcgtgccggtcttcctgccagcgaagcccaccacgacg
ccagcgccgcgaccaccaacaccggcgcccaccatcgcgtcgcagcccctgtccctgcgcccagaggcgtgccg
gccagcggcggggggcgcagtgcacacgagggggctggacccctttgggttttgggtgctggtggtggttggtgga
gtcctggcttgctatagcttgctagtaacagtggcctttattattttctgggtgaggagtaagaggagcaggctcctgcac
agtgactacatgaacatgactccccgccgccccgggcccacccgcaagcattaccagccctatgccccaccacgcg
acttcgcagcctatcgctccctcgagagagtgagagtgaagttcagcaggagcgcagacgcccccgcgtaccagca
gggccagaaccagctctataacgagctcaatctaggacgaagagaggagtacgatgttttggacaagagacgtggcc
gggaccctgagatggggggaaagccgagaaggaagaaccctcaggaaggcctgtacaatgaactgcagaaagat
aagatggcggaggcctacagtgagattgggatgaaaggcgagcgccggaggggcaaggggcacgatggccttta
ccagggtctcagtacagccaccaaggacacctacgacgcccttcacatgcaggccctgccccctcgcggttccgga
gccacgaacttctctctgttaaagcaagcaggagacgtggaagaaaaccccggtcccggttccggagagggcagag
gaagcttgctcacctgcggggatgtggaggagaatccaggtcctatgcacagctcagcactgctctgttgcctggtcct
cctgactggggtgagggccagcccaggccagggcacccagtctgagaacagctgcacccacttcccaggcaacct
gcctaacatgcttcgagatctccgagatgccttcagcagagtgaagactttctttcaaatgaaggatcagctggacaact
 
tgttgttaaaggagtccttgctggaggactttaagggttacctgggttgccaagccttgtctgagatgatccagttttacct
ggaggaggtgatgccccaagctgagaaccaagacccagacatcaaggcgcatgtgaactccctgggggagaacct
gaagaccctcaggctgaggctacggcgctgtcatcgatttcttccctgtgaaaacaagagcaaggccgtggagcagg
tgaagaatgcctttaataagctccaagagaaaggcatctacaaagccatgagtgagtttgacatcttcatcaactacata
gaagcctacatgacaatgaagatacgaaacggttccggagccacgaacttctctctgttaaagcaagcaggagacgt
ggaagaaaaccccggtcccggttccggagagggcagaggaagcttgctcacctgcggggatgtggaggagaatcc
aggtcctatgccgccctccgggctgcggctgctgctgctgctgctaccgctgctgtggctactggtgctgacgcctgg
ccggccggccgcgggactatccacctgcaagactatcgacatggagctggtgaagcggaagcgcatcgaggccat
ccgcggccagatcctgtccaagctgcggctcgccagccccccgagccagggggaggtgccgcccggcccgctgc
ccgaggccgtgctcgccctgtacaacagcacccgcgaccgggtggccggggagagtgcagaaccggagcccga
gcctgaggccgactactacgccaaggaggtcacccgcgtgctaatggtggaaacccacaacgaaatctatgacaagt
tcaagcagagtacacacagcatatatatgttcttcaacacatcagagctccgagaagcggtacctgaacccgtgttgct
ctcccgggcagagctgcgtctgctgaggctcaagttaaaagtggagcagcacgtggagctgtaccagaaatacagca
acaattcctggcgatacctcagcaaccggctgctggcacccagcgactcgccagagtggttatcttttgatgtcaccgg
agttgtgcggcagtggttgagccgtggaggggaaattgagggctttcaccttagcgcccactgctccagagacagca
gggataacacactgcaagtggacatcaacgggttcactaccggccgccgaggtgacctggccaccattcatggcatg
aaccggcctttcctgcttctcatggccaccccgctggagagggcccagcatctgcaaagctcccggcaccgccgagc
cctggacaccaactattgcttcagctccacggagaagaactgctgcgtgcggcagctgtacattgacttccgcaagga
cctcggctggaagtggatccacgagcccaagggctaccatgccaacttctgcctcgggccctgcccctacatttggag
cctggacacgcagtacagcaaggtcctggccctgtacaaccagcataacccgggcgcctcggcggcgccgtgctgc
gtgccgcaggcgctggagccgctgcccatcgtgtactacgtgggccgcaagcccaaggtggagcagctgtccaaca
tgatcgtgcgctcctgcaagtgcagctgacctcgactgtgccttctagttgccagccatctgttgtttgcccctcccccgt
gccttccttgaccctggaaggtgccactcccactgtcctttcctaataaaatgaggaaattgcatcgcattgtctgagtag
gtgtcattctattctggggggtggggtggggcaggacagcaagggggaggattgggaagacaatagcaggcatgct
ggggatgcggtgggctctatggcttctgaggcggaaagaaccagctggggctctagggggtatccccactagtcgtg
taccagctgagagactctaaatccagtgacaagtctgtctgcctattcaccgattttgattctcaaacaaatgtgtcacaaa
gtaaggattctgatgtgtatatcacagacaaaactgtgctagacatgaggtctatggacttcaagagcaacagtgctgtg
gcctggagcaacaaatctgactttgcatgtgcaaacgccttcaaagatccacctgcGTTC
SEQ ID NO: 23 
TTGAGATCCTTTTTTTCTGCGCGTAATCTGCTGCTTGCAAACAAAAAAA
CCACCGCTACCAGCGGTGGTTTGTTTGCCGGATCAAGAGCTACCAACTC
TTTTTCCGAAGGTAACTGGCTTCAGCAGAGCGCAGATACCAAATACTGT
TCTTCTAGTGTAGCCGTAGTTAGGCCACCACTTCAAGAACTCTGTAGCA
CCGCCTACATACCTCGCTCTGCTAATCCTGTTACCAGTGGCTGCTGCCA
 
GTGGCGATAAGTCGTGTCTTACCGGGTTGGACTCAAGACGATAGTTACC
GGATAAGGCGCAGCGGTCGGGCTGAACGGGGGGTTCGTGCACACAGCC
CAGCTTGGAGCGAACGACCTACACCGAACTGAGATACCTACAGCGTGA
GCTATGAGAAAGCGCCACGCTTCCCGAAGGGAGAAAGGCGGACAGGT
ATCCGGTAAGCGGCAGGGTCGGAACAGGAGAGCGCACGAGGGAGCTT
CCAGGGGGAAACGCCTGGTATCTTTATAGTCCTGTCGGGTTTCGCCACC
TCTGACTTGAGCGTCGATTTTTGTGATGCTCGTCAGGGGGGCGGAGCCT
ATGGAAA
SEQ ID NO: 24 
CAAATAAAACGAAAGGCTCAGTCGAAAGACTGGGCCTTTCGTTTTATCT
GTTGTTTGTCGGTGAACGCTCTCCTGAGTAGGACAAAT
SEQ ID NO: 25 
TGGCGGGCAGGACGCCCGCCATAAACTGCCAGGCATCAAATTAAGCAG
AAGGCCATCCTGACGGATGGCCTTTTTGCGTTTCTACAAA
SEQ ID NO: 26 
ATTCTCACCAATAAAAAACGCCCGGCGGCAACCGAGCGTTCTGAACAA
ATCCAGATGGAGTTCTGAGGTCATTACTGGATCTATCAACAGGAGTC
SEQ ID NO: 27 
gctagcgtttctgcacacttaagcttGAGACGggtaccgagctcggatccgaattctgcagatatccagcacagtg
gcggccgctcgagtGTGCAGtgtgagcaagggcgaggagctgttcaccggggtggtgcccatcctggtcgag
ctggacggcgacgtaaacggccacaagttcagcgtgtccggcgagggcgagggcgatgccacctacggcaagctg
accctgaagttcatctgcaccaccggcaagctgcccgtgccctggcccaccctcgtgaccaccctgacctacggcgt
gcagtgcttcagccgctaccccgaccacatgaagcagcacgacttcttcaagtccgccatgcccgaaggctacgtcc
aggagcgcaccatcttcttcaaggacgacggcaactacaagacccgcgccgaggtgaagttcgagggcgacaccct
ggtgaaccgcatcgagctgaagggcatcgacttcaaggaggacggcaacatcctggggcacaagctggagtacaa
ctacaacagccacaacgtctatatcatggccgacaagcagaagaacggcatcaaggtgaacttcaagatccgccaca
acatcgaggacggcagcgtgcagctcgccgaccactaccagcagaacacccccatcggcgacggccccgtgctgc
tgcccgacaaccactacctgagcacccagtccgccctgagcaaagaccccaacgagaagcgcgatcacatggtcct
gctggagttcgtgaccgccgccgggatcactctcggcatggacgagctgtacaagaCGTCTCactagtAAA
AAAAAAAAAAAAAAAAAAAAAAAAAAAAAAAAAAAAAAAAAAAAA
AAAAAAAAAAAAAAAAAAAAAAAAAAAAAAAAAAAAAAAAAAAAA
AAAAAAAGgaagagcctagctacctactcgagtctagagggccc
SEQ ID NO: 28 
gctagcgtttctgcacacttaagcttGAGACGggtaccgagctcggatccgaattctgcagatatccagcacagtg

gcggccgctcgagtGTGCAGtaCGTCTCactagtAAAAAAAAAAAAAAAAAAAAAAA

AAAAAAAAAAAAAAAAAAAAAAAAAAAAAAAAAAAAAAAAAAAAAAA

AAAAAAAAAAAAAAAAAAAAAAAAAAAAAAAAAGGAAGAGCctagctaccta

ctcgagtctagagggccc
SEQ ID NO: 29

TAATACGACTCACTATAagGGGTCTTCTGGTCCCCACAGACTCAGAGAG
AACCCGCCACCATGGTGAGCAAGGGCGAGGAGCTGTTCACCGGGGTGG
TGCCCATCCTGGTCGAGCTGGACGGCGACGTAAACGGCCACAAGTTCA
GCGTGTCCGGCGAGGGCGAGGGCGATGCCACCTACGGCAAGCTGACCC
TGAAGTTCATCTGCACCACCGGCAAGCTGCCCGTGCCCTGGCCCACCCT
CGTGACCACCCTGACCTACGGCGTGCAGTGCTTCAGCCGCTACCCCGAC
CACATGAAGCAGCACGACTTCTTCAAGTCCGCCATGCCCGAAGGCTAC
GTCCAGGAGCGCACCATCTTCTTCAAGGACGACGGCAACTACAAGACC
CGCGCCGAGGTGAAGTTCGAGGGCGACACCCTGGTGAACCGCATCGAG
CTGAAGGGCATCGACTTCAAGGAGGACGGCAACATCCTGGGGCACAAG
CTGGAGTACAACTACAACAGCCACAACGTCTATATCATGGCCGACAAG
CAGAAGAACGGCATCAAGGTGAACTTCAAGATCCGCCACAACATCGAG
GACGGCAGCGTGCAGCTCGCCGACCACTACCAGCAGAACACCCCCATC
GGCGACGGCCCCGTGCTGCTGCCCGACAACCACTACCTGAGCACCCAG
TCCGCCCTGAGCAAAGACCCCAACGAGAAGCGCGATCACATGGTCCTG
CTGGAGTTCGTGACCGCCGCCGGGATCACTCTCGGCATGGACGAGCTG
TACAAGTAAtagtaagctggagcctcggtggccatgcttcttgccccttgggcctccccccagcccctcctcc
ccttcctgcacccgtacccccgtggtctttgaataaagtctgagtgggcggcaactagtAAAAAAAAAA
AAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAgGgaa
gagc

Although the present application has been described in conjunction with one or more embodiments, it should be understood that the present application is not limited to these embodiments. The descriptions in the present application are intended to encompass all variations and equivalents, which are all included within the spirit and scope of the appended claims. All documents cited herein are incorporated herein by reference in their entireties.

## Claims

1. A vector, **characterized in that** the vector comprises:
i) an origin of replication;
ii) a first terminator;
iii) a) a target DNA expression cassette, comprising a promoter and a target DNA sequence, wherein the target DNA sequence comprises a DNA sequence capable of forming a non-B DNA conformation, or b) a target DNA sequence, comprising a DNA sequence capable of forming a non-B DNA conformation;
iv) a second terminator; and
v) a selectable marker and a promoter that controls the expression of the selectable marker,
wherein the first terminator and the second terminator are respectively located upstream and downstream of the target DNA expression cassette of a), and configured to terminate transcription outside the target DNA expression cassette of a) upstream and downstream of the target DNA expression cassette of a); or
the first terminator and the second terminator are respectively located upstream and downstream of the target DNA sequence of b), and configured to terminate transcription outside the target DNA sequence of b) upstream and downstream of the target DNA sequence of b).

2. The vector according to claim 1, **characterized in that** the origin of replication is spaced at least 150 bp in a replication direction from the DNA sequence capable of forming a non-B DNA conformation in the target DNA expression cassette of a), or the DNA sequence capable of forming a non-B DNA conformation in the target DNA sequence of b).

3. The vector according to claim 2, **characterized in that** the origin of replication is spaced at least 500 bp in a replication direction from the DNA sequence capable of forming a non-B DNA conformation in the target DNA expression cassette of a), or the DNA sequence capable of forming a non-B DNA conformation in the target DNA sequence of b).

4. The vector according to any one of claims 1-3, **characterized in that** the DNA sequence capable of forming a non-B DNA conformation is a direct repeat sequence, an inverted repeat sequence, or a homopurine-homopyrimidine sequence.

5. The vector according to claim 4, **characterized in that** the DNA sequence capable of forming a non-B DNA conformation is a poly(A) tail-coding sequence.

6. The vector according to any one of claims 1-5, **characterized in that** the origin of replication is a pMB1 origin of replication, a pBR322 origin of replication, a ColE1 origin of replication, an R6K origin of replication, a p15A origin of replication, a pSC101 origin of replication, a pUC origin of replication, a pET origin of replication, or a derivative thereof.

7. The vector according to any one of claims 1-6, **characterized in that** the first terminator is selected from one or more of an rrnB T1 terminator, an rrnB T2 terminator, a lambda t0 terminator, a TonB terminator, an Amp terminator, a T7 terminator, and a CYC 1 terminator; and the second terminator is selected from one or more of an rrnB T1 terminator, an rrnB T2 terminator, a lambda t0 terminator, a TonB terminator, an Amp terminator, a T7 terminator, and a CYC1 terminator.

8. The vector according to any one of claims 1-7, **characterized in that** the selectable marker is an ampicillin resistance gene, a tetracycline resistance gene, a chloramphenicol resistance gene, a kanamycin resistance gene, or a streptomycin resistance gene.

9. The vector according to any one of claims 1-8, **characterized in that** the target DNA sequence in the target DNA expression cassette of a) further comprises a cloning site, and the target DNA sequence of b) further comprises a cloning site.

10. The vector according to any one of claims 1-9, **characterized in that** the target DNA expression cassette further comprises an enhancer and/or a transcription terminator.

11. The vector according to claim 10, **characterized in that** the vector sequentially comprises:
a pUC origin of replication, an rrnB T1 terminator and an rrnB T2 terminator, a CMV enhancer, a CMV promoter, a target DNA sequence, a bGH poly(A) signal, a lambda t0 terminator, and a kanamycin resistance element; or
a pUC origin of replication, an rrnB T1 terminator and an rrnB T2 terminator, a target DNA sequence, a lambda t0 terminator, and a kanamycin resistance element.

12. A vector, **characterized in that** the vector comprises:
i) an origin of replication;
ii) a first terminator;
iii) a) a target DNA expression cassette, sequentially comprising a first ITR or a derivative sequence thereof, a promoter, a target DNA sequence, and a second ITR or a derivative sequence thereof; or b) a target DNA sequence set, sequentially comprising a first ITR or a derivative sequence thereof, a target DNA sequence, and a second ITR or a derivative sequence thereof;
iv) a second terminator; and
v) a selectable marker and a promoter that controls the expression of the selectable marker,
wherein the first terminator and the second terminator are respectively located upstream and downstream of the target DNA expression cassette of a), and configured to terminate transcription outside the target DNA expression cassette of a) upstream and downstream of the target DNA expression cassette of a); or
the first terminator and the second terminator are respectively located upstream and downstream of the target DNA sequence set of b), and configured to terminate transcription outside the target DNA sequence set of b) upstream and downstream of the target DNA sequence set of b).

13. The vector according to claim 12, **characterized in that** the origin of replication is spaced at least 150 bp, preferably at least 500 bp, in a replication direction from the target DNA expression cassette of a) or the target DNA sequence set of b).

14. The vector according to claim 12 or 13, **characterized in that** the first terminator is selected from one or more of an rrnB T1 terminator, an rrnB T2 terminator, a lambda t0 terminator, a TonB terminator, an Amp terminator, a T7 terminator, and a CYC1 terminator; and the second terminator is selected from one or more of an rrnB T1 terminator, an rrnB T2 terminator, a lambda t0 terminator, a TonB terminator, an Amp terminator, a T7 terminator, and a CYC1 terminator.

15. The vector according to any one of claims 12-14, **characterized in that** the origin of replication is a pMB1 origin of replication, a pBR322 origin of replication, a ColE1 origin of replication, an R6K origin of replication, a p15A origin of replication, a pSC101 origin of replication, a pUC origin of replication, a pET origin of replication, or a derivative thereof.

16. The vector according to any one of claims 12-15, **characterized in that** the selectable marker is an ampicillin resistance gene, a tetracycline resistance gene, a chloramphenicol resistance gene, a kanamycin resistance gene, or a streptomycin resistance gene.

17. The vector according to any one of claims 12-16, **characterized in that** the first ITR or the derivative sequence thereof comprises a nucleotide sequence set forth in SEQ ID NO: 9, 10, 11, or 12; and the second ITR or the derivative sequence thereof comprises a nucleotide sequence set forth in SEQ ID NO: 9, 10, 11, or 12.

18. The vector according to any one of claims 12-17, **characterized in that** the target DNA expression cassette further comprises an enhancer and/or a transcription terminator.

19. The vector according to claim 18, **characterized in that** the vector sequentially comprises:
a pUC origin of replication, an rrnB T1 terminator and an rrnB T2 terminator, a first ITR or a derivative sequence thereof, a CMV enhancer, a chicken β-actin promoter, a target DNA sequence, a bGH poly(A) signal, a second ITR or a derivative sequence thereof, a lambda t0 terminator, and a kanamycin resistance element.

20. A method for modifying a vector, **characterized in that** the method comprises:
i) providing a vector, comprising:
a) an origin of replication;
b) b-i) a cloning site,
b-ii) a DNA expression cassette comprising a promoter and a cloning site,
b-iii) a DNA expression cassette comprising a promoter, a cloning site, and a DNA sequence capable of forming a non-B DNA conformation,
b-iv) a target DNA sequence comprising a DNA sequence capable of forming a non-B DNA conformation,
b-v) a target DNA expression cassette comprising a promoter and a target DNA sequence, wherein the target DNA sequence comprises a DNA sequence capable of forming a non-B DNA conformation,
b-vi) a DNA sequence set sequentially comprising a first ITR or a derivative sequence thereof, a cloning site, and a second ITR or a derivative sequence thereof,
b-vii) a DNA expression cassette sequentially comprising a first ITR or a derivative sequence thereof, a promoter, a cloning site, and a second ITR or a derivative sequence thereof,
b-viii) a target DNA expression cassette sequentially comprising a first ITR or a derivative sequence thereof, a promoter, a target DNA sequence, and a second ITR or a derivative sequence thereof, or
b-ix) a target DNA sequence set sequentially comprising a first ITR or a derivative sequence thereof, a target DNA sequence, and a second ITR or a derivative sequence thereof; and
c) a selectable marker and a promoter that controls the expression of the selectable marker; and
ii) incorporating a first terminator and a second terminator into the vector, wherein the first terminator and the second terminator are respectively located upstream and downstream of the sequence of b), and configured to terminate transcription outside the sequence of b) in the vector upstream and downstream of the sequence of b).

21. The method according to claim 20, **characterized in that** the method further comprises spacing the origin of replication at least 150 bp, preferably at least 500 bp, in a replication direction from b-i) the cloning site, b-ii) the cloning site in the DNA expression cassette, b-iii) the DNA sequence capable of forming a non-B DNA conformation in the DNA expression cassette, b-iv) the DNA sequence capable of forming a non-B DNA conformation in the target DNA sequence, b-v) the DNA sequence capable of forming a non-B DNA conformation in the target DNA expression cassette, b-vi) the DNA sequence set, b-vii) the DNA expression cassette, b-viii) the target DNA expression cassette, or b-ix) the target DNA sequence set.

22. The method according to claim 20 or 21, **characterized in that** the vector further comprises a lac promoter, and the method further comprises inactivating the lac promoter.

23. A composition, **characterized in that** the composition comprises the vector according to any one of claims 1-11, the vector according to any one of claims 12-19, or a vector obtained by the method according to any one of claims 20-22.

24. A method for replicating a target DNA sequence, **characterized in that** the method comprises:
i) providing the vector according to any one of claims 1-11, or the vector according to any one of claims 12-19;
ii) transforming/transfecting the vector of step i) into a host cell; and
iii) culturing the host cell under conditions conducive to vector replication.

25. A method for expressing a target DNA sequence, **characterized in that** the method comprises:
i) providing the vector according to any one of claims 1-11, which vector comprises a) the target DNA expression cassette; and
ii) transforming/transfecting the vector of step i) into a host cell, and culturing the host cell under conditions conducive to expression of the target DNA sequence; or linearizing the vector of step i) and performing *in-vitro* transcription; or
i) providing the vector according to any one of claims 12-19, which vector comprises a) the target DNA expression cassette; and
ii) transforming/transfecting the vector of step i) into a host cell, and culturing the host cell under conditions conducive to expression of the target DNA sequence;
linearizing the vector of step i) and performing *in-vitro* transcription; or
co-transforming/co-transfecting the vector of step i) with a packaging plasmid and a helper plasmid into a host cell, culturing the host cell under conditions conducive to vector packaging, recovering the packaged vector, transforming/transfecting the packaged vector into the host cell, and culturing the host cell under conditions conducive to expression of the target DNA.

26. The method according to claim 25, **characterized in that** the method further comprises, between step i) and step ii), transforming/transfecting the vector of step i) into a host cell, and culturing the host cell under conditions conducive to vector replication.

27. A method for preparing a recombinant adeno-associated virus vector, **characterized in that** the method comprises:
i) providing the vector according to any one of claims 12-19;
ii) transforming/transfecting the vector of step i) into a host cell;
iii) culturing the host cell under conditions conducive to vector replication; and
iv) harvesting the vector from the host cell of step iii), co-transforming/co-transfecting the harvested vector with a packaging plasmid and a helper plasmid into a host cell, and culturing the host cell under conditions conducive to vector packaging.
